(19) Europäisches Patentamt
European Patent Office
Office européen des brevets

(11) **EP 1 704 816 B1**

(12) **EUROPEAN PATENT SPECIFICATION**

(45) Date of publication and mention
of the grant of the patent:
**01.06.2011 Bulletin 2011/22**

(51) Int Cl.:
*G06T 7/00* (2006.01)   *A61B 5/00* (2006.01)
*G06F 19/00* (2011.01)

(21) Application number: **06011951.8**

(22) Date of filing: **22.10.2002**

(54) **Information processing apparatus and method**

Vorrichtung und Verfahren zur Informationsverarbeitung

Appareil et procédé de traitement d'informations

(84) Designated Contracting States:
**DE FR GB**

(30) Priority: **22.10.2001 JP 2001324036**

(43) Date of publication of application:
**27.09.2006 Bulletin 2006/39**

(62) Document number(s) of the earlier application(s) in
accordance with Art. 76 EPC:
**02777919.8 / 1 452 129**

(73) Proprietor: **Olympus Corporation
Tokyo 151-0072 (JP)**

(72) Inventors:
 • **Nishimura, Hirokazu
  2-3, Kuboyama-cho, Hachioji-shi, Tokyo (JP)**
 • **Tanaka, Hideki
  2-3, Kuboyama-cho, Hachioji-shi, Tokyo (JP)**
 • **Yamazaki, Kenji
  2-3, Kuboyama-cho, Hachioji-shi, Tokyo (JP)**

(74) Representative: **von Hellfeld, Axel
Wuesthoff & Wuesthoff
Patent- und Rechtsanwälte
Schweigerstrasse 2
81541 München (DE)**

(56) References cited:
 **US-A- 4 945 409   US-A- 5 872 861**

 • **KREVSKY ET AL: "Enhanced imaging of
  angiodysplasias using remote endoscopic
  digital spectroscopy" GASTROINTESTINAL
  ENDOSCOPY, ELSEVIER, NL, vol. 44, no. 5,
  November 1996 (1996-11), pages 598-602,
  XP005127768 ISSN: 0016-5107**
 • **ENGLMEIER K-H ET AL: "A method for the
  estimation of the hemoglobin distribution in
  gastroscopic images" INTERNATIONAL
  JOURNAL OF BIO-MEDICAL COMPUTING,
  ELSEVIER SCIENCE PUBLISHERS, SHANNON,
  IE, vol. 41, no. 3, June 1996 (1996-06), pages
  153-165, XP004008763 ISSN: 0020-7101**

EP 1 704 816 B1

## EP 1 704 816 B1

**Description**

Technical Field

The present invention relates to a diagnostic support apparatus.

Background Art

In the medical field, there is increasing practice of making diagnoses using imaging equipment such as X-ray apparatuses, CT apparatuses, MRI apparatuses, ultrasound observation apparatuses, and endoscopic apparatuses.

For example, with an endoscopic apparatus, a doctor can observe the organs in the body cavity and make a diagnosis by inserting the elongated insertion portion into the body cavity and using a solid-stage imaging element or the like as an imaging means. In addition, an ultrasound endoscopic apparatus is also widely used, which irradiates the organs in the body cavity with ultrasound waves and allows a doctor to observe the state of the organs in the body cavity on the monitor screen by means of reflection, transmittance, or the like of the ultrasound waves, thereby allowing the doctor to make an examination or diagnosis.

Furthermore, image filing systems and the like have been popularized, which can save images sensed by these apparatuses upon adding various information to the images and allow them to be retrieved, acquired, and displayed as needed.

However, final diagnoses using these medical imaging apparatuses are mainly based on the subjective points of view of doctors. Demands have therefore arisen for the realization of a diagnostic support apparatus which directly leads to objective, numerical diagnoses. A diagnostic support apparatus is designed to find a lesion from an image to be diagnosed by performing threshold processing or using a statistical/non-statistical discriminator on the basis of various characteristic values calculated from the image or a region of interest set on the image, and to present a doctor classification to specific findings and lesions, thereby supporting an objective, numerical diagnosis.

In contrast to this, in the field of breast cancer diagnosis (mammography) using X-ray images, a diagnostic support apparatus for recognizing an abnormal finding such as calcification and assisting a doctor's diagnosis has been put into practice. A diagnostic support apparatus which assists differentiation of lesions is also disclosed in, for example, Jpn. Pat. Appln. KOKAI Publication No. 10-14864. This apparatus is designed to realize diagnostic support for differentiation of various diseases on the basis of many patients, examinations, and image information recorded in an image filing system or the like.

The conventional diagnostic support apparatuses have, however, the following problems.

(1) Problem Concerning Diagnostic Support Contents

Diagnostic support contents that can be used in clinical examination are fixed in each diagnostic support apparatus to be used, including, for example, "finding of breast cancer shadow" and "examination on pneumoconiosis" (in the present invention, various diagnostic support types and contents are generally referred to as diagnostic support contents hereinafter; a detailed description thereof will be made later). In spite of the fact that a diagnostic support apparatus basically uses a general-purpose computer or workstation as hardware, the user cannot easily obtain desired diagnostic support information in accordance with diagnosis purposes and contents or make additions, improvements, and the like with respect to diagnostic support contents.

(2) Problem Concerning Development of Diagnosis

Support Contents

In general, diagnostic support contents are developed on the basis of element techniques provided from mathematical engineering organizations and companies and data and medical knowledge provided from a limited number of specific university hospitals and various kinds of medical facilities. These element techniques are highly specialized into image analysis techniques and identification/classification techniques.

Image filing systems have currently been used in many medical facilities and organizations, and much data is stored in the systems. The respective medial facilities differ in the numbers of specialties and cases for each disease. In spite of the fact that various diagnostic support contents can be developed by integrating this data and most advanced medical knowledge, no versatile tool is available or cannot be obtained. This hinders improvement in this field. Although various medical facilities and doctors have made many studies that can be implementation elements for diagnostic support, the results cannot be clinically used under present circumstances because of this problem.

(3) Problem Concerning Improvements to Diagnostic

Support Contents

The diagnostic support contents in a diagnostic support apparatus have already been established, and the user cannot improve the contents by, for example, adding case data. For example, more useful diagnostic support can be realized by implementing basic diagnostic support contents and improving the contents by, for example, adding data while clinically using them in many medical facilities, or by adding new information. However, as in the case of problem (2) described above, there is no means, such as a tool, which is necessary to improve the diagnostic support contents.

Document US 5,872,861 concerns a digital image processing method for automatic detection of stenoses. The stenoses is detected by measurement of the intensity of pixels along central lines of vessels. The detection comprises deciding whether a pixel zone is a potential stenosis zone when the intensity of its pixels exhibits a local variation so as to assume an intermediate value between the intensity of the pixels to both sides of the zone and the intensity of the pixels of the image background, defining an icon around potential stenosis zones and extracting pixels of edges of the vessel segments in the icons.

Document Krevsky et al.: "Enhanced imaging of angiodysplasias using remote endoscopic digital spectroscopy", GASTROINTESTINAL ENDOSCOPY, ELSEVIER, NL, vol. 44, no. 5, November 1996, pages 598 - 602, ISSN: 0016-5107 concerns enhanced imaging of angiodysplasias using remote endoscopic digital spectroscopy. Regions of interest in a living body, for example blood vessels, are determined based on mean color ratios.

Disclosure of Invention

The present invention concerns a diagnostic support apparatus having the features of claim 1 and a diagnostic support method having the features of claim 6.

It is an object of the present invention to provide a diagnostic support apparatus which allows various information, image data, and expert medical knowledge accumulated in many medical facilities to be widely used on diagnostic support apparatuses, can improve the performance of the diagnostic support apparatus, and allows selective use of diagnostic support in accordance with purposes and contents.

It is another object of the present invention to provide a diagnostic support apparatus which can easily and effectively assist various processes and operations required to create diagnostic support contents.

A diagnostic support apparatus according to the first aspect comprises:

diagnostic support content storage means for storing a plurality of diagnostic support contents for providing diagnostic support;
selection means for selecting a desired diagnostic support content from the plurality of diagnostic support contents stored in the diagnostic support content storage means;
information acquisition means for acquiring diagnostic information concerning at least one of a patient, an examination, and an image from a medical system;
diagnostic support information creating means for creating diagnostic support information on the basis of the diagnostic support content selected by the selection means and the diagnostic information acquired from the medical system; and
diagnostic support information display means for displaying the diagnostic support information created by the diagnostic support information creating means.

A diagnostic support method of providing diagnostic support using a computer according to the second aspect comprises:

a step of acquiring a diagnostic support content;
a step of inputting diagnostic information concerning at least one of a patient as a diagnostic support target, an examination, and an image;
a step of creating diagnostic support information using the diagnostic support content and the diagnostic information; and
a step of displaying the diagnostic support information.

An information processing apparatus according to the third aspect comprises:

storage means for storing processing data constituted by at least one image data, character string data, and numerical value data;

graph creating means for creating graph information from the numerical value data;

image list information creating means for creating image list information from the image data;

table list information creating means for creating table list information from the character string data and the numerical value data;

display means for displaying the graph information, the image list information, and the table list information;

selection means for selecting information displayed on the display means; and

information management means for managing the graph information, the image list information, and the table list information displayed on the display means.

A diagnostic support apparatus for supporting a diagnosis by an examiner according to the fourth aspect comprises

image storage means for storing image data input from an endoscopic device, characteristic value calculation means for calculating at least one characteristic value to quantify a finding associated with a diagnosis from image data stored in the image storage means, and diagnostic support information display means for displaying diagnostic support information on the basis of a calculation result obtained by the characteristic value calculation means,

the characteristic value calculation means including

blood vessel extraction means for extracting a transmission blood vessel image in the image data stored in the image storage means; and

blood vessel characteristic value calculation means for representing a running state of a see-through blood vessel image as a characteristic value on the basis of an output from the blood vessel extraction means.

Brief Description of Drawings

FIG. 1 is a view showing a form of a diagnostic support system according to the first embodiment;

FIG. 2 is a view for explaining the arrangement of a diagnostic support content server #2 in this embodiment;

FIG. 3 is a view for explaining the arrangement of a diagnostic support execution terminal #3 in this embodiment;

FIG. 4 is a block diagram of a main program #31 executed by a control means #9 of the diagnostic support content server #2 in this embodiment;

FIG. 5 is a block diagram showing a diagnostic support content distribution executing section #32 in more detail;

FIG. 6 is a flowchart for explaining a series of operations in distribution of diagnostic support contents in this embodiment;

FIG. 7 is a block diagram of a main program #51 executed by a control means #12 of the diagnostic support execution terminal #3 in this embodiment;

FIG. 8 is a flowchart for explaining a series of operations accompanying the reception of a diagnostic support content by the diagnostic support execution terminal #3 in this embodiment;

FIG. 9 is a view showing the first example of a diagnostic support content;

FIG. 10 is a view showing the second example of a diagnostic support content;

FIG. 11 is a view showing the third example of a diagnostic support content;

FIG. 12 is a view showing detailed display of the third example of a diagnostic support content;

FIG. 13 is a view showing the fourth example of a diagnostic support content;

FIG. 14 is a view showing the fifth example of a diagnostic support content;

FIG. 15 is a view showing the arrangement of a diagnostic support content object A60;

FIG. 16 is a flowchart for explaining a series of operations accompanying the creation of diagnostic support information and updating/addition of a diagnostic support content by the diagnostic support execution terminal #3 in this embodiment;

FIG. 17 is a view showing a diagnostic support content list menu;

FIG. 18 is a view showing a form of a diagnostic support system according to the second embodiment;

FIG. 19 is a view for explaining the arrangement of a diagnostic support content creating terminal #102 in this embodiment;

FIG. 20 is a block diagram of a main program #121 executed by a control means #12 of the diagnostic support content creating terminal #102 in this embodiment;

FIG. 21 is a flowchart for explaining a series of operations in diagnostic support content creation;

FIG. 22 is a view showing a window group for data set creation;

FIG. 23 is a view showing an examination condition setting window A120;

FIG. 24 is a view showing a text information setting window A125;

FIG. 25 is a view showing a reference image setting window A130;

FIG. 26 is a view showing a call diagnostic support content selection window A140;

FIG. 27 is a view showing a diagnostic support main menu window A200;

FIG. 28 is a view showing an examination condition setting window A210;

FIG. 29 is a view showing a diagnostic support content setting window A220;

FIG. 30 is a view showing a terminal authentication information setting window A230;

FIG. 31 is a flowchart for explaining a series of operations accompanying the creation of diagnostic support information and updating/addition of a diagnostic support content by a diagnostic support execution terminal #3 in this embodiment;

FIG. 32 is a view showing a display example of a diagnostic support execution window;

FIG. 33 is a view showing a diagnostic support content server selection window A260;

FIG. 34 is a view showing a diagnostic support content creating main window A270;

FIG. 35 is a view showing a diagnostic support content list menu;

FIG. 36 is a view showing a diagnostic support execution terminal main program;

FIG. 37 is a flowchart for explaining operation associated with updating/addition of a diagnostic support content by the diagnostic support execution terminal #3 in this embodiment;

FIG. 38 is a view showing an example of the contents of an update/add inquiry information file;

FIG. 39 is a block diagram of a main program #121, which shows the arrangement of a diagnostic support content creating section #127 according to the third embodiment;

FIG. 40A is a view showing an image information format;

FIG. 40B is a view showing item management information contents;

FIG. 40C is a view showing auxiliary information contents;

FIG. 41 is a view showing an item selection window;

FIG. 42 is a flowchart (part 1) for explaining a series of operations accompanying graph information creation in this embodiment;

FIG. 43 is a flowchart (part 2) for explaining a series of operations accompanying graph information creation in this embodiment;

FIG. 44 is a flowchart (part 3) for explaining a series of operations accompanying graph information creation in this embodiment;

FIG. 45 is a flowchart (part 4) for explaining a series of operations accompanying graph information creation in this embodiment;

FIG. 46 is a flowchart (part 5) for explaining a series of operations accompanying graph information creation in this embodiment;

FIG. 47 is a view showing a display example of a graph in this embodiment, and more specifically, an example of a one-dimensional scatter diagram;

FIG. 48 is a view showing a display example of a graph in this embodiment, and more specifically, an example of a histogram;

FIG. 49 is a view showing a display example of a graph in this embodiment, and more specifically, an example of superimposing $\underline{t}$ test results on an average value bar graph;

FIG. 50 is a view showing a display example of a graph in this embodiment, and more specifically, an example of superimposing $\chi^2$ test results on a case count bar graph;

FIG. 51 is a view showing display of statistics or statistical test results associated with accompanying data items;

FIG. 52 is a view showing display of an operation window having a check box 165;

FIG. 53 is a flowchart showing a modification of the processing flow in FIG. 44;

FIGS. 54A and 54B are views showing the contents displayed when the check box 165 is checked in operation for the graph information display in FIG. 47;

FIGS. 55A to 55C are views each displaying graphs equal in number to the number of times a statistical test is performed;

FIG. 56 is a block diagram of a main program #121, which shows the arrangement of a diagnostic support content creating section #127 according to the fourth embodiment;

FIG. 57 is a flowchart for a display information management section 172, which explains linking operation between an image information list 173 and graph information 160 in this embodiment;

FIG. 58 is a view showing display of a list of image information acquired from a storage means management section #123 as an image information list 173;

FIG. 59 is a view showing graph information 160;

FIG. 60 is a view showing operation of enclosing graph elements with a rectangle on the graph information 160;

FIG. 61 is a flowchart for the display information management section 172 and an information setting section 181, which explains display changing operation for the image information list 173 and graph information 160, accompanying a change of the settings of image information;

FIG. 62 is a block diagram of a block diagram of the main program #121, which shows the arrangement of the diagnostic support content creating section #127 according to the fourth embodiment;

FIG. 63 is a view showing a menu 190 including selected element information updating 191 and selected element region-of-interest setting 192;

FIG. 64 is a view showing a setting operation window for the accompanying data of image information, which is used by an item value setting section 183;

FIG. 65 is a view showing the arrangement of an information update window 184;

FIG. 66 is a view showing how the item values of corresponding items are stored in a menu 230 and displayed;

FIG. 67 is a view for explaining a modification of the fifth embodiment;

FIG. 68 is a view showing a display example of item names set by the item value setting section 183;

FIG. 69 is a block diagram of a main program #121, which shows the arrangement of a diagnostic support content creating section #127 according to the fifth embodiment;

FIG. 70 is a flowchart for a region-of-interest setting section 201, which explains setting of a region of interest in this embodiment;

FIG. 71 is a view showing display of an image information list 173 in this embodiment;

FIG. 72A is a view showing the operation of a mouse 23 in a moving step (TI-2) and display on an image;

FIG. 72B is a view showing the operation of the mouse 23 in a size changing step (TI-4) and display on an image;

FIG. 72C is a view showing the operation of the mouse 23 in a position temporarily determining step (TI-3) and display on an image;

FIG. 73 is a block diagram of a main program #121 according to the seventh embodiment;

FIG. 74 is a flowchart for a marker rendering section 213, which explains marker rendering on image data in accordance with an item contained in accompanying data in this embodiment;

FIGS. 75A, 75B, and 75C are views for explaining rendering operation in the marker rendering section;

FIG. 76 is a block diagram of a main program #121 according to the eighth embodiment;

FIG. 77 is a flowchart for a character information erasing section 240 and character information rendering section 241, which explains how patient examination information is erased from image data and item information contained in accompanying data is rendered on the image data;

FIG. 78 is a view showing a display example of image data obtained by erasing the patient examination information rendered on the image data and rendering the values of characteristic values 1 and 2;

FIG. 79 is a block diagram of a main program #51 according to the ninth embodiment;

FIG. 80 is a block diagram showing an image processing section 220 in detail;

FIG. 81 is a view showing an image processing table 251;

FIG. 82 is a flowchart for an image processing section 250, which explains calculation of an image processing value corresponding to the pixel value of image data;

FIG. 83 is a block diagram of a main program #51 according to the 10th embodiment;

FIG. 84A is a flowchart for a data embedding section 260, which explains embedding of accompanying data and region-of-interest data in image data in this embodiment;

FIG. 84B is a flowchart for a data embedding section 230, which explains acquisition of accompanying and region-of-interest data embedded in image data in this embodiment;

FIG. 85 is a view for explaining the operation in FIG. 84A;

FIG. 86 is a view for explaining the operation in FIG. 84A;

FIG. 87 is a view showing the arrangement of a main program #121 having a characteristic value calculation means 008 according to the 11th embodiment, which is an embodiment of the present invention;

FIG. 88 is a view showing the arrangement of the characteristic value calculation means 008 in the 11th embodiment;

FIG. 89 is a view showing the arrangement of a blood vessel extraction means 101 in the characteristic value calculation means 008;

FIG. 90 is a flowchart for mainly explaining processing in the blood vessel extraction means 101;

FIG. 91 is a block diagram of a preprocessing section 111;

FIG. 92 is a schematic flowchart showing processing performed by a blood vessel candidate extracting section 121 for extracting blood vessel candidates on the basis of outputs from an edge information detecting section 122 and color tone calculating section 123;

FIG. 93 is a view showing an example of a spatial filter for performing second-order differentiation processing in the edge information detecting section 122;

FIG. 94 is a schematic flowchart showing processing performed by a shape edge determining section 132 on the basis of an output from a density gradient information calculating section 131;

FIG. 95 is a schematic flowchart showing the processing of separating and removing a form edge from a blood vessel candidates on the basis of the results obtained by the blood vessel candidate extracting section 121 and shape edge determining section 132;

FIG. 96 is a conceptual view of a density distribution, density gradient, second-order differentiation, color tone data, and blood vessel candidate data (to be described later) on a horizontal line of an image on which a blood vessel and shape edge exist;

FIG. 97 is a conceptual view of the density distribution, density gradient, and shape edge data based on shape edge determination (to be described later) at a blood vessel and shape edge; and

FIG. 98 is a conceptual view of the logical product of the blood vessel candidate data and shape edge data at a blood vessel and shape edge.

Best Mode for Carrying Out the Invention

[First Embodiment]

The details of diagnostic support in the present invention will be described first.

In general, a doctor performs diagnostic operation based on his/her own judgment. This means that the diagnosis results may vary depending on the differences between the experiences and differences in subjective judgment of doctors. With regard to this problem, diagnostic support is aimed at realizing accurate diagnoses without variations by providing various information, e.g., objective representation of information concerning findings, display of disease classification results obtained by an identification/classification technique, such as a linear discrimination function or neural network, and display of typical and similar cases as references at the time of diagnosis. Diagnostic support contents are the contents and types of support information to be provided for diagnostic support. For example, variations ① to ⑤ are conceivable as diagnostic support contents. Such a diagnostic support content is created as needed in accordance with imaging equipment (modality; the present invention will exemplify an endoscopic system), examination regions, the names of patients of interest, and the like.

① Objective Representation of Image Findings and Statistical Information Display Pertaining to Disease

In the medical endoscope field, for example, a color tone is one of important image findings. As a numerical value (characteristic value) objectively representing a difference in color tone, the IHb value is widely used. The IHb value is a value obtained for each pixel of an endoscopic image formed from an RGB color signal according to

$$32\log_2 Ri/Gi$$

This value is known as a value which has correlation with a submucous blood flow. As the mucous membrane becomes redder, the IHb value increases. In this case, "i" is a suffix representing a pixel number, and an average value in an overall image or a region of interest is used as diagnostic support information.

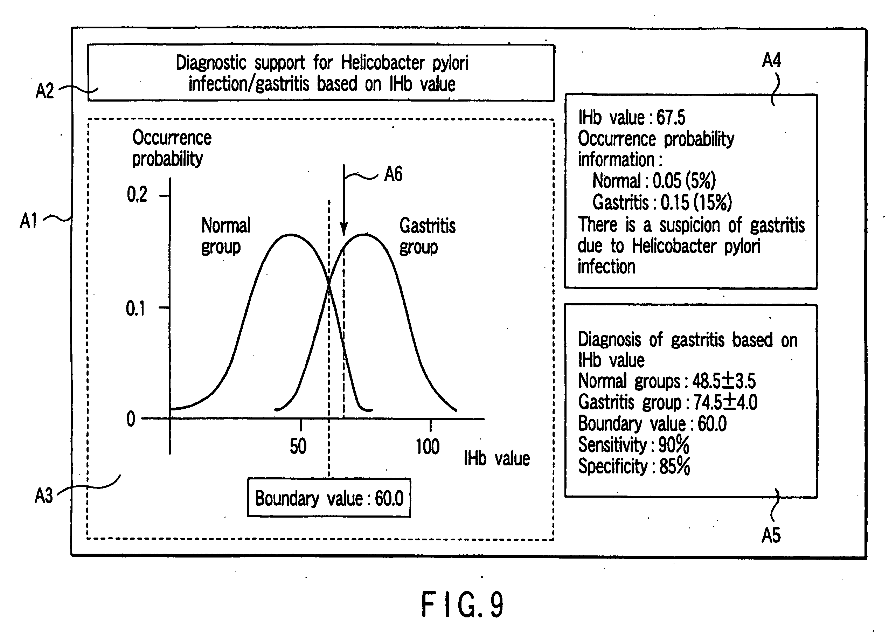

FIG. 9 shows an example of the diagnostic support content for providing diagnostic support for gastritis by using the IHb value. FIG. 9 shows the contents of a display window presented to a doctor. A display area A1 is constituted by a diagnostic support content name A2, graph information area A3, diagnostic information area A4, and statistical information area A5. In the graph information area A3, the occurrence probability distributions of IHb values in a normal group and disease group (gastritis group in this case) are graphically represented, and a pointer A6 indicating the position of the IHb value obtained from a case as a diagnosis target is superimposed on this representation. In the diagnostic information area A4, occurrence probability information obtained by referring to the IHb value of the case as the diagnosis target and the normal and gastritis groups in the graph information area A3 is displayed, and text information such as "there is a suspicion of gastritis due to Helicobacter pylori infection" is also output. The following statistical information is displayed in the statistical information area A5: average values $\pm$ standard deviations of IHb values in the normal and gastritis groups, a boundary value at which the occurrence probabilities in the respective groups coincide with each other, the sensitivity of the diagnostic support information using the IHb values, a specificity, and the like. The doctor executes a final diagnosis by referring to these pieces of diagnostic support information. Therefore, a diagnosis which has been dependent on a subjective judgment such as "the surface of the mucous membrane is red" is made on the basis of objective, statistical grounds by referring to the diagnostic support content described in this embodiment.

Alternatively, a pseudo-color image may be generated on the basis of the IHb values to be displayed together with the endoscopic image.

Diagnostic support using such characteristic values and statistical information is not limited to the color tone of an endoscopic image, and a diagnostic support content can be created, as needed, in accordance with another modality such as an X-ray image or ultrasound image and various types of findings such as structural components and density information. In addition, a similar diagnostic support content can be created with respect to the red blood cell count obtained by a blood examination, numerical values other than characteristic values which are obtained from an image, and the like.

② Disease Type Display Based on Identification/Classification Result Using Feature Amounts Obtained by Application of Image Analysis Technique

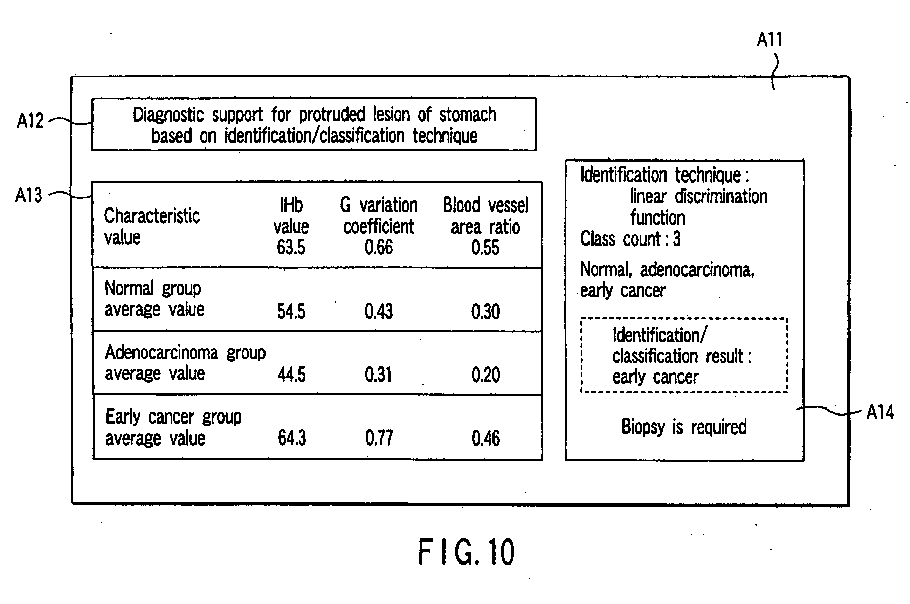

FIG. 10 shows an example of a diagnostic support content for providing diagnostic support for a protruded lesion of stomach (adenocarcinoma or early cancer) using a plurality of types of characteristic values obtained from an endoscopic image and an identification/classification technique. FIG. 10 shows the contents of a display window presented to the doctor. A display area A11 is constituted by a diagnostic support content name A12, calculated characteristic value information area A13, and diagnostic information area A14. The information displayed in the calculated characteristic value information area A13, which is associated with the values of characteristic values (three types in this case, i.e., the IHb value, G variation coefficient, and blood vessel area ratio) used for diagnostic support, includes the values calculated from a diagnosis target and average values as diagnosis results in a normal group, adenocarcinoma group, and early cancer group. In the diagnostic information area A14, an identification/classification technique name (linear discrimination function in this case), a class name as an identification result, and an identification/classification result are displayed.

In addition, text information such as "a biopsy is required" for a more accurate diagnosis is displayed (biopsy is a diagnostic method of sampling a mucous tissue using a special needle and checking a tissue image under a microscope).

③ Display of Images of Typical Case and Similar Case and Patient Examination Information

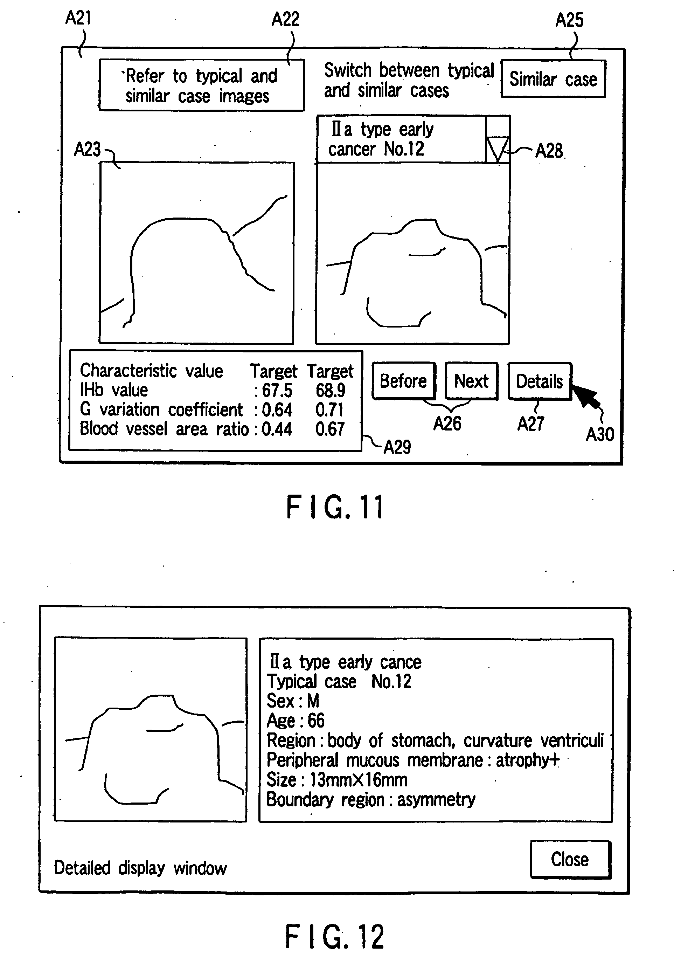

FIG. 11 shows an example of a diagnostic support content for providing diagnostic support by displaying images of typical and similar cases of suspected diagnosis result for comparison with an image of a case as a diagnosis target. FIG. 11 shows the contents of a display window presented to the doctor. This window is formed as a window that allows the doctor to interactively give instructions using an input means such as a mouse. As the contents shown in FIG. 11, the following are displayed in a display area A21: a diagnostic support content name A22, a diagnosis target image display area A23, a reference image display area A24, a button A25 for selecting the typical case or similar case as a reference image, selection buttons A26 for selecting and displaying consecutive images of a plurality of reference images, a details display button A27 for displaying the details of a reference image, a diagnosis name display/pull-down menu A28 for reference images, a comparative information display area A29 for various characteristic values of a diagnosis target image and reference image, and a cursor A30 for selecting each button and menu by mouse operation and clicking.

An image selected as a reference image is an image of a case based on the diagnosis results obtained from the above diagnostic support contents ① and ② or the diagnosis name manually designated by the doctor using the menu A28. In this case, the reference image assigned No. 12 corresponding to IIa type early stomach cancer is displayed. When the details display button A27 is clicked, the detailed display window of the reference image in FIG. 12 opens as another window to display various information. When a similar case image is selected as a reference image, a case image similar to the values of the respective characteristic values displayed in the comparative information display area A29 is retrieved and displayed.

In this manner, a typical case image or similar case image of a suspected disease is displayed to allow the doctor to compare and examine in making a diagnosis. This makes it possible to compensate for the differences in experience and knowledge between doctors and improve the accuracy of a diagnosis.

④ Display of Information such as Findings of Interest with respect to Suspected Disease

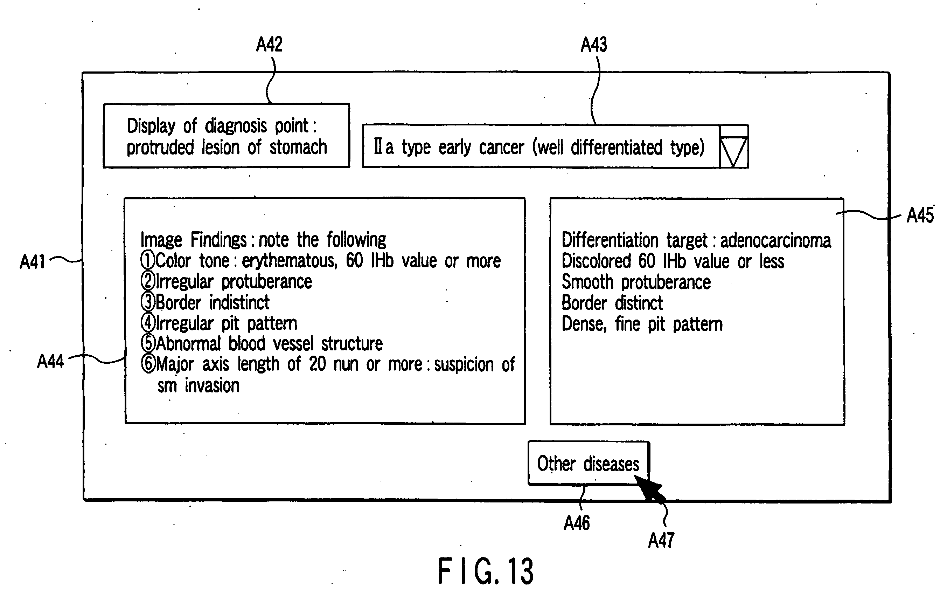

FIG. 13 shows an example of a diagnostic support content for providing diagnostic support in diagnosing a suspected disease with respect to a case as a diagnosis target by displaying findings of interest and information of differences from a disease as a differentiation target. FIG. 13 shows the contents of a display window presented to the doctor. This window is formed as a window that allows the doctor to interactively give instructions using an input means such as a mouse. As the contents shown in FIG. 13, the following are displayed in a display area A41: a diagnostic support content name A42, a diagnosis target disease name display/pull-down menu A43, a diagnosis target disease information display area A44, a differentiation target disease information area A45, a display information change button A46 for displaying another differentiation target disease information, and a cursor A47 for selecting each button and menu by mouse operation and clicking.

The information displayed in the diagnosis target disease information display area A44 is information concerning image findings which are important for a diagnosis with respect to the diagnosis results obtained by the above diagnostic support contents ① and ② and the diagnosis name manually designated by the doctor using the menu A43. In addition, similar information concerning another disease whose differentiation is important is displayed in the differentiation target disease information area A45. Differentiation target diseases are set for each diagnosis name. For IIa type early stomach cancer in this case, protruded lesions such as adenocarcinoma, hyperplastic polyp, and lymphoma are set as differentiation targets. Such pieces of finding information of interest are sequentially displayed by clicking the display information change button A46. By always presenting contents based on latest medical information as such pieces of information, the doctor can make a diagnosis with reference to these pieces of information as well as on the basis of his own memory and experience.

⑤ Display of Information such as Examination Items and Treatment Contents for Disease

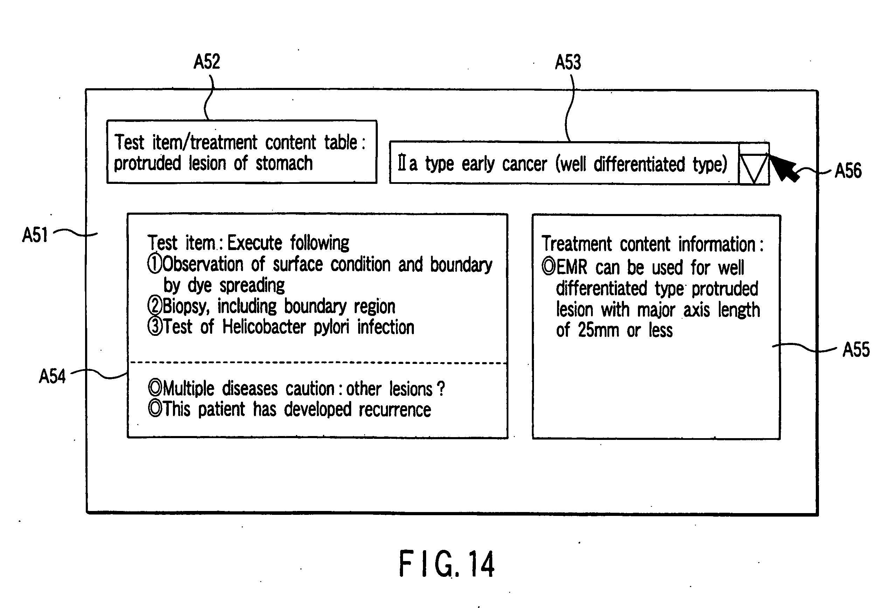

FIG. 14 shows an example of the diagnostic support content for providing diagnostic support for a case as a diagnosis target in a diagnosis of a suspected disease by displaying information concerning selection of an examination item to be executed and a suitable treatment. FIG. 14 shows the contents of a display window presented to the doctor. This window is formed as a window that allows the doctor to interactively give instructions using an input means such as a mouse. As the contents shown in FIG. 14, the following are displayed in a display area A51: a diagnostic support content name A52, a diagnosis target disease name display/pull-down menu A53, an examination content display area A54 for a diagnosis target disease, a treatment content display area A55 for the diagnosis target disease, and a cursor A56 for selecting a menu by mouse operation and clicking.

The information displayed in the examination content display area A54 is information concerning an examination item which is important for a diagnosis with respect to the diagnosis results obtained by the above diagnostic support contents ① and ② and the diagnosis name manually designated by the doctor using the menu A53. Information concerning treatment contents after diagnosis confirmation is displayed in the treatment content display area A55. By introducing latest findings as these pieces of information in accordance with medical advances, diagnostic support can be provided, which effectively assists the doctor's memory and experience.

The characteristic values and the like used for the above diagnostic support content can be changed, as needed, in accordance with modality and diagnosis purposes. The respective diagnostic support contents can be simultaneously executed. By simultaneously using a plurality of diagnostic support contents in, for example, a multiwindow form or by combinational display, more information can be presented.

The first embodiment will be described next with reference to the several views of the accompanying rendering. This embodiment relates to a diagnostic support apparatus which can selectively obtain diagnostic support information in accordance with examination purposes and types and allows the use of latest diagnostic support contents.

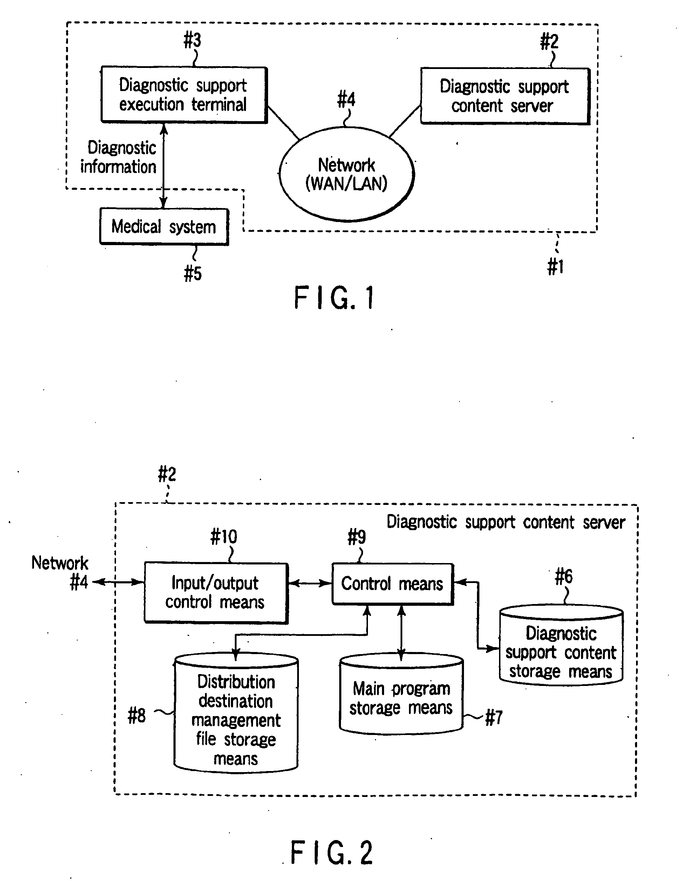

FIG. 1 shows a form of the diagnostic support system according to the first embodiment. Referring to FIG. 1, reference numeral #1 denotes a diagnostic support system according to the first embodiment; #2, a diagnostic support content server which distributes diagnostic support contents through a network #4 formed from a WAN (Wide Area Network) or LAN (Local Area Network); and #3, a diagnostic support execution terminal which is installed in a hospital, clinic, or the like to execute diagnostic support by using the diagnostic support contents distributed from the diagnostic support content server #2 and the diagnostic information obtained by a medical system #5.

In this embodiment, the diagnostic support content server #2 and diagnostic support execution terminal #3 are computers, each having a display means such as a CRT or LCD and input means such as a keyboard and mouse. For the sake of convenience, FIG. 1 shows an arrangement in which one each of the diagnostic support content server and diagnostic support execution terminal is connected to the network, but pluralities of such servers and contents may exist on the same network.

In the diagnostic support system, in order to ensure security, the diagnostic support content server #2 and diagnostic support execution terminal #3 can establish communication by transmitting and receiving authentication information such as the server name, facility name, IDs, and passwords.

FIG. 2 is a view for explaining the arrangement of the diagnostic support content server #2 in this embodiment. The diagnostic support content server #2 is constituted by a diagnostic support content storage means #6 which stores diagnostic support contents and diagnostic support content management information, a control means #9 which controls the operation of the diagnostic support content server #2, a main program storage means #7 which stores the main program to be executed by the control means #9, a distribution destination management file storage means #8 which specifies and authenticates the distribution destination of a diagnostic support content, and an input/output control means #10 which controls input/output operation through the network #4 in distribution of diagnostic support contents. The diagnostic support content storage means #6, main program storage means #7, and distribution destination management file storage means #8 use a hard disk connected to the computer that realizes the diagnostic support content server #2. The control means #9 is operated by executing the main program using the CPU and main memory.

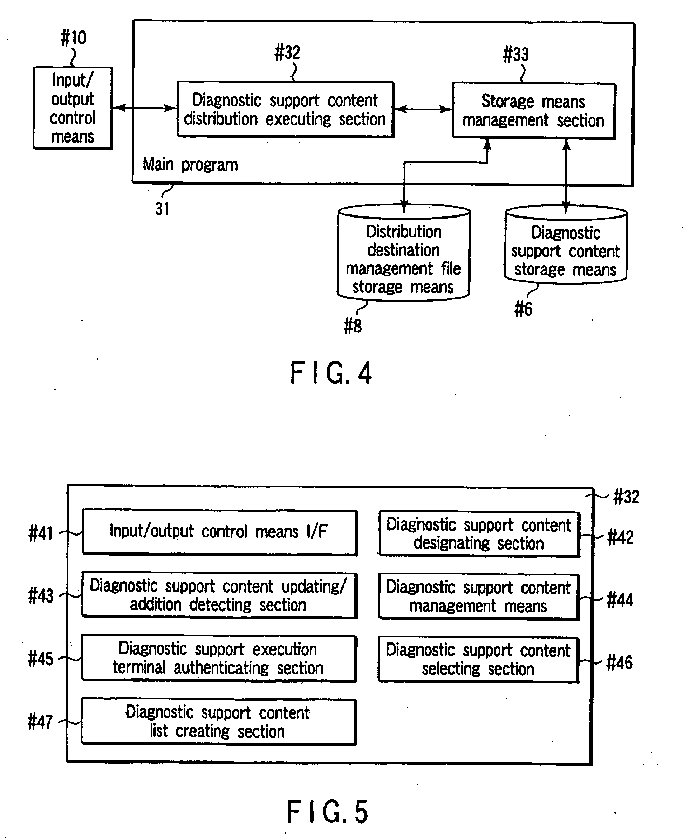

FIG. 4 is a block diagram of a main program #31 executed by the control means #9 of the diagnostic support content server #2 according to this embodiment. This program is constituted by a diagnostic support content distribution executing section #32 which executes a series of operations in distribution of diagnostic support contents, and a storage means management section #33 which controls a series of access operations accompanying retrievals, reads, and the like from the diagnostic support content storage means #6 and distribution destination management file storage means #8.

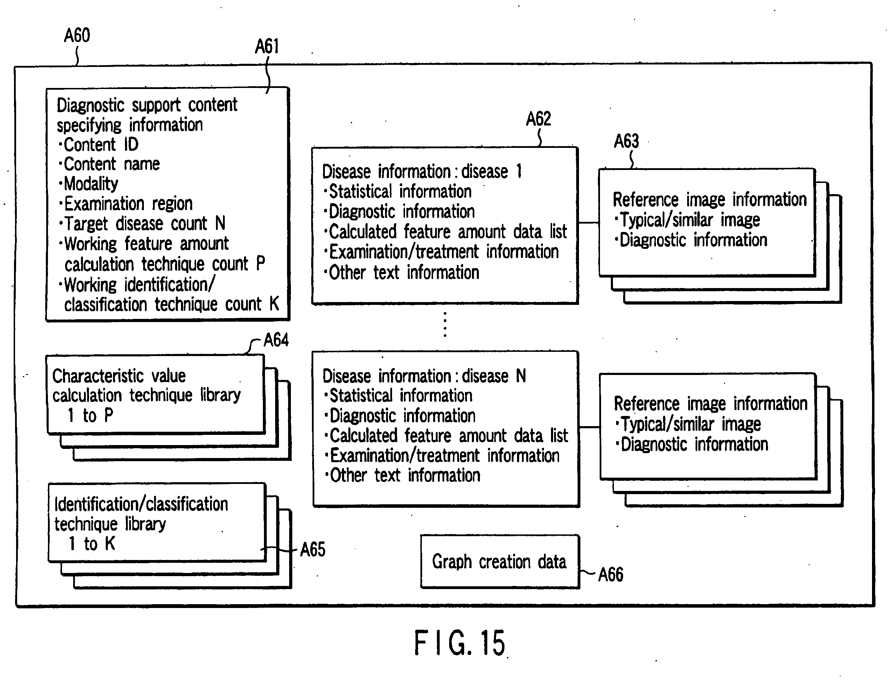

As described above, diagnostic support contents provide various diagnostic support for the diagnosis made by the doctor, and are formed like a diagnostic support content object A60 shown in FIG. 15. Referring to FIG. 15, the diagnostic support content object A60 is a software concept formed by combining various kinds of data and programs as needed, and is constituted by diagnostic support content specifying information A61 which includes an ID, name, and the like for specifying the diagnostic support content, disease information A62 which includes statistical information, diagnostic information, examination/treatment information, a plurality of cases, a characteristic value data list calculated from image data, and the like with respect to N disease types (N $\geqq$ 1) as diagnostic support targets, one or more pieces of reference image information A63 corresponding to each disease type, a characteristic value calculation library A64 for executing P types (P $\geqq$ 1) of characteristic value calculation techniques used for diagnostic support, an identification/ classification library A65 for executing K types (K $\geqq$ 1) identification/classification techniques, and graph creation data A66 which is referred to when a graph is created. The diagnostic support content object includes files and software libraries which implement a diagnostic support content. For example, the diagnostic support content is transmitted/ received, saved, and selected by using these files and software. In addition, updating/addition of a diagnostic support content include changes such as a partial version up with respect to the respective analysis techniques, statistical data, image data, and the like contained in the diagnostic support content. Only changed element items can be transmitted and received.

Note that the diagnostic support content object does not always have all the elements shown in FIG. 15, but is designed to use only elements necessary for diagnostic support information to be created.

As the diagnostic support content management information, in addition to the diagnostic support content specifying information such as the ID, name, and the like of the diagnostic support content object A60, date information such as creation date/update date, creator information, and other explanatory information are formed into a table and stored as a file.

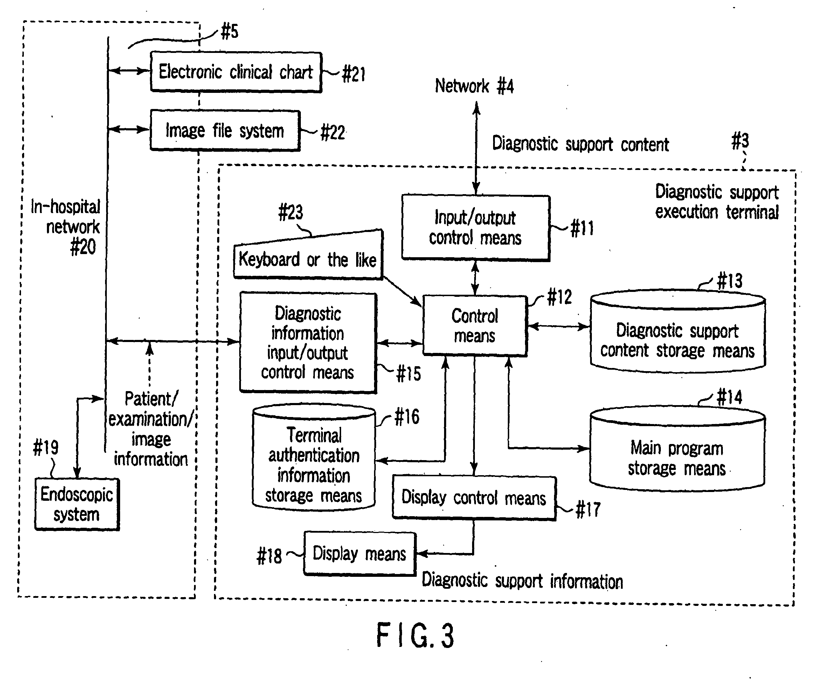

FIG. 3 is a view for explaining the arrangement of the diagnostic support execution terminal #3 in this embodiment. The diagnostic support execution terminal #3 is constituted by a control means #12 for controlling the operation of the diagnostic support execution terminal #3 and creating diagnostic support information, an input/output control means #11 for controlling communication input/output operation through the network #4, a main program storage means #14 for storing the main program to be executed by the control means #12, a diagnostic support content storage means #13 for storing distributed diagnostic support contents and diagnostic support content management information, a terminal authentication information storage means #16 for storing terminal authentication information, e.g., a network address, user name, and ID, which are used to specify the diagnostic support execution terminal #3, a diagnostic information input/output control means #15 for acquiring diagnostic information concerning a patient, examination, and image which is obtained from the medical system #5, a display control means #17 for controlling display of created diagnostic support information, a display means #18 for displaying the created diagnostic support information, and an external input means #23 such as a keyboard or mouse which is used to input an instruction or the like from the operator. Referring to FIG. 3, the medical system #5 is constituted by an electronic clinical chart #21 connected to an in-hospital network #20 formed from a LAN or the like, an image file system #22, and an endoscopic system #19 which is a modality for imaging in this embodiment. These medical systems #5 can exchange information with each other by using a common protocol such as DICOM3.0 which has recently been widely used. The diagnostic support content storage means #13, main program storage means #14, and terminal authentication information storage means #16 use a hard disk connected to the computer that implements the diagnostic support execution terminal #3. The control means #12 is operated by executing the main program using the CPU and main memory.

The operation of the diagnostic support execution terminal #3, accompanying the creation and display of diagnostic support information, will be described next.

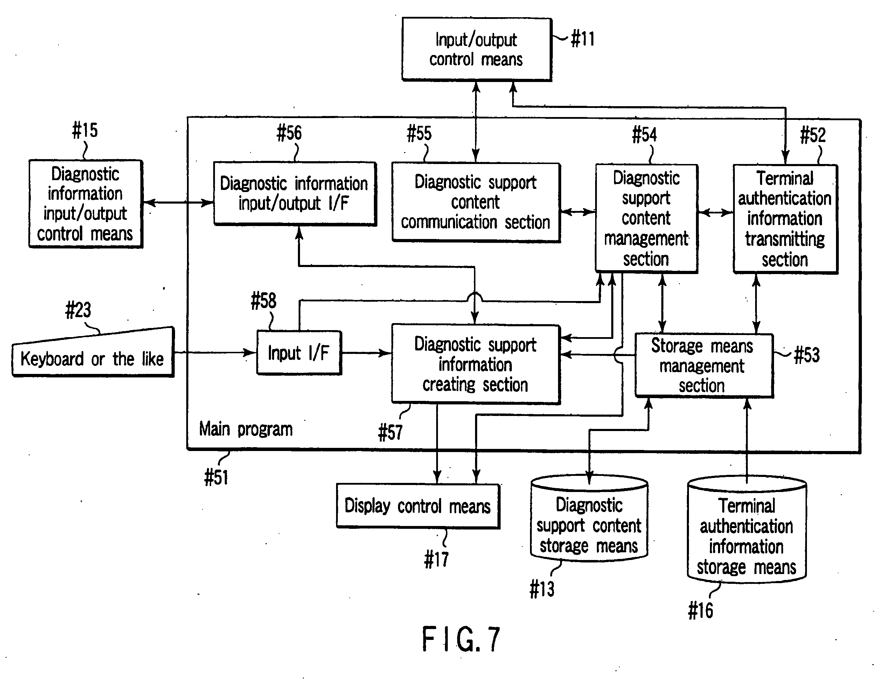

FIG. 7 is a block diagram of a main program #51 executed by the control means #12 of the diagnostic support execution terminal #3 according to this embodiment. This program includes a storage means management section #53 which controls a series of access operations accompanying storage, retrievals, reads, and the like with respect to the diagnostic support content storage means #13, a diagnostic information input/output I/F #56 serving as an interface for inputting/outputting diagnostic information constituted by patient information, examination information, and image information input through the diagnostic information input/output control means #15, an input I/F #58 serving as an interface for inputting from the external input means #23 such as a keyboard or mouse, a diagnostic support information creating section #57 which creates diagnostic support information using the input diagnostic information and diagnostic support content, a terminal authentication information transmitting section #52 which transmits terminal authentication information to the diagnostic support content server #2, the storage means management section #53 which controls a series of access operations accompany storage, retrievals, reads, and the like with respect to the terminal authentication information storage means #16 and diagnostic support content storage means #13, a diagnostic support content communication section #55 which performs a series of communication activities with the diagnostic support content server, accompanying reception of a diagnostic support content, and a diagnostic support content management section #54 which stores the received diagnostic support content in the diagnostic support content storage means #13 through the storage means management section #53, and updates the diagnostic support content management information.

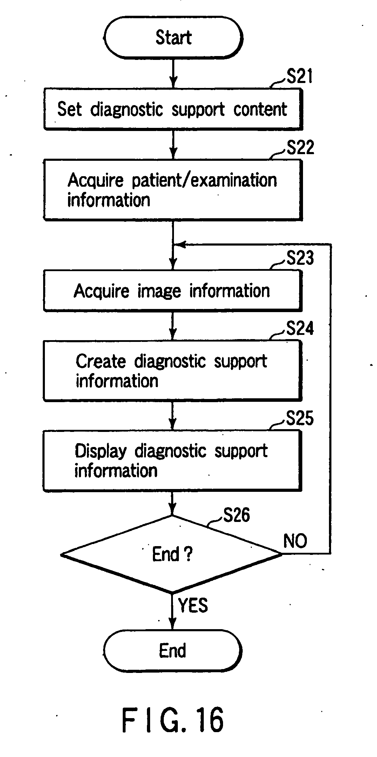

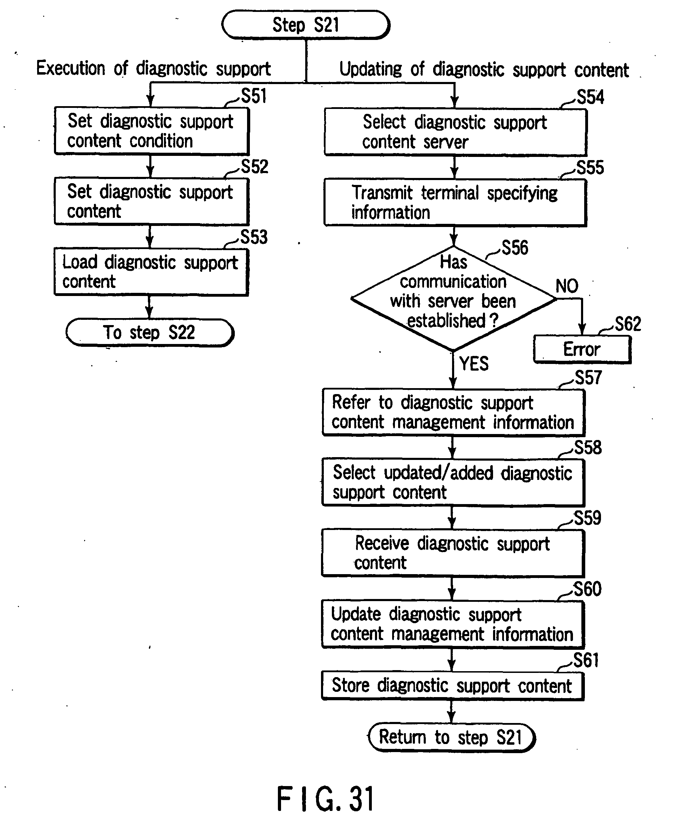

FIGS. 16 and 31 are flowcharts for explaining a series of operations accompanying the creation of diagnostic support information and updating/addition of a diagnostic support content by the diagnostic support execution terminal #3 according to this embodiment. Assume that in creating diagnostic support information at the time of an examination, diagnostic support information is created and displayed on the display means #18 upon reception of an image as a trigger from the endoscopic system #19 connected to the diagnostic support execution terminal #3 on the basis of the arrangement shown in FIG. 3.

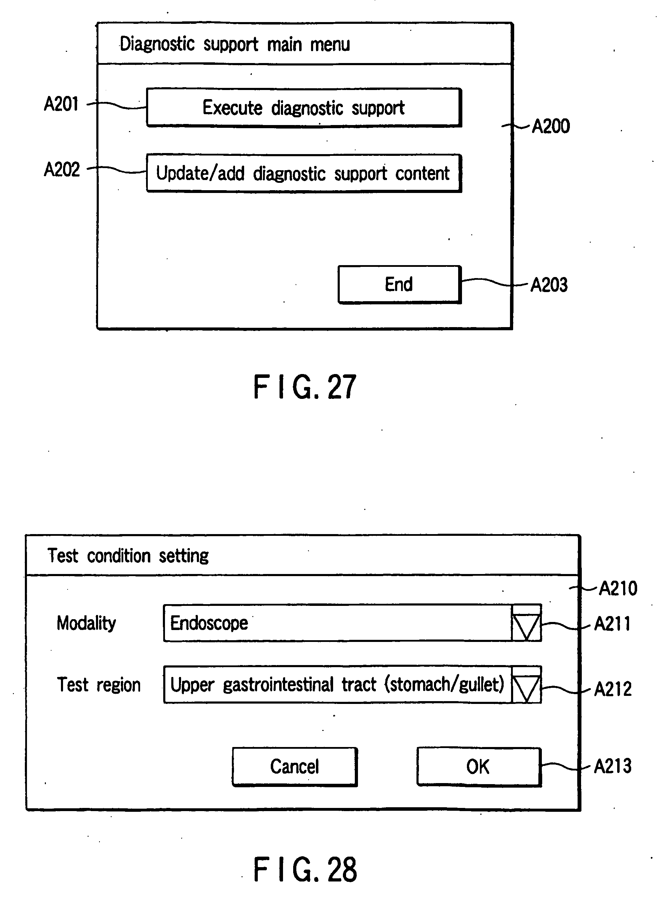

Referring to FIG. 16, in step S21, a diagnostic support content to be executed is set or updating/addition of a diagnostic support content is selected. More specifically, the main program #51 displays a diagnostic support main menu window A200 shown in FIG. 27 on the display means #18. The main menu window A200 has a diagnostic support execution button A201 for executing diagnostic support in an examination, a diagnostic support content change/add button A202 for updating/adding a diagnostic support content through communication with the diagnostic support content server #2, and an end button A203 for ending the operation of the diagnostic support execution terminal. When each button is selected by using the external input means #23 such as a keyboard or mouse, the flow advances to the next operation step. In the main menu window A200, if the diagnostic support execution button A201 is selected, the flow advances to step S51 in FIG. 31. If the diagnostic support content change/add button A202 is selected, the flow advances to step S54 in FIG. 31. The following description is based on the assumption that the diagnostic support execution button A201 is selected.

In step S51, examination condition settings for a diagnostic support content to be executed are made. The contents of the condition settings include items associated with an examination purpose and type. In this embodiment, they are the type of modality as equipment to be used for the examination and an examination region. The condition settings are made in an examination condition setting window A210 shown in FIG. 28 which is displayed on the display means #18, and a modality selection menu A211 and examination region menu A212 which are pull-down menus are used. In each menu, the previously set condition is displayed as an initial value, and the setting is changed, as needed, by operating the external input means #23. After the condition setting, an OK button A213 is selected, and the flow advances to step S52.

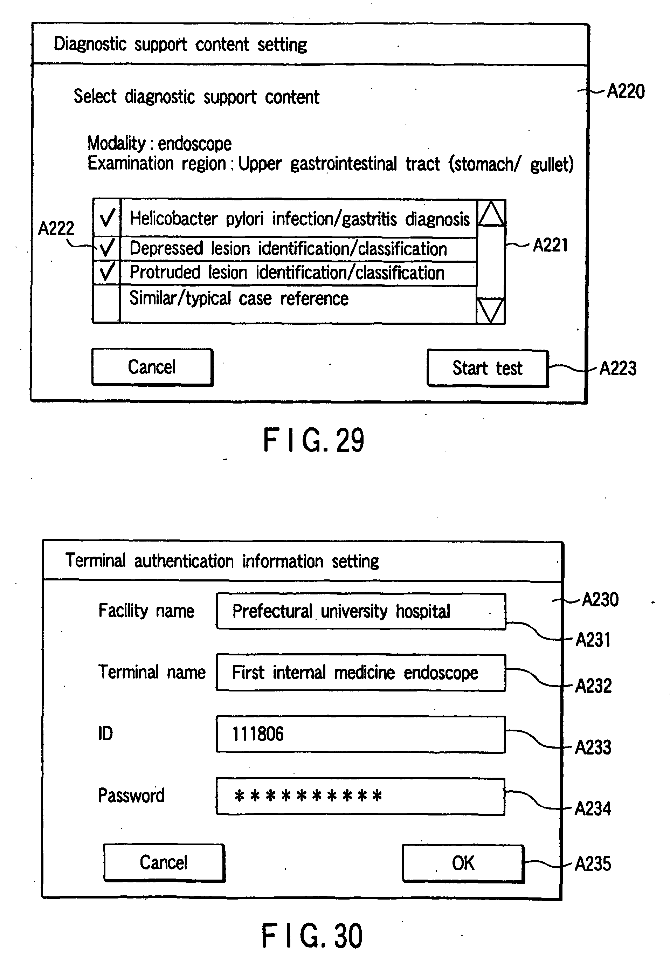

In step S52, a diagnostic support content setting window A220 is displayed, which is shown in FIG. 29 and used to select and set a diagnostic support content in accordance with the set modality type and examination region. The diagnostic support content setting window A220 includes a diagnostic support content menu A221 which displays a list of diagnostic support contents that can be applied to the diagnostic support execution terminal #3 in accordance with the conditions set in the step S51, and a selected state display area A222 which indicates the selected/non-selected state of each diagnostic support content. A desired diagnostic support content is selected/non-selected (which is toggled with clicking of a mouse or the like) by operating the external input means #23 in the diagnostic support content menu A221. As an initial value, the selected state in the previous examination is reproduced. After a diagnostic support content is set, an examination start button A223 is selected. The flow advances to step S53.

In step S53, the set diagnostic support content is loaded (prepared). The main program #51 reads out a diagnostic support content object corresponding to the set diagnostic support content from the diagnostic support content storage means #13, loads necessary data, and links a characteristic value calculation technique library and identification/classification library to be used (They are implemented by a plug-in technique. Plug-in is a known technique generally used in Internet browsers and the like, and hence a detailed description will be omitted.), thereby completing preparations for the operation of the diagnostic support information creating section #57. The flow then advances to step S22 in FIG. 16.

In step S22, patient/examination information used for the set diagnostic support content is acquired from the endoscopic system #19, the electronic clinical chart #21 connected to the in-hospital network #20, the image file system #22, or the like.

In step S23, as an input is input from the endoscopic system #19, image information is acquired.

In step S24, the diagnostic support information creating section #57 creates diagnostic support information on the basis of the patient/examination information acquired in step S22 and the image information acquired in step S23. The diagnostic support information creating section #57 executes processing required to create the respective pieces of information shown in diagnostic support content examples ① to ⑤, e.g., calculation of characteristic values corresponding to diagnostic support contents, execution of identification/classification processing, and creation of statistical information/graphs, and creates a display window.

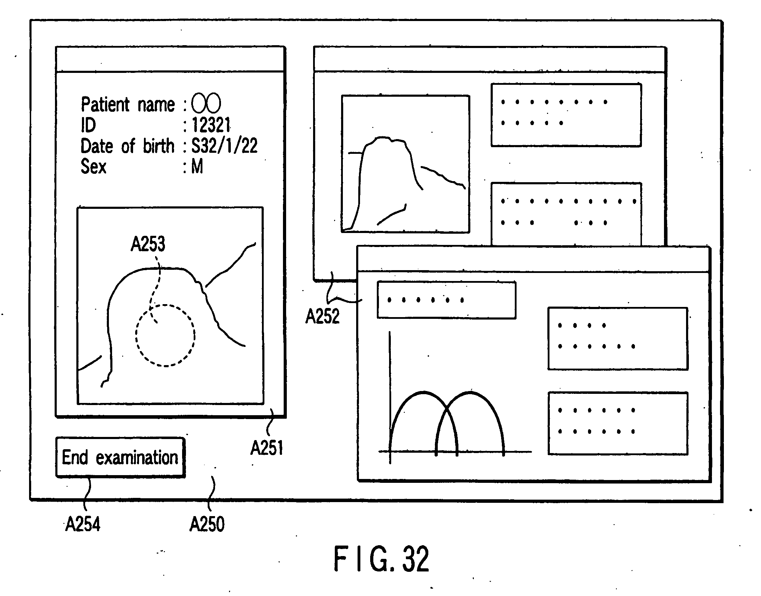

In step S25, the created diagnostic support information is displayed on the display means #18. FIG. 32 shows a display example of a diagnostic support execution window. A diagnostic support execution window A250 is formed as a multi-window display window and includes a patient/examination/image information display window A251 for displaying patient/examination information, the original image input from the endoscopic system #19, and the like, and one or more diagnostic support information display windows A252 for displaying diagnostic support information based on the set diagnostic support content. In addition, region-of-interest information A253 indicating a region of interest in which a characteristic value is to be calculated by using an image analysis technique is superimposed on the original image displayed in the patient/examination/image information display window A251. The doctor conducts an examination by referring to these pieces of patient/examination/image information and diagnostic support information.

If it is determined in step S26 that diagnostic support is terminated, the processing is terminated. Otherwise, step S23 and the subsequent steps are repeated with respect to the next image information input from the endoscopic system #19. When this processing is to be terminated, an examination end button A254 in the diagnostic support execution window A250 is selected. The main program #51 displays the diagnostic support main menu window A200 again to prepare for the next examination or the like.

Operation to be done when the diagnostic support content change/add button A202 is selected in the diagnostic support main menu window A200 in step S21 will be described next. The main program #51 starts updating/adding a diagnostic support content by performing a series of operations including communication with the diagnostic support content server #2.

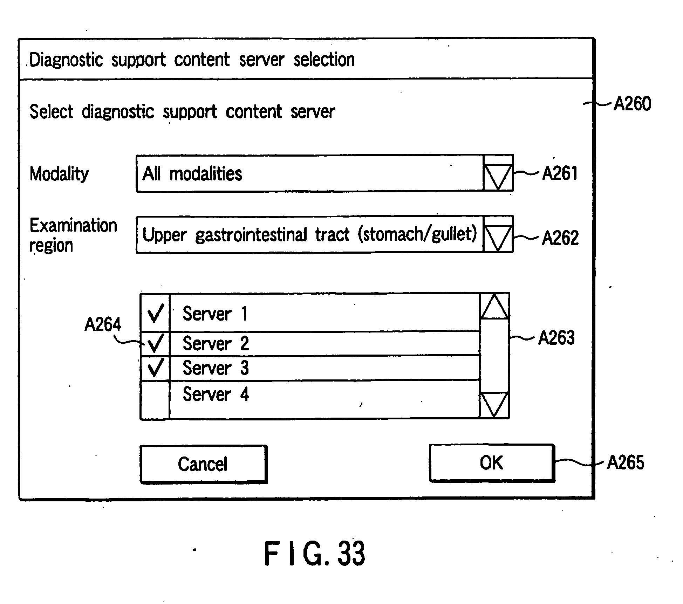

First of all, in step S54, a diagnostic support content server is selected. A diagnostic support content server is selected in a diagnostic support content server selection window A260 in FIG. 33. The diagnostic support content server selection window A260 includes a modality menu A261 and examination region menu A262 which are pull-down menus for selecting a modality and examination region as conditions for a diagnostic support content, a diagnostic support content menu A263 which displays a list of diagnostic support content servers, and a selected state display area A264 which indicates the selected/non-selected state of each diagnostic support content server. When an OK button A265 is selected after the respective menus are set by operating the external input means #23, the flow advances to step S55.

In step S55, self-terminal authentication information is transmitted to the selected diagnostic support content server. In this case, a terminal authentication information setting window A230 shown in FIG. 30 is used. The terminal authentication information setting window A230 includes a facility name input button A231, terminal name input box A232, ID input box A233, and password input box A234 which are respectively used to input a facility name, terminal name, ID, and password. Such pieces of information are input by using the external input means #23. These pieces of information are stored as terminal authentication information in the terminal authentication information storage means #16, and are set as initial values in the input boxes except for a password. When an OK button A235 is selected after each information is input, the main program #51 transmits the information to the diagnostic support content server #2 through the input/output control means #11, and acquires a terminal authentication result. The diagnostic support content server #2 collates the terminal authentication information stored in the distribution destination management file storage means #8 with the received terminal authentication information to determine whether to allow establishment of communication, and transmits the result. If establishment of communication is allowed, communication concerning updating/addition of a diagnostic support content with the diagnostic support execution terminal #3 is established. If establishment of communication is inhibited, a message indicating the reason is transmitted.

If it is determined in step S56 that the diagnostic support execution terminal #3 has been normally authenticated by the diagnostic support content server #2 and communication has been established, the flow advances to step S57. If communication cannot be established for some reason (a problem in a communication line, the expiration of a password, or the like), the flow advances to step S62 to display an error together with the received message.

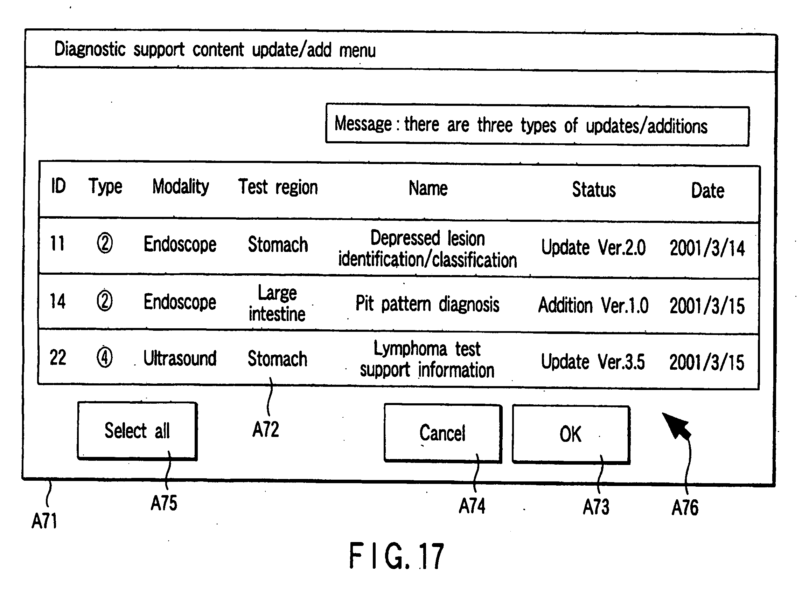

In step S57, after the diagnostic support content management information held in the diagnostic support content server #2 is acquired, the diagnostic support content management information for specifying a diagnostic support content for which updating/addition is to be performed is referred to. The main program #51 generates, through the diagnostic support content communication section #55, a request to acquire the diagnostic support content list information stored in the diagnostic support content storage means #6 of the diagnostic support content server #2. The list information conforms to the diagnostic support content management information held in the diagnostic support content server #2 and includes the following information in the form of a list: diagnostic support content specifying information such as the ID and name of the diagnostic support content, date information such as creation/update date, creator information, and other explanatory information. The diagnostic support content server #2 creates diagnostic support content list information using a diagnostic support content list creating section #47, and transmits it to the diagnostic support execution terminal #3. On the basis of comparison between the stored diagnostic support content management information and the received diagnostic support content list information, the diagnostic support content management section #54 selects a diagnostic support content for which updating, addition, or the like has been done. An update/add menu window A71 for updating and addition of data for the diagnostic support content shown in FIG. 17 is created with respect to the selected diagnostic support content, and is displayed on the display means #18. Referring to FIG. 17, the update/add menu window A71 includes a menu area A72 for displaying a list of updated and added diagnostic support contents and selecting a diagnostic support content therefrom, a cancel button A74, an OK (start) button A73, an all selection button A75 for setting all the diagnostic support contents in the menu area A72 in the selected state, and a mouse cursor A76 for selecting a menu and clicking a button.

In step S58, a desired diagnostic support content is selected from the menu area A72 or the all selection button A75 is selected to set all the diagnostic support contents in the selected state.

In step S59, the OK button A73 in the menu window A71 in FIG. 17 is selected to cause the diagnostic support content communication section #55 to transmit diagnostic support content specifying information such as the ID and name of the selected diagnostic support content to the diagnostic support content server #2 and generate a transmission request. Upon reception of this request, the diagnostic support content server #2 transmits the requested diagnostic support content.

After the diagnostic support content is completely received, the diagnostic support content management section #54 updates the diagnostic support content management information and stores the updated information in the diagnostic support content storage means #13 together with the received diagnostic support content in step S60. The processing is then terminated, and the flow returns to step S21.

As described above, according to the diagnostic support apparatus of the first embodiment, the diagnostic support execution terminal #3 can selectively obtain diagnostic support information in accordance with an examination purpose and type, and a latest diagnostic support content can be used.

[(1-B)th Embodiment]

A diagnostic support apparatus according to the (1-B)th embodiment will be described next with reference to the views of the accompanying rendering. This embodiment relates to a diagnostic support apparatus which allows a diagnostic support execution terminal #3 to always use a latest diagnostic support content.

The form of the diagnostic support apparatus according to this embodiment is the same as that of the diagnostic support apparatus according to the first embodiment shown in FIG. 1. In addition, a diagnostic support content server #2 and the diagnostic support execution terminal #3 have the same arrangements as those in the first embodiment, and different operation is implemented by changing main programs #31 and #51 for the respective operations.

In this embodiment, upon detecting that a diagnostic support content is updated or added, the diagnostic support content server #2 distributes the updated or added diagnostic support content to the predetermined diagnostic support execution terminal #3.

The operation of the diagnostic support content server #2 which is associated with diagnostic support content distribution in this embodiment will be described with reference to FIGS. 4, 5, and 6. FIG. 5 is a block diagram showing a diagnostic support content distribution executing section #32 in more detail, which is comprised of an input/output control means I/F #41 which serves as an interface with an input/output control means #10 to communicate with the diagnostic support execution terminal #3 through a network #4, a diagnostic support content designating section #42 which designates diagnostic support content distribution, a diagnostic support content updating/addition detecting section #43 which detects updating/addition of a diagnostic support content stored in a diagnostic support content storage means #6, a diagnostic support content management means #44 for managing the diagnostic support content management information stored in the diagnostic support content storage means #6, a diagnostic support execution terminal authenticating section #45 which specifies and authenticates a diagnostic support execution terminal as a distribution destination, a diagnostic support content selecting section #46 which selects and designates a diagnostic support content to be distributed, and a diagnostic support content list creating section #47 which creates information of a list of diagnostic support contents that can be distributed.

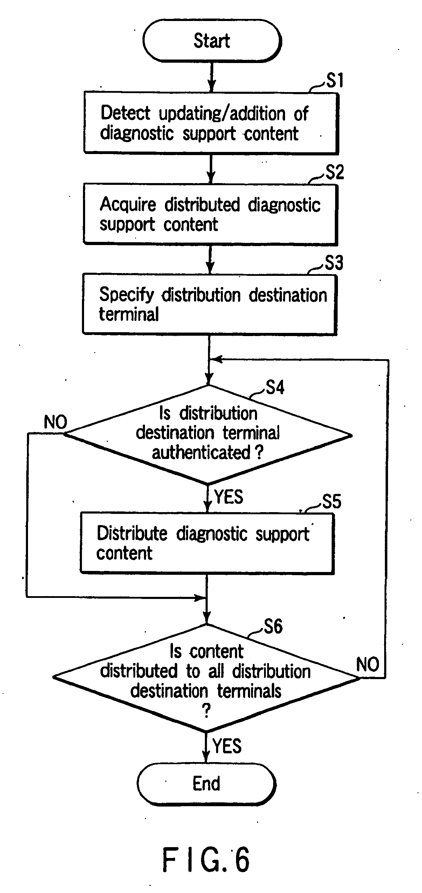

FIG. 6 is a flowchart for explaining a series of operations in distributing a diagnostic support content in this embodiment. In step S1, the diagnostic support content updating/addition detecting section #43 detects that the diagnostic support content stored in the diagnostic support content storage means #6 are updated or added. This detection is executed by making the diagnostic support content updating/addition detecting section #43 refer to the diagnostic support content management information acquired by the diagnostic support content management means #44 and detect a change in the date information of the diagnostic support content or the contents of file information. On the basis of the detection result, the diagnostic support content updating/addition detecting section #43 notifies the diagnostic support content designating section #42 of the occurrence of the diagnostic support content to be distributed.

In step S2, the updated/added diagnostic support content is acquired. In this case, the diagnostic support content selecting section #46 selects the updated/added diagnostic support content, and acquires the content from the diagnostic support content storage means #6 through a storage means management section #33.

In step S3, a diagnostic support execution terminal as a distribution destination is specified. In this case, the diagnostic support execution terminal authenticating section #45 accesses a distribution destination management file storage means #8 through the storage means management section #33 to acquire terminal specifying information such as the network address of the diagnostic support execution terminal as the distribution destination, a facility name, and a password.

In step S4, the distribution destination terminal is authenticated by using the distribution destination terminal authentication information obtained in step S3. In this case, the diagnostic support execution terminal authenticating section #45 compares the distribution destination terminal authentication information with the terminal authentication information of the diagnostic support execution terminal #3 which is obtained through the input/output control means I/F #41 and network #4. If it is determined upon completion of authentication that distribution can be done, the flow advances to step S5. If the terminal authentication information cannot be recognized for some reason and it is determined that distribution is inhibited, the flow advances to step S6.

In step S5, the diagnostic support content is distributed through the input/output control means I/F #41 in accordance with an instruction from the diagnostic support content designating section #42.

In step S6, the diagnostic support content designating section #42 checks whether the processing is completed with respect to all the diagnostic support execution terminals as the distribution destinations specified in step S3. If YES in step S6, the series of operations is terminated. If NO in step S6, the series of operations in steps S4 to S6 is executed again.

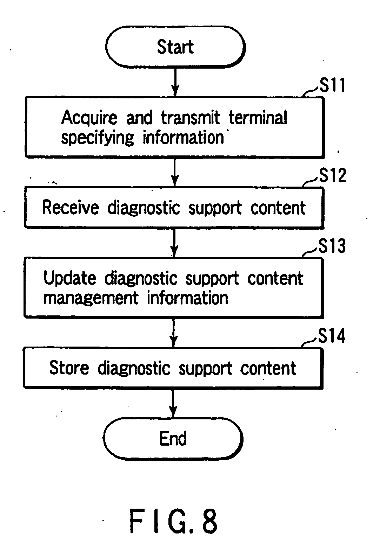

The operation of the diagnostic support execution terminal #3, accompanying the distribution of a diagnostic support content, will be described next with reference to FIGS. 7 and 8.

FIG. 8 is a flowchart for explaining a series of operations of the diagnostic support execution terminal #3, accompanying the reception of the diagnostic support content, in this embodiment. Also refer to FIG. 6 in association with the operation of the diagnostic support content server #2.

Accompanying the distribution of a diagnostic support content by the diagnostic support content server #2 shown in FIG. 6, processing is started upon communication establishment in the distribution destination terminal specifying processing in step S3. In step S11 in FIG. 8, a terminal authentication information transmitting section #52 acquires the terminal authentication information stored in a terminal authentication information storage means #16 by instructing a storage means management section #53 to acquire terminal authentication information, and transmits the information to the diagnostic support content server #2 through an input/output control means #11.

In step S4 in FIG. 6, the terminal is authenticated as a distribution destination terminal. The flow then advances to step S5 to start transmitting the diagnostic support content. The operation of the diagnostic support content server #2 at this time corresponds to the reception of the diagnostic support content in step S12. A diagnostic support content communication section #55 operates through the input/output control means #11.

In step S13, a diagnostic support content management section #54 updates the diagnostic support content management information. In step S14, the diagnostic support content management section #54 stores the diagnostic support content and diagnostic support content storage information in a diagnostic support content storage means #13, thus terminating the series of operations.

As described above, according to the diagnostic support apparatus of the (1-B)th embodiment, as a diagnostic support content in the diagnostic support content server #2 is updated/added, the content is distributed to the diagnostic support execution terminal #3. This makes it possible to always use a latest diagnostic support content for an examination.

[(1-C)th Embodiment]

A diagnostic support apparatus according to the (1-C)th embodiment will be described next with reference to the several views of the accompanying rendering. This embodiment relates to a diagnostic support apparatus which allows a diagnostic support execution terminal #3 to always use a latest diagnostic support content with ease. More specifically, the diagnostic support execution terminal #3 makes an inquiry as to whether a diagnostic support content is updated or added, and generates a transmission request if a content is updated or added.

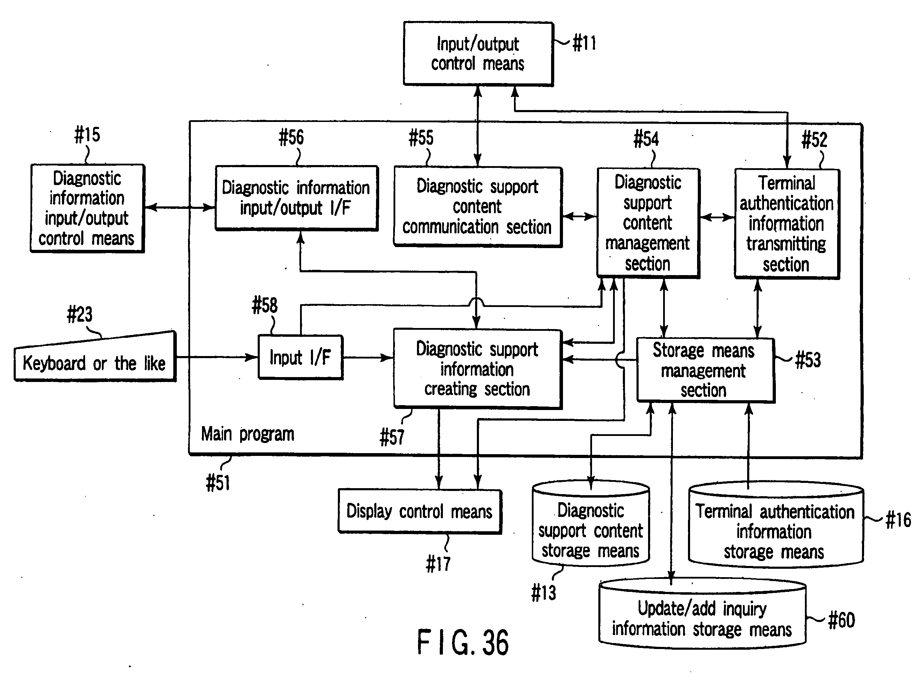

The form of the diagnostic support apparatus according to this embodiment is the same as that of the diagnostic support according to the first embodiment shown in FIG. 1. In addition, a diagnostic support content server #2 has the same arrangement as that in the first embodiment, and different operation is implemented by changing a main program #31. Although the diagnostic support execution terminal #3 has almost the same arrangement as that in the first embodiment except that in addition to the constituent elements shown in FIG. 7, the terminal further includes an update/add inquiry information storage means #60 for storing, for example, a condition setting file for inquiring the diagnostic support content server #2 whether a diagnostic support content is updated/added, as shown in FIG. 36. By changing a main program #51, predetermined operation can be implemented.

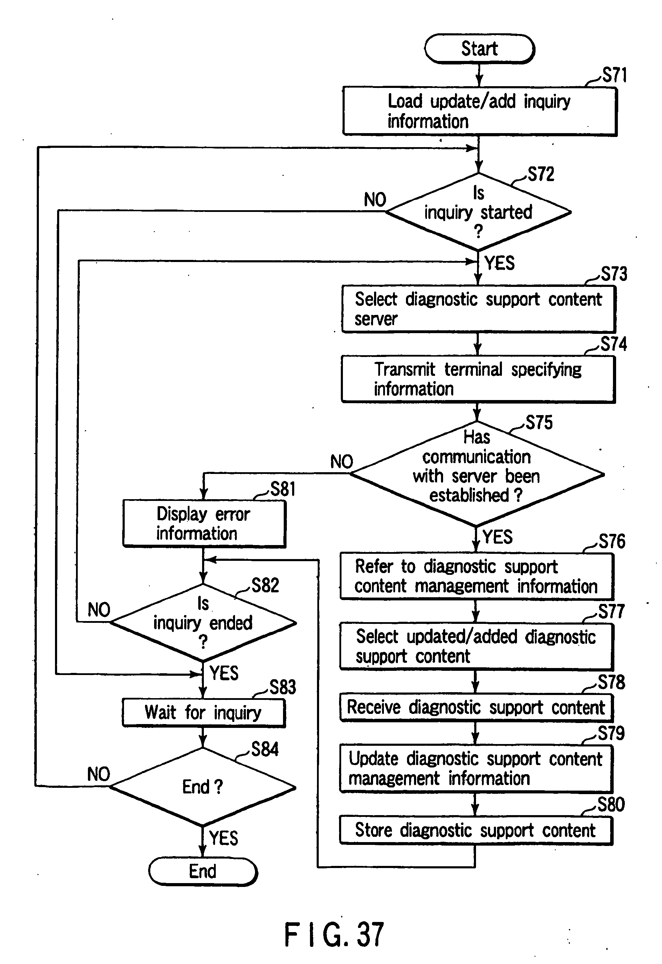

FIG. 37 is a flowchart for explaining the operation of the diagnostic support execution terminal #3 which is associated with updating/addition of a diagnostic support content in this embodiment. When the diagnostic support execution terminal #3 is started (the power is turned on to start the main program), each of the following processes is executed by the main program #51. The operation is mainly performed by a diagnostic support content management section #54.

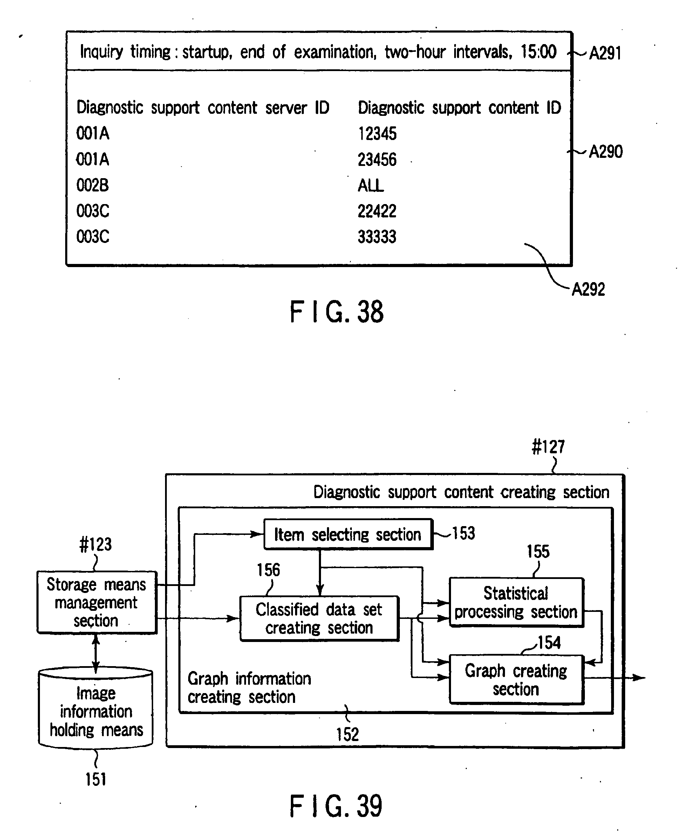

First of all, in step S71, an update/add inquiry information file in which various setting information concerning updating/addition of a diagnostic support content with respect to the diagnostic support content server #2 is written is acquired from the update/add inquiry information storage means #60 through a storage means management section #53. FIG. 38 shows an example of the contents of the add/update inquiry information file. Referring to FIG. 38, an update/add inquiry information file A290 includes timing setting information A291 for setting a specific timing at which an inquiry is made to the diagnostic support content server #2, and content setting information A292 for setting the execution of an inquiry about a specific diagnostic support content with respect to a specific diagnostic support content server. An inquiry timing is set to, for example, the time of occurrence of some event such as startup or end of an examination, a periodic time setting such as two-hour intervals, or a specific timing setting such as 15:00. Content setting information is set such that information such as an ID for specifying a diagnostic support content server is associated with information such as an ID for specifying a diagnostic support content, and if "ALL" is set, all the diagnostic support contents are set as inquiry targets. In addition, information representing a modality as a target for a diagnostic support content, an examination region, and the like can be used. Information such as "no inquiry should be made during an examination" can also be set as a flag. Note that an update/add inquiry information file is created and edited using a setting window and text editor (not shown) .

In step S72, it is checked whether an inquiry to the diagnostic support content server #2 is started. If, for example, the inquiry timing is set to the time of startup, the flow immediately advances to step S73. If the time setting or the like indicates that the current time is not the timing of starting an inquiry, the flow advances to step S83.

In step S73, the diagnostic support content server #2 is selected on the basis of the content setting information loaded in step S71. In the case shown in FIG. 38, since three diagnostic support content servers are set, inquires are sequentially made to the respective servers.

In steps S74 and S75, communication with the target diagnostic support content server is established by processing similar to that in each of steps S55 and S56 described in association with the operation of the diagnostic support execution terminal #3 in the first embodiment. If communication establishment fails, the flow advances to step S81. Otherwise, the flow advances to step S78.

In step S81, error information including a message concerning the communication establishment error transmitted from the diagnostic support content server and the like is displayed on a display means #18, and an error log file is output, as needed. The flow then advances to step S82.

In step S76, a diagnostic support content which has been updated/added is confirmed by the same processing as that in step S57 in the first embodiment.

In step S77, by further referring to the diagnostic support content which is recognized as an updated/added content in step S76 and the update/add inquiry information file, the diagnostic support content to be distributed by the diagnostic support content server #2 is selected.

In steps S78, S79, and S80, processing similar to that in steps S59, S60, and S61 described in the first embodiment is performed to receive the diagnostic support content and store the diagnostic support content management information in a diagnostic support content storage means #13 upon updating the information.

In step S82, it is checked whether inquires to all the set diagnostic support content servers #2 are completed. If NO in step S82, the flow returns to step S73 to repeat the subsequent processing. If YES in step S82, the flow advances to steps S83 and S84.

In step S83, the diagnostic support execution terminal #3 is set in the standby state with respect to an inquiry to the diagnostic support content server #2. The diagnostic support content management section #54 repeats end determination in step S84 and inquiry start determination in step S72 at, for example, periodic intervals by using time information from the system clock and OS. During this period, in practice, the diagnostic support execution terminal #3 operates to provide diagnostic support information in an examination. If an instruction to end the main program #51 is issued, the operation is terminated through the determination in step S84.

At the set inquiry timing, a series of operation in step S73 and the subsequent steps is executed.

As described above, according to the diagnostic support apparatus of the (1-C)th embodiment, a latest diagnostic support content can always be used by inquiring a diagnostic support content server about updating/addition of a diagnostic support content on the basis of set information.


[Second Embodiment]

The second embodiment will be described with reference to the several views of the accompanying rendering. This embodiment will exemplify a diagnostic support apparatus which allows many medical facilities/organizations to freely create a diagnostic support content, allows the wide use of various medical information, image data, and expert medical knowledge accumulated in the respective facilities/organizations on diagnostic support apparatuses, and can improve the performance of the diagnostic support apparatus by effectively using case data dispersed in many medical facilities/organizations because, for example, data can be easily added to created a diagnostic support content.

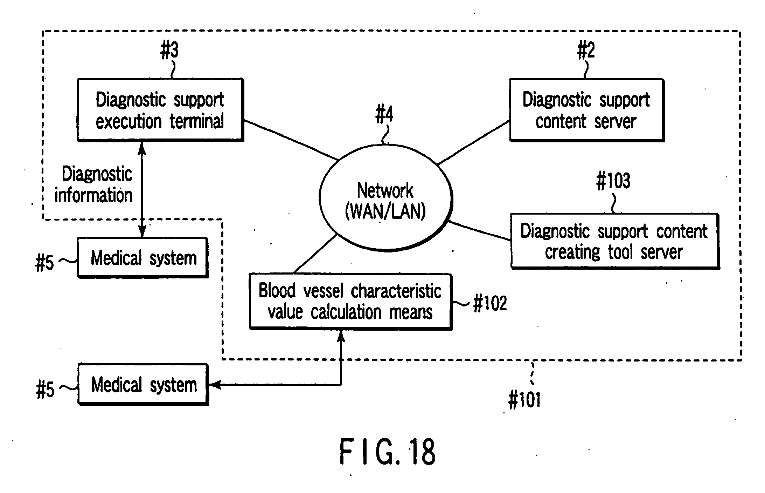

FIG. 18 shows a form of the diagnostic support system according to the second embodiment. Referring to FIG. 18, reference numeral #101 denotes a diagnostic support system according to the second embodiment. Reference numerals #2 to #5 denote the same constituent elements as in the first embodiment shown in FIG. 1. This embodiment further includes a diagnostic support content creating terminal #102 for creating a diagnostic support content distributed by the diagnostic support content server #2 and used by the diagnostic support execution terminal #3, and a diagnostic support content creating tool server #103 which provides a diagnostic support content creating tool for creating a diagnostic support content.

The diagnostic support content creating terminal #102 and diagnostic support content creating tool server #103 are also computers, each having a display means such as a CRT or LCD and input means such as a keyboard and mouse. For the sake of convenience, FIG. 18 shows an arrangement in which one each of the diagnostic support content server, diagnostic support execution terminal, diagnostic support content creating terminal, and diagnostic support content creating tool server is connected to the network, but pluralities of such servers and terminals may exist on the same network.

The diagnostic support content creating terminal #102 is installed in a hospital/clinic or medical institute to create a diagnostic support content using the diagnostic information obtained by the medical system #5 connected to the LAN, as in the case of the diagnostic support execution terminal #3, and transmit the content to the diagnostic support content server #2. In addition, the diagnostic support content creating terminal #102 receives a diagnostic support content that have already existed in the diagnostic support content server #2, updates/improves the content by, for example, adding new data or disease information as a diagnosis target, and transmits the resultant content to the diagnostic support content server #2.

The diagnostic support content creating tool server #103 provides various types of image processing/analysis/characteristic value calculation techniques, identification/classification techniques such as a discrimination function and neural network, statistical test techniques such as $t$ test, various multivariate analysis techniques, graph creating tools, and the like in the form of software libraries, which are used by the diagnostic support content creating terminal #102 to create a diagnostic support content.

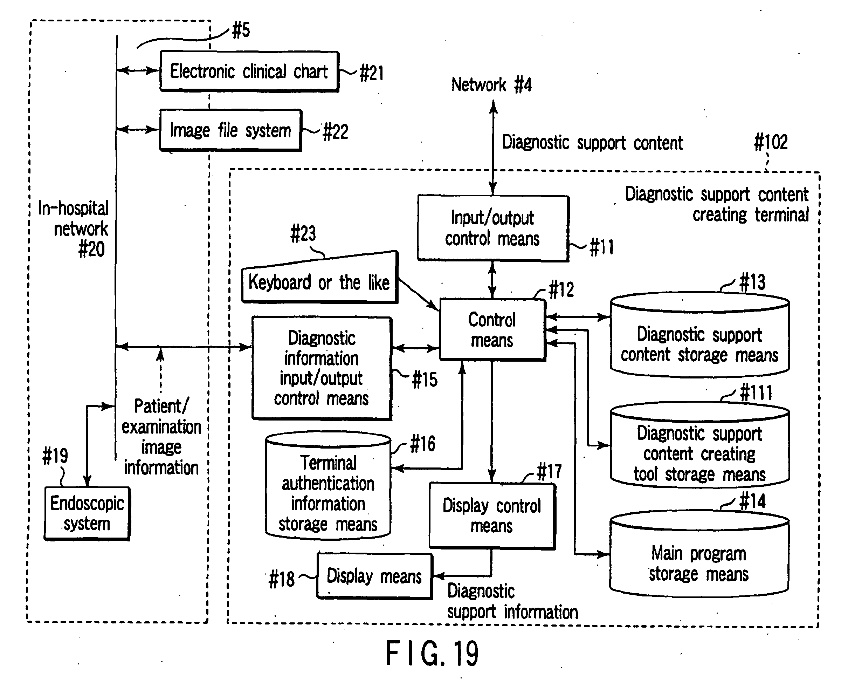

FIG. 19 is a view for explaining the arrangement of the diagnostic support content creating terminal #102 in this embodiment. The arrangement of the diagnostic support content creating terminal #102 is almost the same as that of the diagnostic support execution terminal #3. The same reference numerals as in FIG. 19 denote the same constituent elements in FIG. 3. The diagnostic support content creating terminal #102 further includes a diagnostic support content creating tool storage means #111 which stores the above diagnostic support content creating tool. The diagnostic support content creating tool storage means #111 uses the hard disk connected to the computer realizing the diagnostic support content creating terminal #102, as well as a diagnostic support content storage means #13, main program storage means #14, and terminal authentication information storage means #16.

The operation of creating a diagnostic support content in the diagnostic support content creating terminal #102 will be described next.

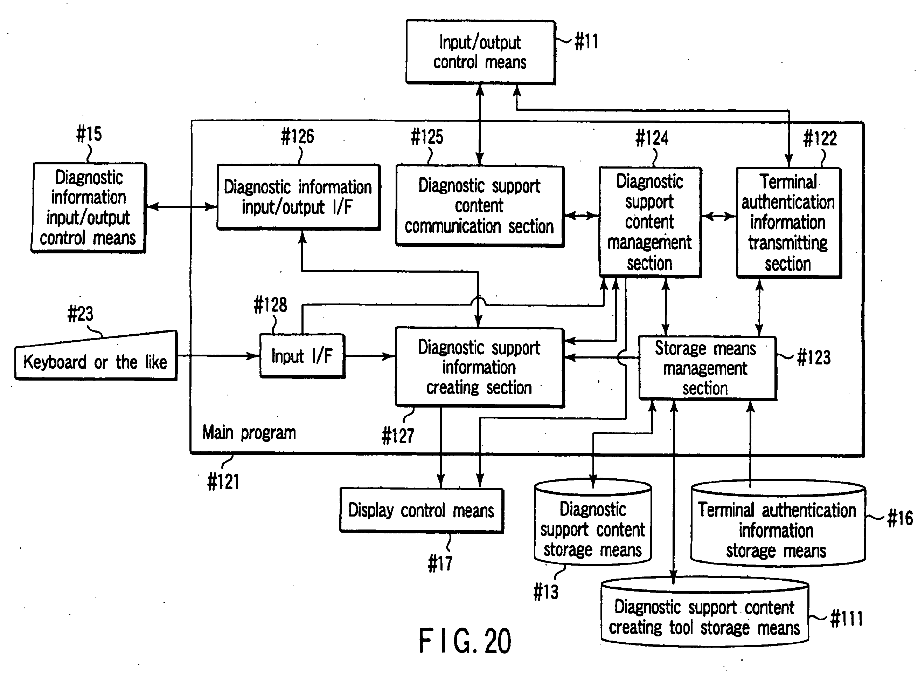

FIG. 20 is a block diagram of a main program #121 executed by a control means #12 of the diagnostic support content creating terminal #102 in this embodiment. Referring to FIG. 20, the main program #121 includes a storage means management section #123 which controls a series of access operations accompanying storage, retrievals, reads, and the like with respect to pieces of information stored in the diagnostic support content storage means #13, terminal authentication information storage means #16, and diagnostic support content creating tool storage means #111, a diagnostic support information input/output I/F #126 which inputs/outputs diagnostic information constituted by patient information, examination information, and image information input through a diagnostic information input/output control means #15, an input I/F #128 serving as an interface for inputting information from an external input means #23 such as a keyboard or mouse, a diagnostic support content creating section #127 for creating a diagnostic support content using the input diagnostic information and diagnostic support content creating tool, a terminal authentication information transmitting section #122 which transmits terminal authentication information to the diagnostic support content server #2 and diagnostic support content creating tool server #103, a diagnostic support content communication section #125 for performing a series of communication operations with the diagnostic support content server #2, accompanying transmission/reception of a diagnostic support content, and with the diagnostic support content creating tool server #103, accompanying reception of a diagnostic support content creating tool, and a diagnostic support content management section #124 which stores the received diagnostic support content and diagnostic support content creating tool in the diagnostic support content storage means #13 and diagnostic support content creating tool storage means #111, respectively, through the storage means management section #123, and updates the respective pieces of management information.

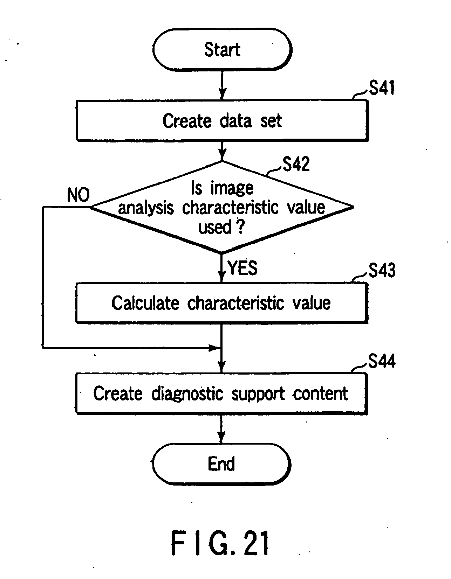

FIG. 21 is a flowchart for explaining the flow of a series of operations in creating a diagnostic support content. In step S41, a data set is created. In this case, the data set is a set of various data (patient information, examination information, image information, diagnosis result information, and the like) and creation conditions (the type of diagnostic support content, diagnostic support content creating tool to be used, and the like) which are required for the creation of a diagnostic support content. Assume that the diagnostic support content described with reference to FIG. 9 is to be created. The data to be used are "endoscopic image data diagnosed as normal and gastritis", which are acquired from, for example, an electronic clinical chart #21 and image file system #22 connected to the medical system #5. The creation conditions to be used are "IHb value calculation, statistical information of normal and gastritis groups, occurrence probability distribution calculation, and graph creation".

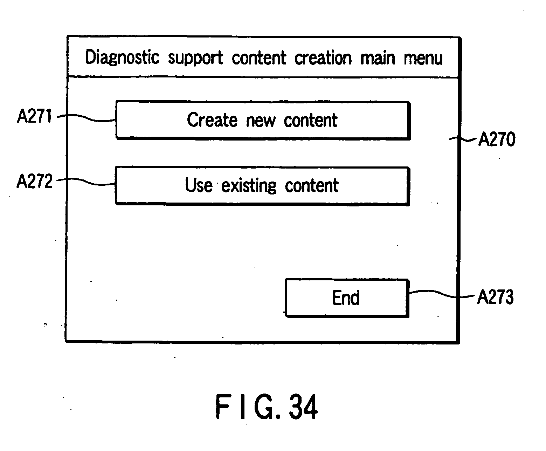

More specifically, first of all, a diagnostic support content creating main window A270 shown in FIG. 34 is displayed on a display means #18. The diagnostic support content creating main window A270 includes a new creation button A271 for creating a new diagnostic support content, an existing content use button A272 for calling out diagnostic support content stored in the diagnostic support content storage means #13 to create a diagnostic support content by reusing the data and conditions, and an end button A273 for ending diagnostic support content creation.

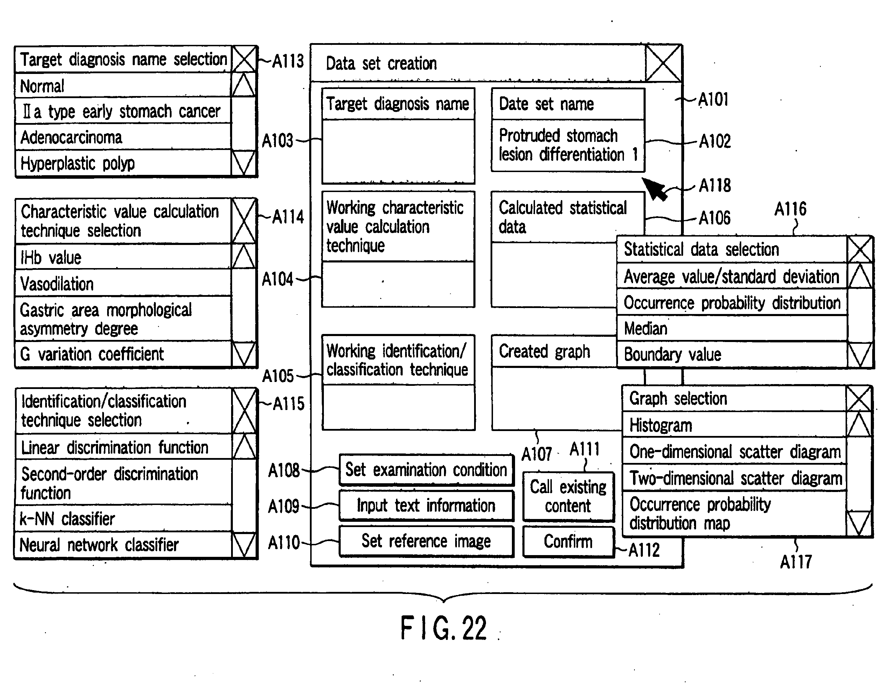

When the new creation button A271 is selected in the diagnostic support content creating main window A270, windows for creating a data set shown in FIG. 22 are displayed on the display means #18, and condition selection, information input operation, and the like are performed by using the external input means #23 such as a keyboard and mouse.

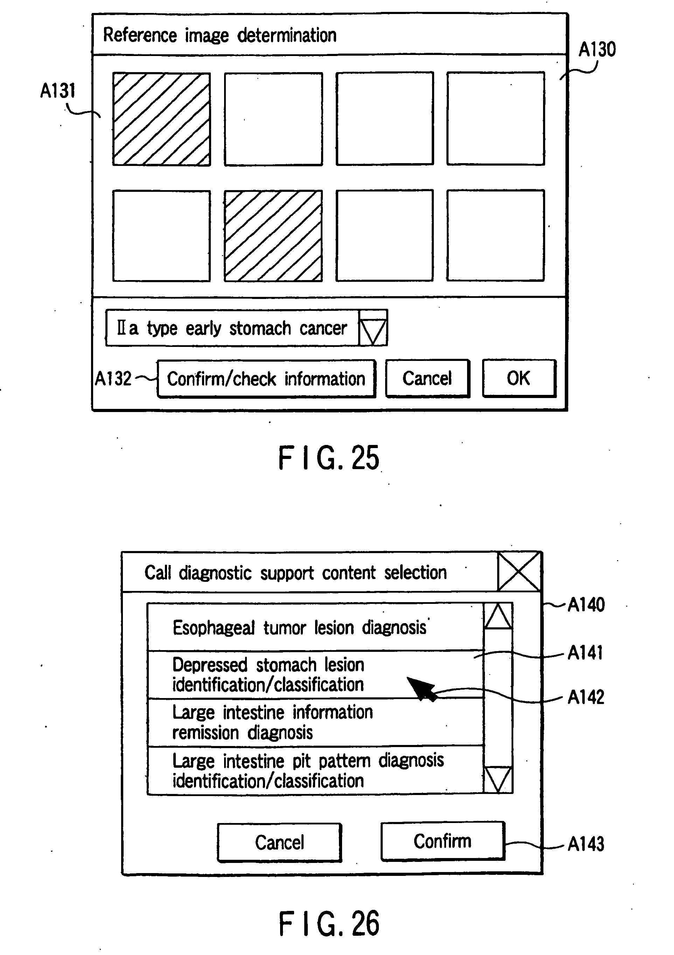

When the existing content use button A272 is selected, a call diagnostic support content selection window A140 shown in FIG. 26 is displayed. The call diagnostic support content selection window A140 includes a diagnostic support content name display area A141 which displays a list of diagnostic support contents that can be called out from the diagnostic support content storage means #13 on the basis of the diagnostic support content management information, and also functions as a menu. A diagnostic support content to be called out is selected by clicking a mouse cursor A142. Subsequently, when a confirm button A143 is clicked, a diagnostic support content creating section #127 reads out the selected diagnostic support content from the diagnostic support content storage means #13, and displays the respective pieces of information used for the creation in the respective setting areas (to be described later) in a data set creating window A101 upon setting them on the basis of the contents of a diagnostic support content object A60 forming the diagnostic support content. The respective set contents can be reused by, for example, changing the target disease and adding case data as needed.

Referring to FIG. 22, the data set creating window A101 is constituted by a data set name input area A102 for inputting a data set name (which coincides with the name of a diagnostic support content in this embodiment), a target diagnosis name setting area A103 for setting the type of disease as a diagnostic support target, a working characteristic value calculation technique setting area A104 for setting a characteristic value calculation technique to be used, a working identification/classification technique setting area A105 for setting an identification/classification technique to be used, a calculation statistical data setting area A106 for setting statistical data to be calculated, a creation graph setting area A107 for setting a graph to be created, an examination condition setting button A108 for setting a modality, examination region, and the like, a text information input button A109 for inputting findings used for diagnostic support and text information of a treatment and the like, a reference image setting button for setting typical case data and similar case data corresponding to each diagnosis, an existing content call button A111 (which facilitates switching to the mode of reusing an existing content even after the new creation mode is selected) for calling out a diagnostic support content when, for example, adding data and changing creation conditions on the basis of an existing diagnostic support content, and a confirm button A112 for confirming data set creation.

Reference numerals A113 to A117 denote a target diagnosis name selection window, characteristic value calculation technique selection window, identification/classification technique selection window, statistical data selection window, and graph selection window, respectively, which are windows for selecting various times with respect to the setting areas A103 to A107. In these selection windows, various types of diagnostic support content creating tools which are stored in the diagnostic support content creating tool storage means #111 and can be used by the diagnostic support content creating section #127 are displayed as menus. These tools can be input to the respective setting areas A103 to A107 by double-clicking with a mouse cursor #A118 or drag-and-drop operation to the corresponding setting areas. In addition, the respective types of diagnostic support content creating tools set in the respective setting areas A103 to A107 can be canceled by double-clicking them.

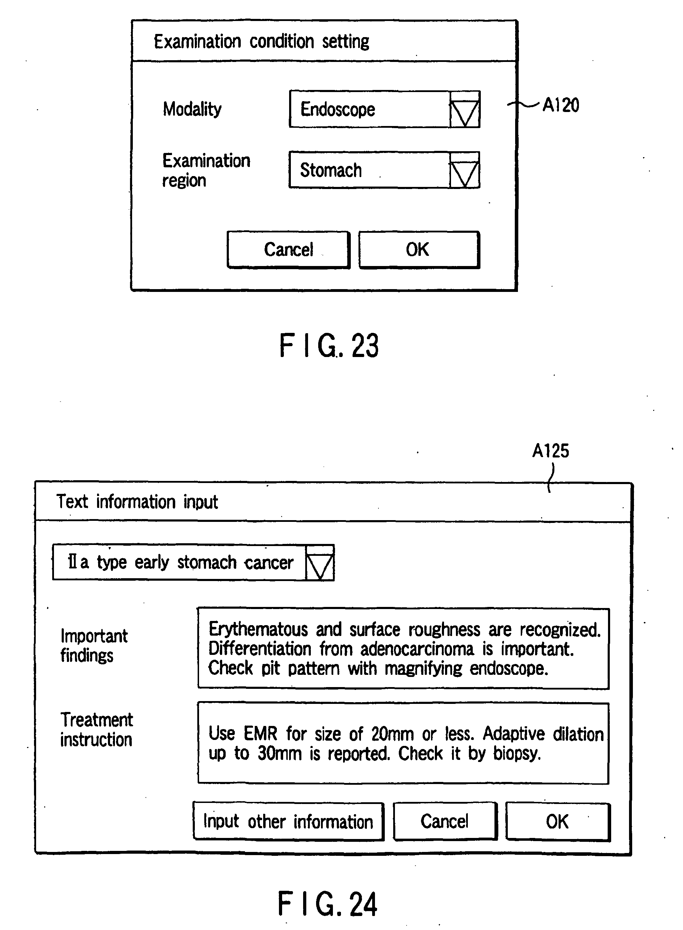

When the examination condition setting button A108 is clicked, an examination condition setting window A120 shown in FIG. 23 is displayed to allow the operator to set a modality and examination region for the diagnostic support content to be created.

When the text information input button A109 is clicked, a text information setting window A125 shown in FIG. 24 is displayed to allow the operator to input important findings and medical knowledge such as a procedure/treatment instruction and the like for each disease type in the form of text information using the external input means #23 such as a keyboard.

When a reference image setting button A110 is clicked, a reference image setting window A130 shown in FIG. 25 is displayed. In the reference image setting window A130, for example, image data retrieved and acquired from the image file system #22 connected to the medical system #5 is displayed in an image list A131. In the image list A131, a desired image is selected as a reference image. When an information confirm/add button A132 is clicked, the patient information, examination information, and image information acquired together with the image from the image file system #22 can be checked, and additional information such as a comment can be added.

When the existing content call button A111 is clicked in creating a data set, the call diagnostic support content selection window A140 shown in FIG. 26 is displayed. The operator then makes a series of settings as in the case wherein the existing content use button A272 is selected.

After a data set is created in step S41, it is checked in step S42 whether to use a characteristic value obtained by using an image analysis technique. If a characteristic value calculation technique is set in the working characteristic value calculation technique setting area A104 in step S41, the flow advances to step S43. If no technique is set in this area, the flow advances to step S44.

In step S43, the characteristic value set in the working characteristic value calculation technique setting area A104 is calculated. An image corresponding to the diagnosis set in the target diagnosis name setting area A103 is retrieved and acquired from the image file system #22, and a characteristic value is calculated by using the characteristic value calculation technique library acquired from the diagnostic support content creating tool storage means #111.

In step S44, a diagnostic support content is created by executing the respective types of libraries acquired from the diagnostic support content creating tool storage means #111 using the respective set items, acquired diagnostic data, and calculated characteristic values. The diagnostic support content is completed as a diagnostic support content object together with a library necessary for execution on the diagnostic support execution terminal #3, and are stored in the diagnostic support content storage means #13 after the diagnostic support information management information is updated.

The created diagnostic support content is transmitted to the diagnostic support content server #2 through the network #4. In transmission and reception between a diagnostic support content creating terminal #202 and the diagnostic support content server #2, terminal specifying information is recognized, a diagnostic support content is transmitted and received, the diagnostic support content management information in the diagnostic support content server is updated, and a diagnostic support content is stored.

A diagnostic support content creating tool used in the diagnostic support content creating terminal #202 can be acquired from the diagnostic support content creating tool server #203 through the network #4. A latest diagnostic support content creating tool can be used in accordance with an improvement, addition, or the like. Note that the operation accompanying the transmission and reception of a diagnostic support content and diagnostic support content creating tool is similar to distribution and reception of a diagnostic support content described in the first embodiment, and hence a detailed description thereof will be omitted.

In this embodiment, the diagnostic support content server #2, diagnostic support execution terminal #3, diagnostic support content creating terminal #102, and diagnostic support content creating tool server #103 have been described as independent computers. However, they can be implemented on one computer by integrating the respective functions.

In addition, diagnostic support content creating tools and a diagnostic support content are software. Obviously, therefore, they can be acquired by using media such as floppy disks as well as being transmitted and received through a network.

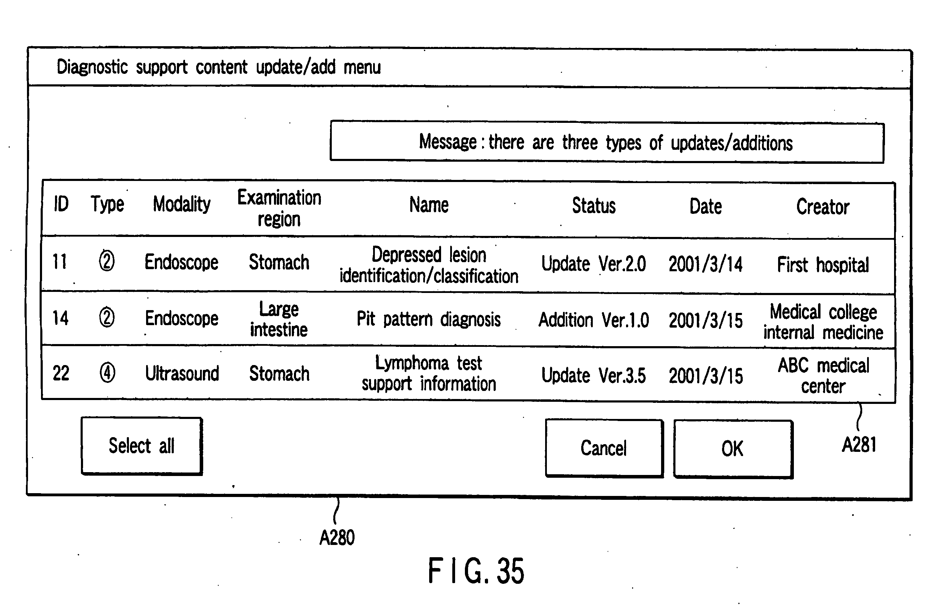

As described above, according to the diagnostic support apparatus of the second embodiment of the , many medical facilities/organizations can freely create a diagnostic support content, and various medical information, image data, and expert medical knowledge accumulated in the respective facilities/organizations can be widely used on diagnostic support apparatuses. In addition, since addition of data and the like can be done with respect to the created diagnostic support content, the performance of the diagnostic support apparatus can be improved by effectively using case data dispersed in many medical facilities/organizations. In the arrangement of the diagnostic support apparatus according to this embodiment, for example, the diagnostic support content list menu shown in FIG. 35 is used in place of the menu shown in FIG. 17 when a diagnostic support content is to be acquired. Referring to FIG. 35, the names of facilities/organizations which have created diagnostic support contents are added to a menu area A281 which is displayed in an update/add menu window A280 and used to display a list of diagnostic support contents and select a content. Therefore, each hospital/clinic which uses these data for an actual clinical examination can know that, for example, a given content is created by "most advanced research facility for cancer of the large intestine", and can use the content with high reliability.

[Third Embodiment]

The third embodiment will be described with reference to the several views of the accompanying rendering. The third embodiment is the same as the second embodiment except that the arrangement of the diagnostic support content creating section #127 in the second embodiment is different from that in the third embodiment, and an image information holding means 151 is added. FIG. 39 is a block diagram of a main program #121 showing the arrangement of a diagnostic support content creating section #127 according to the third embodiment. The difference between the second and third embodiments will be described below.

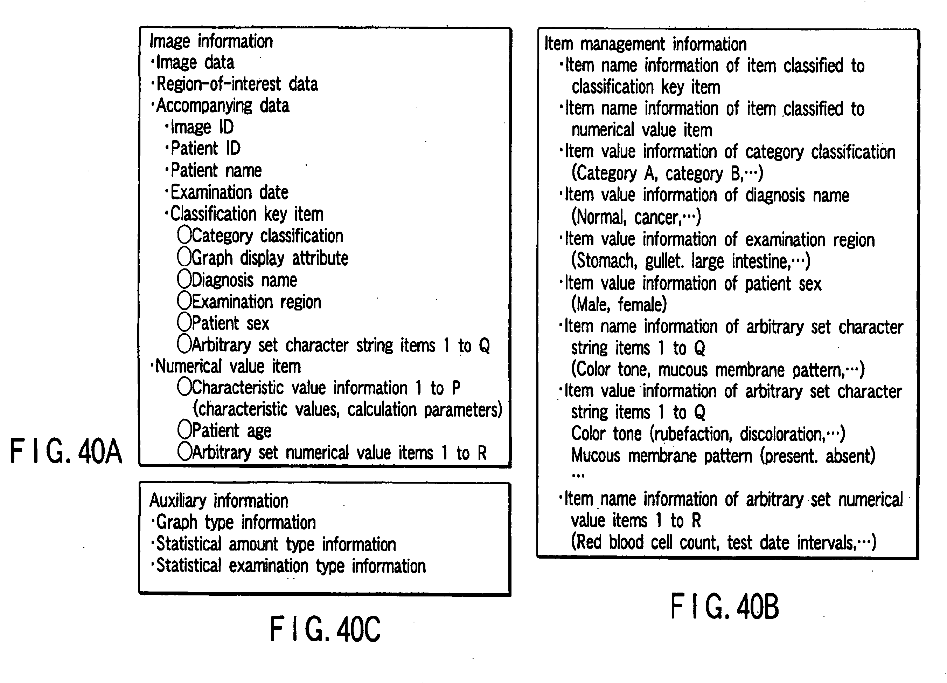

The image information holding means 151 is formed from a hard disk and holds pieces of image information. FIG. 40A shows the format of image information.

Image information is constituted by image data, region-of-interest data, and accompanying data. The image data is digital data of an image signal output from a medical system #5, and is acquired through a diagnostic information input/output control means #15. The region-of-interest data is an area for characteristic value calculation with respect to the image data. The accompanying data is constituted by the patient/examination information acquired through the diagnostic information input/output control means #15 and the information set by a diagnostic support execution terminal #3. In this embodiment, the patient/examination information of the accompanying data includes an image ID, patient ID, patient name, examination name, examination date, patient sex, and patient age. The information set by the diagnostic support execution terminal #3 includes a category classification, graph display attribute, diagnosis name, examination region, characteristic value information constituted by a characteristic value and characteristic value calculation parameter, arbitrary set character string items 1 to Q (Q $\geqq$ 1), and arbitrary set numerical value items 1 to R (R $\geqq$ 1).

Of the respective items included in the accompanying data, the items other than the image ID, patient ID, patient name, and examination date items are held after being classified to a classification key item and numerical value item.

The items classified to the classification key item are the category classification, graph display attribute, diagnosis name, examination region, patient sex, and arbitrary set character string items 1 to Q.

The items classified to the numerical value item are the characteristic value information, patient age, and arbitrary set numerical value items 1 to R.

An image information holding means T1 holds management information of each item contained in image information and auxiliary information to be used for processing in the diagnostic support content creating section #127.

FIG. 40B shows the contents of item management information. The information stored as item management information includes item name information of an item classified to the classification key item, item name information of an item classified to the numerical value key item, and information associated with the item value stored in each of the classification key items of the accompanying data. For example, as the item value information of a diagnosis name, information such as "normal, cancer, polyp, ..." is stored. As item management information, pieces of item name information corresponding to the arbitrary set character string items 1 to Q and arbitrary set character string items 1 to R are stored.

FIG. 40C shows the contents of the auxiliary information. As graph type information, the name information of the graph type created by the diagnostic support content creating section #127 is stored. As statistic type information, the type name information of a statistic computed by the diagnostic support content creating section #127 is stored. As statistical test type information, the type name information of the statistical test computed by the diagnostic support content creating section #127 is stored.

FIG. 39 is a block diagram of a main program #51 executed by a control means #12. An illustration of an arrangement that is not used for the following description is omitted.

A storage means management section #123 controls a series of access operations accompanying storage, retrievals, reads, and the like of image information with respect to the image information holding means 151.

A graph information creating section 152 creates graph information from the image information held in the image information holding means 151. The graph information creating section 152 includes an item selecting section 153, classified data set creating section 156, statistical processing section 155, and graph processing section 154.

The classified data set creating section 156 classifies image information into a plurality of classified data sets on the basis of the classification information set by the item selecting section 153.

A classified data set is a data set of image information classified according to the values of the classification key items of the accompanying data contained in the image information.

The statistical processing section 155 statistically processes the numerical value items contained in a classified data set, and outputs the processing result to the graph processing section 154. The item selecting section 153 designates a specific numerical value item, in the image information in the classified data set, for which statistical processing is to be performed, and a specific type of statistical processing to be performed.

In this embodiment, the statistical processing section 155 processes at least one of statistics such as an average value, standard deviation, standard error, intermediate value, and mode value, or processes at least one of statistical test such as $\underline{t}$ test and $\chi^2$ test.

The graph processing section 154 creates graph information from a numerical value item contained in the classified data set, superimposes statistical processing results on the graph information, and displays the graph information on a display means #18 through a display control means #17. The item selecting section 153 designates a specific numerical value item, of the image information in the classified data set, from which graph information is to be created, and a specific graph to be created.

In this embodiment, the graph processing section 154 processes one of a histogram, one-dimensional scatter diagram, two-dimensional scatter diagram, case count bar graph, and average value bar graph. The form of each graph display will be described later.

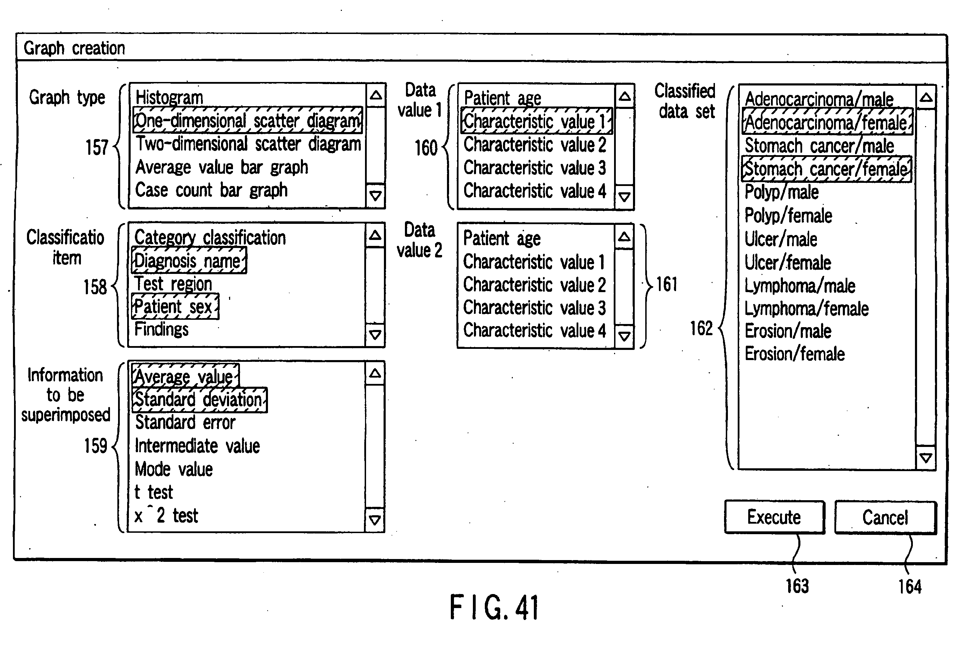

The item selecting section 153 sets classification information to be used by the classified data set creating section 156 by operation with respect to the item selection window shown in FIG. 41, and outputs the information to the classified data set creating section 156. The item selecting section 153 also designates a statistical processing type to be processed with respect to the statistical processing section 155. The item selecting section 153 designates a graph type to be processed with respect to the graph processing section 154. In addition, the item selecting section 153 designates, with respect to the statistical processing section 155 and graph processing section 154, an accompanying data item in image information which is to be processed.

FIG. 41 shows the operation window displayed by the item selecting section 153.

The item selecting section 153 reads out item management information and auxiliary information stored in the image information holding means 151 through the storage means management section #123.

In a graph type selection area 157, the item selecting section 153 displays a list of graph types which can be created by the graph processing section 154, and selects one of the graph types from the contents of the auxiliary information.

In a classification item selection area 158, the item selecting section 153 displays a list of items included in the classification key items of the accompanying data, and selects one or a plurality of types of classification items used for the classification of image information from the contents of the item management information.

In a data value 1 selection area 166, the item selecting section 153 displays a list of item names included in the numerical value items of the accompanying data, and selects one of data types used for graph creation, statistic calculation, or a statistical test from the contents of the item management information.

Likewise, in a data value 2 selection area 161, the item selecting section 153 displays a list of items included in the numerical value items of the accompanying data and selects one of data types to be used for graph creation, statistic calculation, or a statistical test.

The item selecting section 153 validates or invalidates the selection in the data value 1 selection area 166 and data value 2 selection area 161 in accordance with the selection of a graph type in the graph type selection area 157. FIG. 41 shows that the selection in the data value 2 selection area 161 is invalid. Data value 2 is not required for a histogram, one-dimensional scatter diagram, and average value bar graph. If, therefore, a histogram and one-dimensional scatter diagram are selected in the graph type selection area 157, the item selecting section 153 invalidates the selection in the data value 2 selection area 161. In addition, data value 1 and data value 2 are not required for a case count bar graph. If, therefore, a case count bar graph is selected in the graph type selection area 157, the item selecting section 153 invalidates the selection in the data value 1 selection area 166 and data value 2 selection area 161.

In a classified data set selection area 162, the item selecting section 153 displays a list of combinations of item names in accordance with the selection items in the classification item selection area 158 from the contents of the item management information, and selects one or a plurality of combinations. FIG. 41 shows an example in which a list of combinations of diagnosis names and patient sexes is created from diagnosis names and patient sexes as items selected in the classification item selection area 158.

A superimposed information selection area 159 is used to select, from the contents of the accompanying information, one or a plurality of a statistic and statistical test result in the statistical processing section 155 which are to be superimposed on a graph.

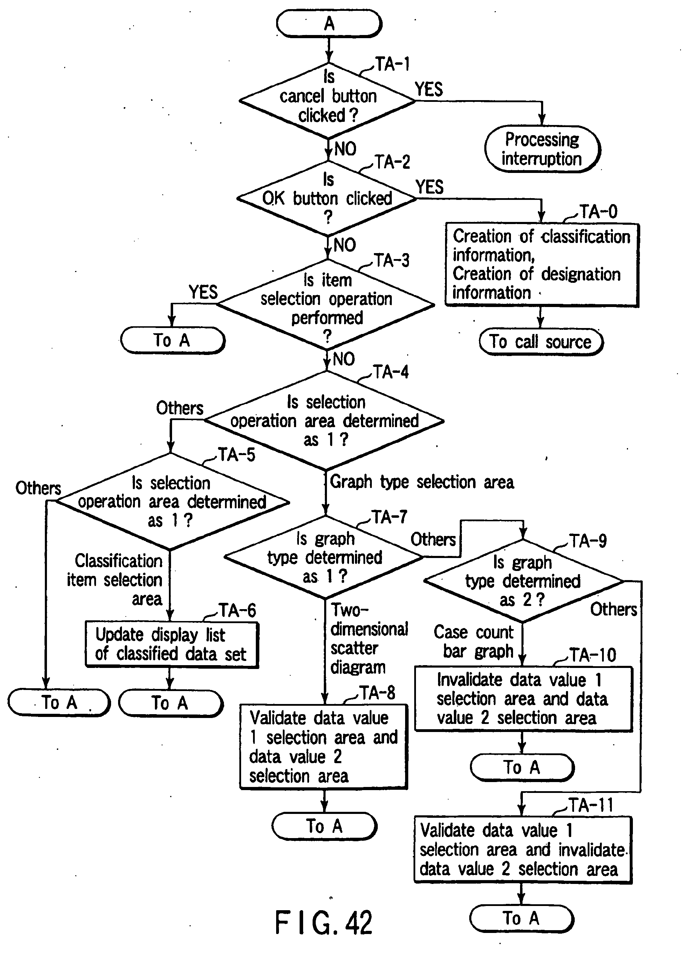

FIGS. 42, 43, 44, 45, and 46 are flowcharts for explaining a series of operations accompanying creation of graph information in this embodiment. Assume that graph information is created and displayed on the display means #18 in response to the operation of a mouse #23 as a trigger.

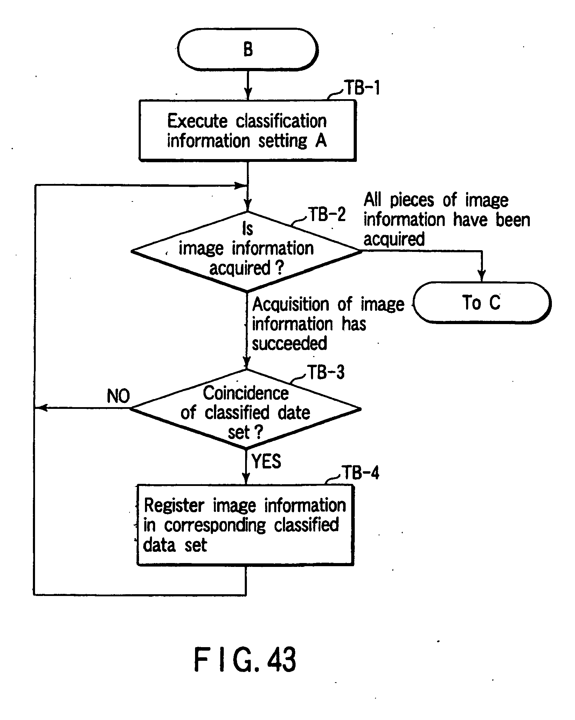

This operation will be described first with reference to FIG. 43. FIG. 43 shows a flowchart in which a classified data set is created from the image information held in the image information holding means 151 in accordance with the classification information created by the item selecting section 153, and the data set is held.

In step TB-1, the item selecting section 153 sets classification information to be used in the classified data set creating section 156 and instruction information for a graph processing section T154 and the statistical processing section 155.

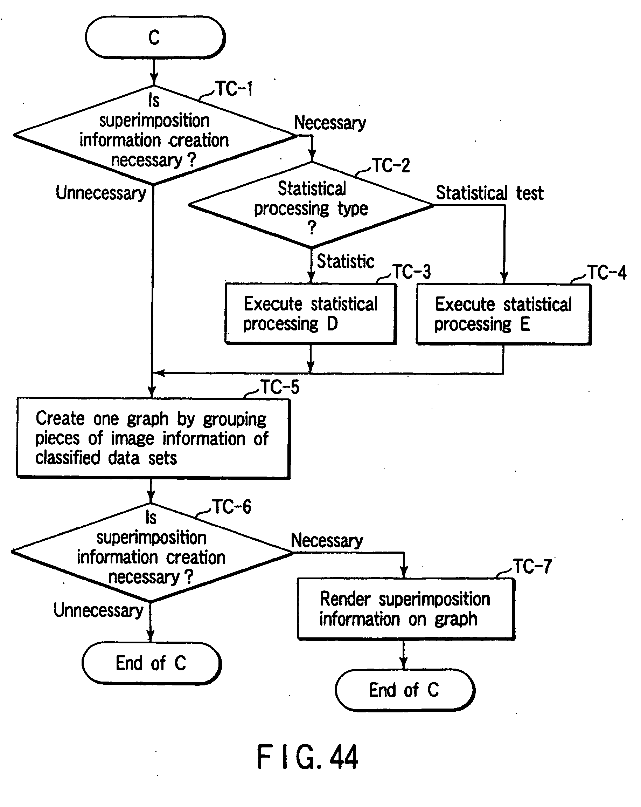

The flow of operation in the item selecting section 153 will be described with reference to FIGS. 42 and 44.

In step TA-1, it is checked whether a cancel button 164 is pressed. If the cancel button 164 is pressed, the subsequent processing is interrupted.

It is checked in step TA-2 whether an OK button 163 is pressed.

If the OK button 163 is pressed, the item selecting section 153 designates a selected graph type in the graph type selection area 157 with respect to the graph processing section 154. The item selecting section 153 also designates selected statistical processing in the superimposed information selection are 159 with respect to the statistical processing section 155. In addition, the item selecting section 153 designates, with respect to the graph processing section 154 and statistical processing section 155, the selection in the data value 1 selection area 166 and the selection in the data value 2 selection area 161. Note that this designation is done only when the data value 1 selection area 166 or data value 2 selection area 161 is valid. The item selecting section 153 outputs classification information constituted by a selected combination in the classified data set selection area 162 to the classified data set creating section 156.

After the above processing, the flow returns to step TB-1 which is the call source.

In step TA-3, it is checked whether item selection operation is performed. If YES in step TA-3, it is checked in steps TA-4 and TA-5 for which selection area the operation has been done.

If the item selection operation has been done in the graph type selection area 157, the selected graph type is determined in steps TA-7 and TA-9. If the selected graph type is a case count bar graph, the data value 1 selection area 166 and data value 2 selection area 161 are invalidated in step TA-10. If the selected graph type is a two-dimensional scatter diagram, the data value 1 selection area 166 and data value 2 selection area 161 are validated in step TA-8. If the selected graph type is other than a case count bar graph and two-dimensional scatter diagram, the data value 1 selection area 166 is validated and the data value 2 selection area 161 is invalidated in step TA-11.

If item selection operation is done in the classification item selection area 158, a combination of item values that selected one or a plurality of classification items can take is created, and the display on the classified data set selection area 162 is updated in step TA-6.

In step TB-2, the classified data set creating section 156 acquires image information held in the image information holding means 151 one by one through the storage means management section #123. In step TB-3, the accompanying data of the acquired image information is compared with the classification information created by the item selecting section 153 to check whether the contents of the accompanying data coincide with the item value combination selected by the item selecting section 153. If they coincide with each other, the image information is registered and held as a data set corresponding to the item value combination in step TB-4. The flow then returns to step TB-2. If they do not coincide with each other, the flow returns to step TB-2.

If all the image information held in the image information holding means 151 is completely acquired in step TB-2, the flow advances to "C" in FIG. 44.

FIG. 44 shows the flow in which the graph processing section 154 creates graph information in accordance with a classified data set.

In step TC-1, the necessity to create superimposition information is determined on the basis of information indicating execution/non-execution of selection statistical processing by the superimposed information selection window 159 which is contained in the classification information created by the item selecting section 153. If the superimposed information selection window 159 is to execute selection statistical processing, the flow advances to step TC-2. Otherwise, the flow advances to step TC-5.

In step TC-2, the statistical processing section 155 determines the type of statistical processing to be executed. If the type is a statistic, the flow advances to step TC-3. If the type is a statistical test, the flow advances to step TC-4.

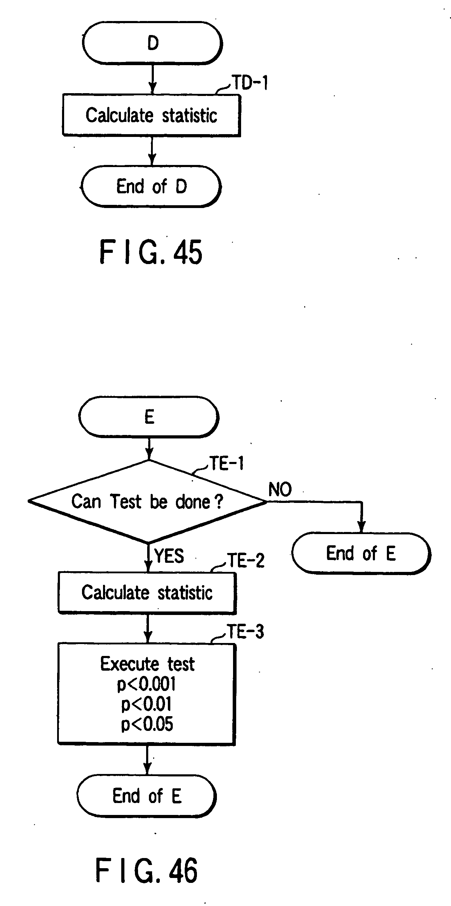

Step TC-3 is an execution step for statistic calculation processing, in which operation is performed in accordance with the processing flow shown in FIG. 45.

In step TD-1 in FIG. 45, statistics associated with the numerical value items designated by the item selecting section 153 are calculated and held for the respective classified data sets held by the classified data set creating section 156.

Step TC-4 is an execution step for statistical test processing, in which operation is performed in accordance with the processing flow in FIG. 46.

In step TE-1 in FIG. 46, it is checked whether there are two or more classified data sets. If YES in step TE-1, it indicates that a test can be performed, and the flow advances to step TE-2. If NO in step TE-1, the flow returns to step TC-4 in FIG. 44.

In step TE-2, a statistic corresponding to the type of statistical test is calculated. In this embodiment, a $\underline{t}$ statistic for the execution of a $\underline{t}$ test or a $\chi^2$ statistic for the execution of a $\chi^2$ test is calculated. Each statistic is calculated with respect to a combination of two classified data sets selected from the classified data sets without redundancy.

The $\underline{t}$ test is used to test the presence/absence of the difference in the average value of numerical value items between two classified data sets. The $\chi^2$ test is used to test the independency of classification key items between two classified data sets.

For example, in the $\underline{t}$ test, a Student's $\underline{t}$ statistic

$$t = \frac{\overline{X} - \overline{Y}}{\sqrt{\left(\frac{1}{m} + \frac{1}{n}\right)s^2}}$$

where $\overline{X}$ and $\overline{Y}$ are sample means, and $\underline{m}$ and $\underline{n}$ are sample counts is calculated.

In addition, $\displaystyle s = \frac{\sum_{i=1}^{m}(X_i - \overline{X})^2 + \sum_{j=1}^{n}(Y_j - \overline{Y})^2}{m + n - 2}$ is calculated from the classified data sets held in the classified data set creating section 156.

In calculating a $\chi^2$ statistic in this embodiment, with respect to the combination of classification key item values which is selected by the item selecting section 153, a contingency table is created by using the classification key located at the head as an attribute for contingency table creation (in the case shown in FIG. 41, a diagnosis of adenocarcinoma/ stomach cancer is used as an attribute for contingency table creation), and

$$\chi^2 = \sum_i \sum_j \frac{\left(nf_{ji} - f_i f_j\right)^2}{nf_i f_j}$$

is calculated by using the accumulated value of the contingency table.

A detailed description of each statistic will be omitted.

In step TE-3, by using the statistics calculated in step TE-2, hypothesis test concerning $p < 0.05$, $p < 0.01$, and $p < 0.001$ is executed with respect to each combination of two classified data sets selected from the classified data sets without redundancy.

If the test result on $p < 0.05$ is rejected, NS is held as a test result.

If the test result on $p < 0.05$ is accepted, and the test result on $p < 0.01$ is rejected, $p < 0.05$ is held as a test result.

Subsequently, in the same manner, in accordance with the test results, $p < 0.01$ or $p < 0.001$ is held as a test result.

In step TC-5, a graph of the graph type designated by the item selecting section 153 is increased, in which data are grouped for each classified data set.

In step TC-6, the necessity for superimposition information creation is determined. If designation information from the item selecting section 153 includes selection of statistical processing in the superimposed information selection window 159, and the flow advances to step TE-2 upon determination in step TE-1 in FIG. 46, the flow advances to step TC-7. Otherwise, the graph information created in step TC-5 is displayed on the display means #18, and the processing is terminated. The graph information displayed in step TC-5 includes no information to be superimposed.

In step TC-7, statistically processed information is superimposed on the graph information created in step TC-5 on the basis of the information created in step TD-1 in FIG. 45 or step TE-3 in FIG. 46, and the resultant information is displayed on the display means #18. The processing is then terminated.

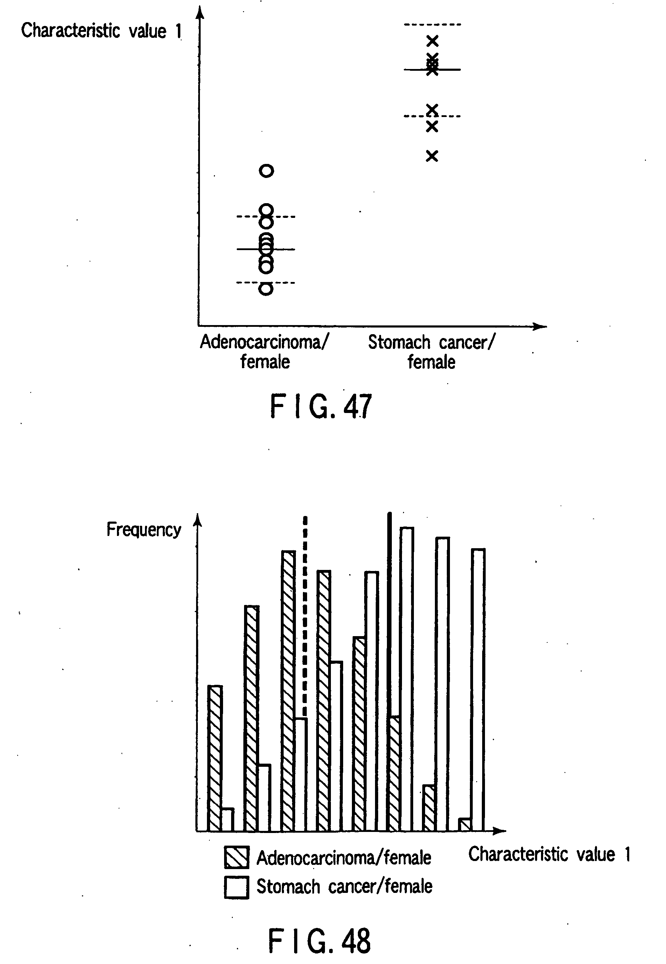

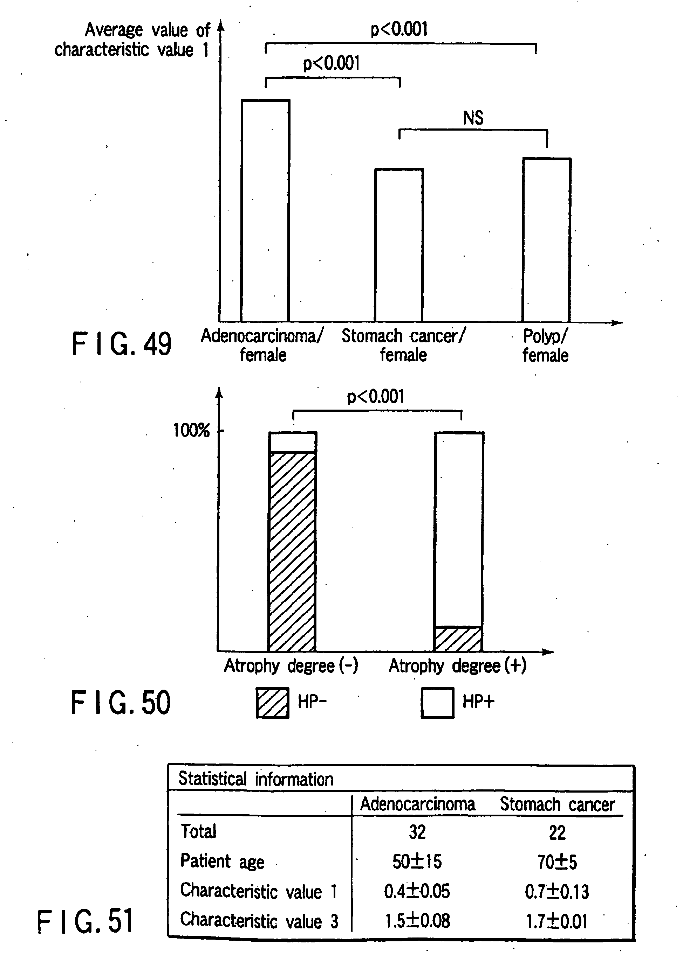

FIGS. 47, 48, 49, and 50 respectively show display examples of graphs according to this embodiment. FIG. 47 shows an example of a one-dimensional scatter diagram, in which the position of average value of characteristic value 1 $\pm$ standard deviation is indicated by line rendering. FIG. 48 shows an example of a histogram, in which the position of the average value of characteristic value 1 is indicated by line rendering. FIG. 49 shows an example of superimposing t test results on an average value bar graph, in which the results obtained by executing a t test of characteristic value 1 with respect to each of three items are plotted. FIG. 50 shows an example of superimposing $\chi^2$ test results on a case count bar graph, in which the case count bar graph and the $\chi^2$ test results obtained when HP+ and HP- are set as attributes of a contingency table are plotted on the basis of four combinations of HP+/atrophy degree (+), HP+/atrophy degree (-), HP-/atrophy degree (+), and HP-/atrophy degree (-).

In this embodiment, graph information grouped for each classified data set is created, and statistical information for each classified data set is superimposed/displayed on the graph information. However, the processing result obtained by the statistical processing section 155 may be displayed on the display means #18 to display, for each classified data set, a statistic or statistical test result concerning the accompanying data item selected by the item selecting section 153, as shown in FIG. 51.

(Effects)

Displaying statistically processed information on a graph allows easy comparison with the statistically processed information and provides objective understanding of the graph display owing to the statistically processed information.

In creating a graph, classification items and data values are separately displayed and selected. This prevents the operator from mistakenly selecting a classification item as a data value or mistakenly selecting a data value as a classification item, and improves operability.

Since a combination of a plurality of selected classification items is used as a classification item, the labor spent to create a graph is reduced.

Since only items selected from a combination of a plurality of selected classification items are used as classification items, the labor spent to create a graph is reduced.

A modification of the third embodiment will be described next.

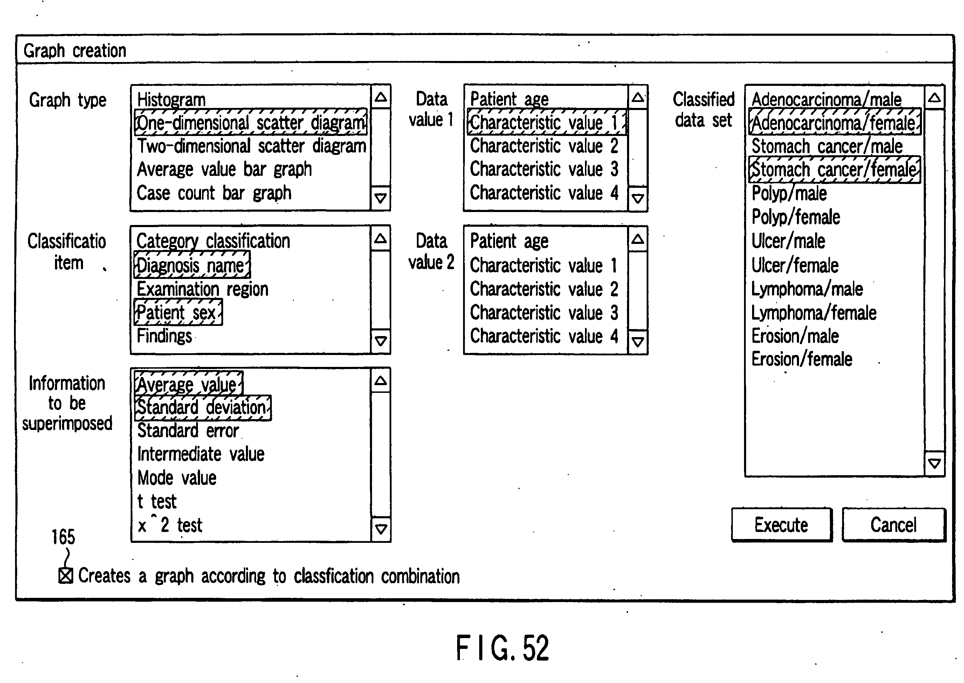

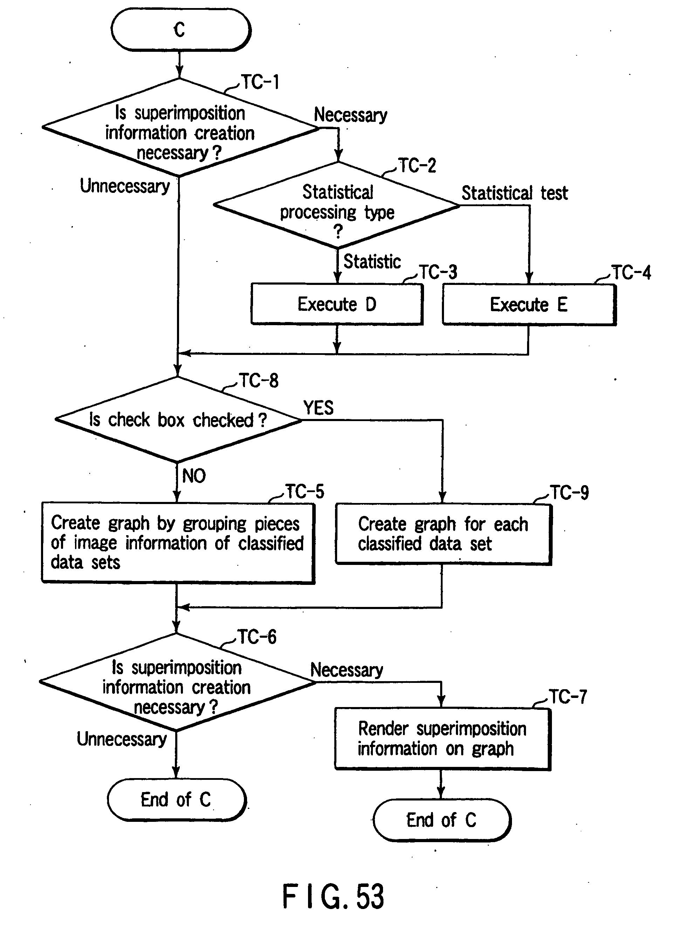

This modification differs from the third embodiment in that the item selecting section 153 displays an operation window having a check box 165, as shown in FIG. 52. FIG. 44 showing the processing flow in the third embodiment is revised into FIG. 53. The difference between FIGS. 53 and 44 is that steps TC-8 and TC-9 are inserted in FIG. 53.

The item selecting section 153 transfers the checked state of the check box 165 in FIG. 52 as designation information to the graph processing section 154.

In step TC-8 in the processing flow shown in FIG. 53, the graph processing section 154 checks from the designation information from the item selecting section 153 whether the check box 165 is checked. If the check box is not checked, one piece of graph information is created by grouping image information of each classified date set in step TC-5 in the same manner as in the third embodiment.

If the check box 165 is checked, pieces of graph information equal in number to classified data sets are created in step TC-9. In addition, if the check box 165 is checked, statistic information for each classified date set is superimposed on corresponding graph information in step C-7.

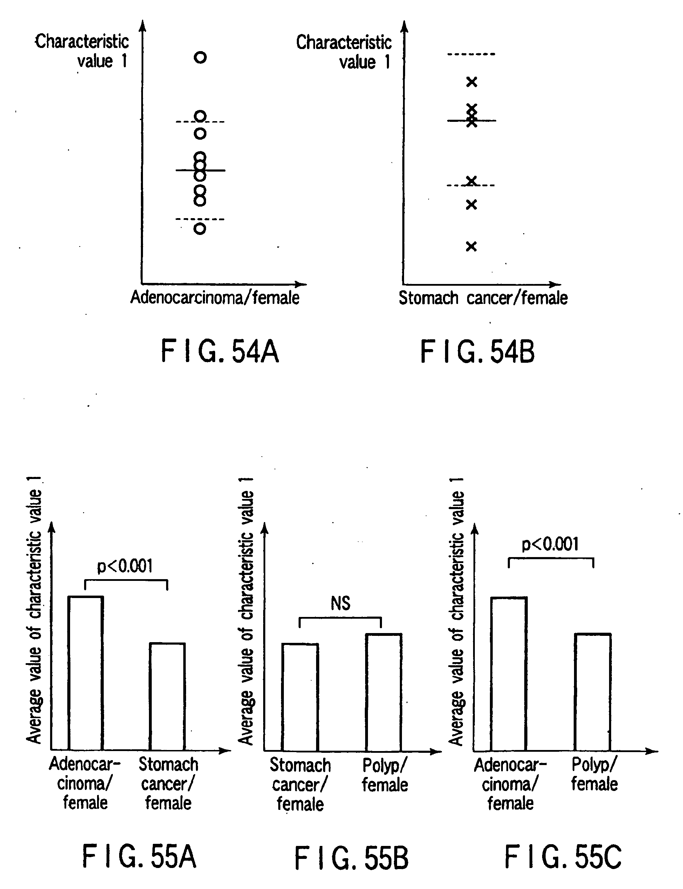

FIGS. 54A and 54B show the content to be displayed when the check box 165 is checked in operation for the graph information display shown in FIG. 47. As shown in FIGS. 54A and 54B, a graph is displayed for each classified data set. This reduces the labor spent to repeatedly create graphs for the respective classified data sets. In addition, the axial scale of each classified date set is increased to make it easier to read the value of each item from the graph. Furthermore, this reduces the overlap between the respective graph elements to prevent misidentification of the frequency distributions of graph elements.

Assume that in step TC-9, when the check box 165 is checked, graph information is created for each of a combination of two classified data sets selected from the classified data sets without redundancy. In this case, as shown in FIGS. 55A to 55C, graphs equal in number to the number of times a statistical test is executed are displayed, and hence the resultant display is easier to read than the display in FIG. 49.

[Fourth Embodiment]

The fourth embodiment will be described with reference to the several views of the accompanying rendering. The fourth embodiment is the same as the third embodiment except that the arrangement of a diagnostic support content creating section #127 is different from that in third embodiment.

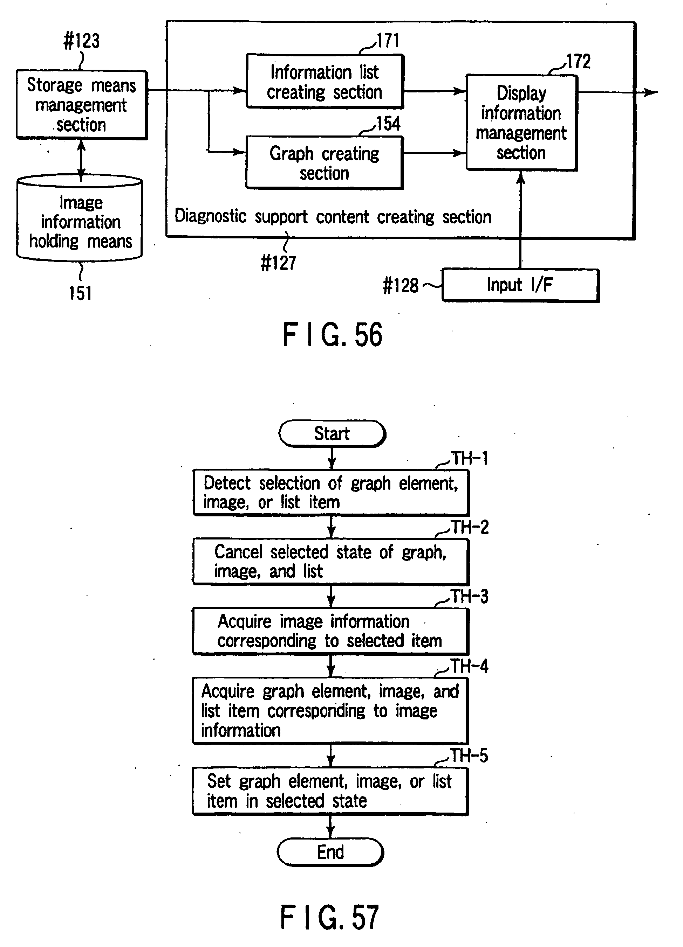

FIG. 56 is a block diagram of a main program #121, which shows the arrangement of the diagnostic support content creating section #127 according to the fourth embodiment. The difference between this embodiment and the third embodiment will be described below.

The diagnostic support content creating section #127 includes an information list creating section 171, graph creating section 154, and display information management section 172.

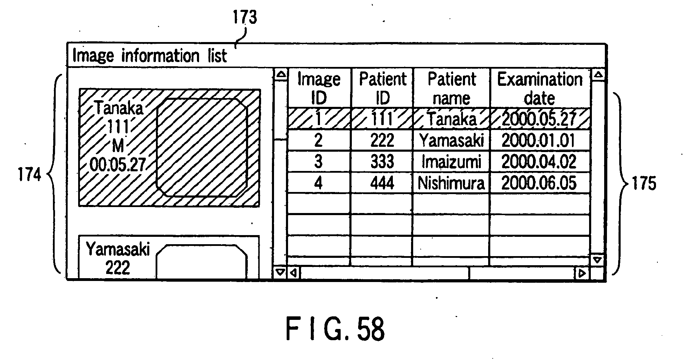

The information list creating section 171 displays a list of image information acquired from a storage means management section #123 as an image information list 173 shown in FIG. 58 on a display means #18.

The image information list 173 includes an image data display area 174 and accompanying data display area 175.

In the image data display area 174, the image data of the image information acquired from the storage means management section #123 is displayed as an image list.

In the accompanying data display area 175, the accompanying data of the image information acquired from the storage means management section #123 is displayed as a list.

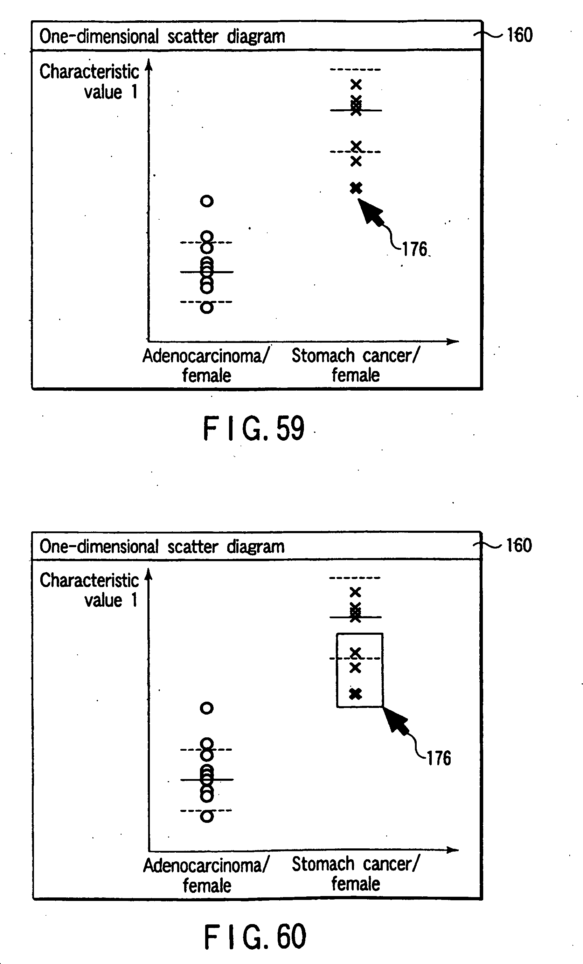

The graph creating section 154 is similar to the graph creating section in the third embodiment, and displays, for example, graph information 160 shown in FIG. 59 on the display means #18.

The display information management section 172 holds the correspondence between image information and each image displayed in the image data display area 174, the correspondence between image information and each line of a list displayed in the accompanying data display area 175, and the correspondence between image information and each graph element displayed on the graph information 160.

The display information management section 172 detects the operation of a mouse cursor 176 by a mouse #23, and acquires the operation information of the mouse cursor 176 through the input I/F #58.

When an image in the image data display area 174 is selected by the operation of the mouse cursor 176, the display information management section 172 acquires image information corresponding to the selected image from the correspondence between the image imaging and each image display in the image data display area 174.

When a line in the image data display area 174 is selected by the operation of the mouse cursor 176, the display information management section 172 acquires image information corresponding to the selected line from the correspondence between the image information and each line displayed in the image data display area 174.

When a graph element on the graph information 160 is selected by the operation of the mouse cursor 176, the display information management section 172 acquires image information corresponding to the selected graph element from the correspondence between the image information and each graph element displayed on the graph information 160.

The display information management section 172 selects an image in the image data display area 174 displayed on the display means #18, and inverts the color tone of the display. The display information management section 172 selects a line in the accompanying data display area 175 displayed on the display means #18, and inverts the color tone of the display. The display information management section 172 selects an image on the graph information 160 displayed on the display means #18, and changes the color tone of the display. In this embodiment, an image whose color tone is inverted or not inverted is displayed in the image data display area 174, a line whose color tone is inverted or not inverted is displayed in the accompanying data display area 175, and a black or red graph element is displayed on the graph information 160.

FIG. 57 is a flowchart for the display information management section 172, which explains linking operation between the image information list 173 and the graph information 160 in this embodiment. Assume that the image information list 173 and graph information have already been displayed on the display means #18.

In step TH-1, the display information management section 172 detects that an image in the image data display area 174, a line in the accompanying data display area 175, or a graph element on the graph information 160 is selected with the mouse cursor 176, and acquires the operation information of the mouse cursor 176.

In step TH-2, all images to be displayed in the image data display area 174 are displayed as images whose color tones are not inverted.

Alternatively, in step TH-2, all lines to be displayed in the accompanying data display area 175 are displayed as lines whose color tones are not inverted.

Alternatively, all graph elements to be displayed on the graph information 160 are displayed as black graph elements.

In step TH-3, the display information management section 172 acquires image information from the image, line, or graph element, selected by the selecting operation in step TH-1, using the correspondence between image information and each image displayed in the image data display area 174, the correspondence between image information and each line displayed in the accompanying data display area 174, and the correspondence between image information and each graph element displayed on the graph information 160.

In step TH-4, the display information management section 172 acquires an image in the image data display area 174, a line in the accompanying data display area 175, and a graph element in the graph information 160, which correspond to the image information acquired in step TH-3, by using the correspondence between the image information and each image displayed in the image data display area 174, the correspondence between the image information and each line displayed in the accompanying data display area 175, and the correspondence between the image information and each graph element displayed on the graph information 160.

In step TH-5, the image in the image data display area 174, the line in the accompanying data display area 175, and the graph element on the graph information 160, which are acquired in step TH-4, are displayed on the display means #18 as a color-tone-inverted image, color-tone-inverted line, and a red graph element, respectively.

FIGS. 58 and 59 show how a graph element is displayed as a red graph element upon selection of the graph element with the mouse cursor 176, and an image and line corresponding to image information corresponding to the graph element are inverted/displayed.

When graph elements on the graph information 160 are enclosed with a rectangle by the operation of the mouse cursor 176 as shown in FIG. 60, processing from step TH-3 to step TH-5 is executed with respect to all the graph elements enclosed within the rectangle to display all the graph elements included in the rectangle as red elements and invert/display images corresponding to image information corresponding to the graph elements.

Note that as methods of changing the display of graph elements by selection include, for example, methods of changing the shapes and sizes and enclosing the graph elements within circular and rectangular markers as well as the method of changing the color tone as in this embodiment are available. Methods of changing the display of images include methods of changing the contrast and size and adding makers. Methods of changing the display of lines include methods of changing the display character color, display character thickness, and display character font and adding markers.

(Effect)

Images, accompanying data, and graph elements can be referred to in association with each other, resulting in improved operability.

[Fifth Embodiment]

The fifth embodiment will be described with reference to the several views of the accompanying rendering. The fifth embodiment is the same as the fourth embodiment except that the arrangement of a diagnostic support content creating section #127 is different from that in the fourth embodiment.

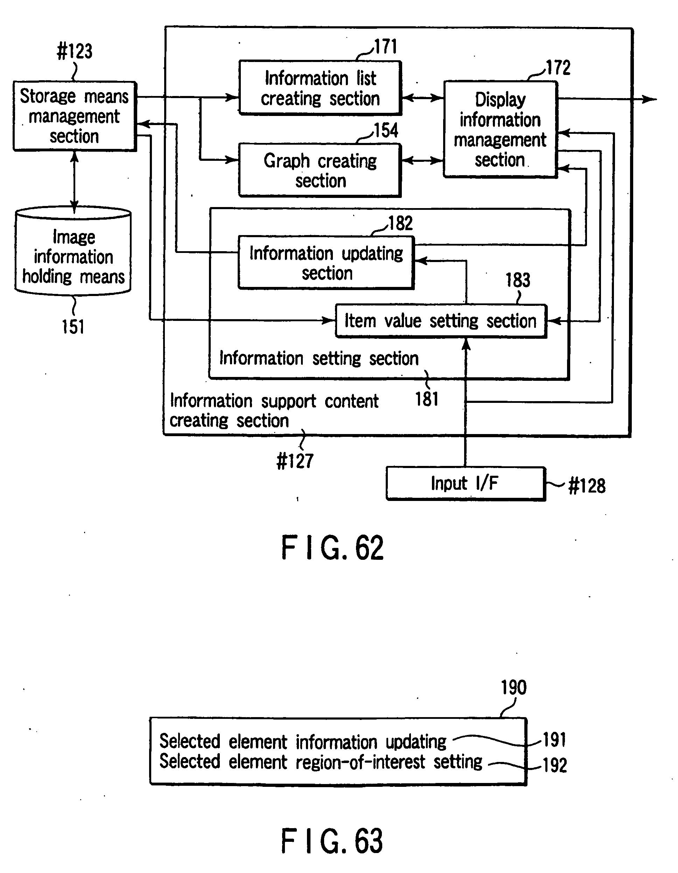

FIG. 62 is a block diagram of a main program #121, which shows the arrangement of the diagnostic support content creating section #127 according to the fourth embodiment. The difference between this embodiment and the fourth embodiment will be described below.

Unlike the diagnostic support content creating section #127 in the fourth embodiment, the diagnostic support content creating section #127 in this embodiment includes an information setting section 181.

A display information management section 172 displays a menu 190 shown in FIG. 63 at the display position of a mouse cursor 176 upon selection of an image in an image data display area 174, a line in accompanying data display area 174, or a graph element on graph information 160 by the operation of the mouse cursor 176. The menu 190 includes a selected element information updating 191 and selected element region-of-interest setting 192. When the selected element information updating 191 is selected, the display information management section 172 acquires image information corresponding to the selection of the image in the image data display area 174, the line in the accompanying data display area 175, or the graph element on the graph information 160 with the mouse cursor 176, and outputs the acquired information to the information setting section 181.

The display information management section 172 causes an information list creating section 171 and graph creating section 154 to re-create an image information list 173 and the graph information 160 in accordance with instructions to re-create the image information list 173 and graph information 160 from the information setting section 181, and updates the display on a display means #18.

Assume that the graph creating section 154 in this embodiment determines whether to use image information for graph creation, in accordance with the contents of graph creation attributes contained in the accompanying data of the image information.

The information setting section 181 sets accompanying data for the image information transferred from the information setting section 181, and updates the image information held in an image information holding means 151. The information setting section 181 also issues, to the display information management section 172, instructions to re-create the image information list 173 and graph information 160.

The information setting section 181 includes an item value setting section 183 and information updating section 182.

The information updating section 182 updates the image information held in the image information holding means 151 by transferring the image information for which the accompanying data is set by the item value setting section 183 to a storage means management section #123, and also updates the display contents of the accompanying data display area 175 on the image information list 173 and the display contents of the graph information 160 by issuing re-creating instructions to the display information management section 172.

The item value setting section 183 sets accompanying data for the image information.

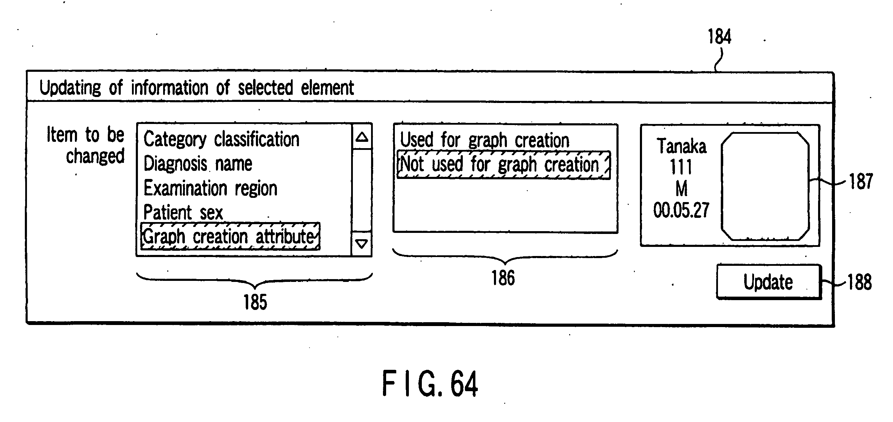

FIG. 64 shows a setting operation window for accompanying data for image information, which is operated by the item value setting section 183. The setting operation window is formed from an information update window 184. The information update window 184 includes a change item selection area 185, change item value selection area 186, image data display area 187, and update button 188.

The items displayed in the change item selection area 185 and change item value selection area 186 are selected one by one.

In the image data display area 187, the image data of set image information is displayed.

The item value setting section 183 acquires item management information held in the image information holding means 151, acquires all pieces of item name information of the items, of the item management information, which are classified to the classified key item, and stores them in the change item selection area 185.

The item value setting section 183 also acquires item value information corresponding to the items selected by the change item selection area 185 from the item management information held in the image information holding means 151 through the storage means management section #123.

When the update button 188 is clicked with a mouse #23, an item to be changed and item value with respect to the accompanying data of the image information are transferred to the information updating section 182.

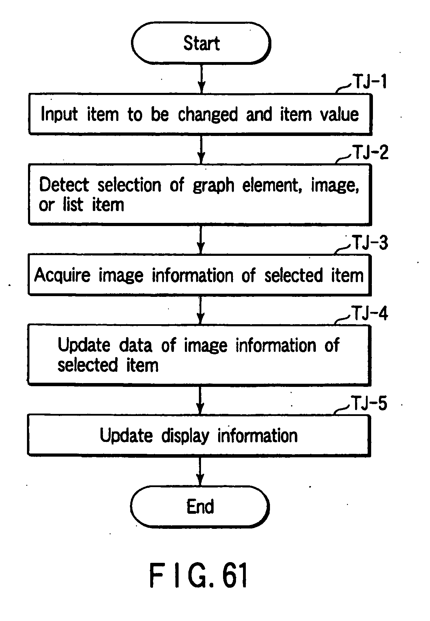

FIG. 61 is a flowchart for the display information management section 172 and information setting section 181, which explains operation of changing the display of the image information list 173 and graph information 160 accompanying a change of the settings of image information. FIG. 61 is a flowchart showing processing after the image information list 173 and graph information are displayed on the display means #18 and the selected element information updating 191 is selected from the menu 190 by the operation of the mouse #23.

Steps TJ-1, TJ-2, and TJ-5 are processing steps in the display information management section 172.

Steps TJ-3 and TJ-4 are processing steps in the information setting section 181.

In step TJ-1, the display information management section 172 detects the selection of an image in the image data display area 174, a line in the image data display area 174, or a graph element on the graph information 160 with the mouse cursor 176, and acquires operation information.

In step TJ-2, the display information management section 172 acquires image information from the image, line, or graph element, selected by the selecting operation in step TH-1, using the correspondence between image information and each image displayed in the image data display area 174, the correspondence between image information and each line displayed in the accompanying data display area 174, and the correspondence between image information and each graph element displayed on the graph information 160.

In step TJ-3, the image information acquired in step TJ-2 is set by the item value setting section 183. The image data of the image information is displayed in the image data display area 187 in the information update window 184 shown in FIG. 64. When an item in the change item selection area is selected with the mouse #23, item contents corresponding to the selected item are displayed in the change item value selection area 186. Of the contents displayed in the change item value selection area 186, the contents set in the accompanying data of the image information are inverted/displayed. When an item in the change item value selection area is selected with the mouse #23, the item inverted before the selection is restored, and the selected item is inverted/displayed.

When the update button 188 is clicked with the mouse #23, the item value setting section 183 acquires the selected item in the change item selection area 185 and the selected item value in the change item value selection area 186.

In step TJ-4, the information updating section 182 transfers the image information set by the item value setting section 183 to the storage means management section #123 to hold, in the image information holding means 151, the image information whose settings have been changed.

In step TJ-5, the item value setting section 183 instructs the display information management section 172 to re-create the image information list 173 and graph information 160. The display information management section 172 instructs the information list creating section 171 and graph creating section 154 to re-create the image information list 173 and graph information 160.

The graph creating section 154 determines, in accordance with the contents of a graph creation attribute as an accompanying data item in image information, whether to use the image information to graph creation. Therefore, the graph creating section 154 makes a setting to display/non-display elements on the graph information 160, in accordance with the graph creation attribute settings in the item value setting section 183.

In this embodiment, the image data of image information is displayed in the image data display area 187 in the information update window 184. In this case, the image data to be displayed may be reduced to reduce the size of the information update window 184 as to allow the operator to see the display of the image data display area 174 and make accompanying data settings while referring to the accompanying data of other image information.

(Effects)

Since a graph element is selected on the graph information 160 and image information is set for the selected graph element, operability is improved.

In addition, since whether or not to display a graph element is selected by operation on the graph information 160, unnecessary graph elements can be easily removed from the graph information 160.

Furthermore, in setting image information, image data is displayed. Therefore, when a plurality of graph elements are selected and pieces of image information are consecutively set, the operator can know the contents of image data. This prevents error settings in image information.

A modification of the fifth embodiment will be described next.

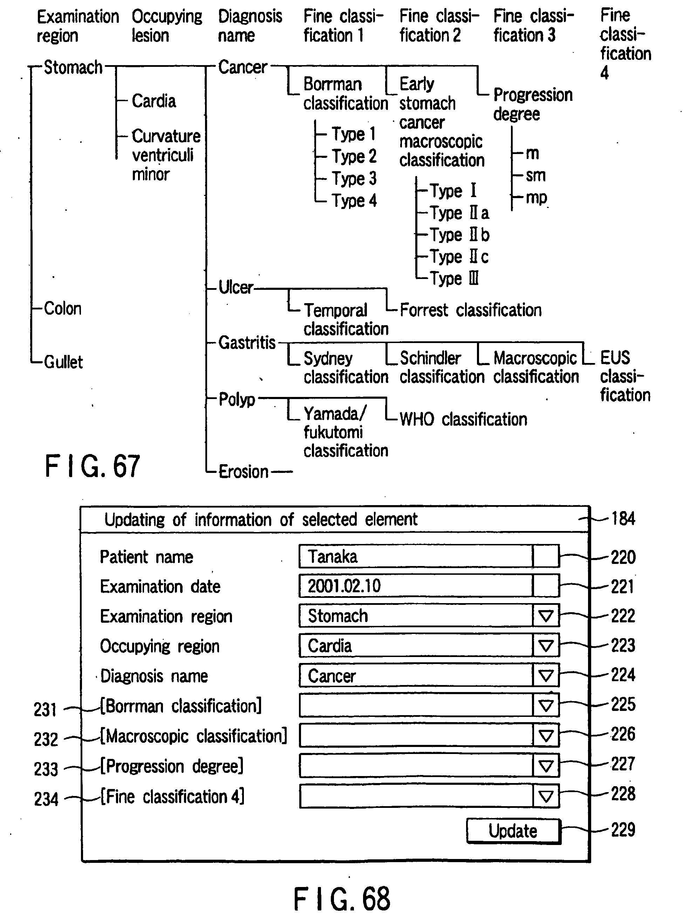

This modification will be described with reference to FIG. 67 and FIGS. 40A to 40C.

In the item management information, contents corresponding to hierarchical information shown in FIG. 67 are stored as the item name information of arbitrary set character string items 1 to Q and the item value information of the arbitrary set character string items 1 to Q. In addition, in the item management information, the item values (not shown) of at higher hierarchical levels of items and pieces of information (not shown) at item hierarchical levels which respectively correspond to the arbitrary set character string items 1 to Q are stored.

For example, information contents an occupying lesion of the stomach is stored such that the item name of arbitrary set character string item 1 is set to occupying lesion of stomach; the item value information of arbitrary set character string item 1, to cardia, curvature ventriculi minor, ...; the item value of the higher hierarchical level of arbitrary set character string item 1, to stomach; and the item hierarchical level, to 1. In another example, likewise, the item name of arbitrary set character string item 2 is set to early cancer macroscopic classification; the item value information of arbitrary set character string item 2, to I type, IIa type,...; the item value of the higher hierarchical level of arbitrary set character string item 2, to cancer; and the item hierarchical level, to 4.

Item hierarchical levels are set such that 1 corresponds to an occupying lesion position; 3, the position of fine classification 1; 4, the position of fine classification 2; 5, the position of fine classification 3; and 6, the position of fine classification 4.

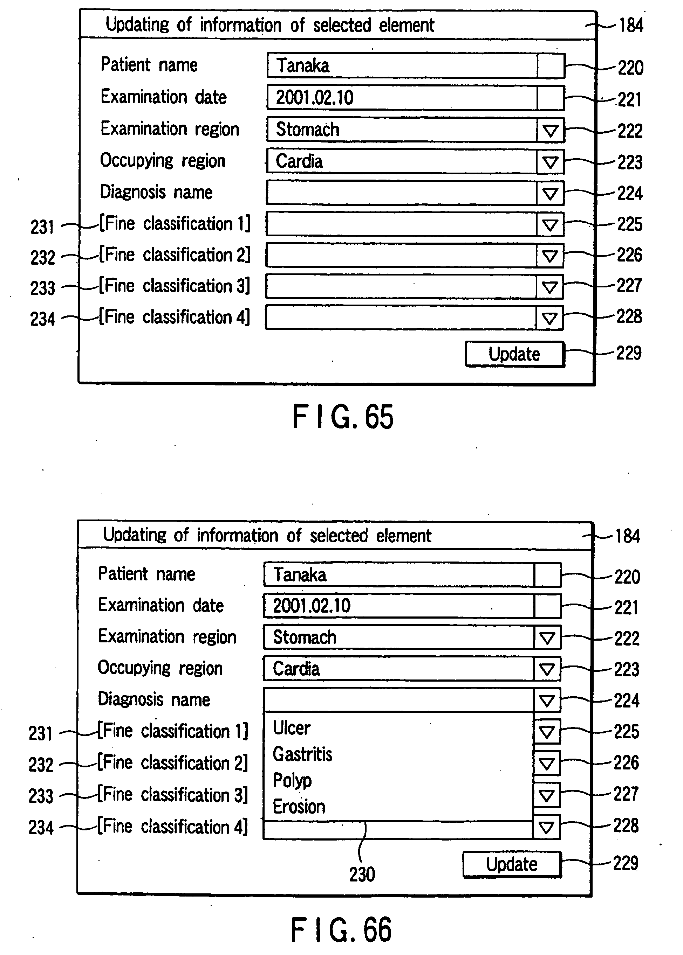

This modification will be described next with reference to FIG. 65.

FIG. 65 shows a setting operation window for the accompanying data of image information, which is operated by the item value setting section 183. The setting operation window is formed from the information update window 184.

The information update window 184 includes a patient name input item 220, an examination date input item 221, an examination region input item 222, an occupying region input item 223, a diagnosis name input item 224, a fine classification 1 input item 225, a fine classification 2 input item 226, a fine classification 3 input item 227, a fine classification 4 input item 228, an update button 229, a fine classification 1 item name field 231, a fine classification 2 item name field 232, a fine classification 3 item name field 233, and a fine classification 4 item name field 234.

When item value setting section 183 starts processing, the item value setting section 183 acquires item management information held in the image information holding means 151 through the storage means management section #123, and creates the hierarchical information shown in FIG. 67 from the item value information of an examination region, the item value information of a diagnosis, the item name information of arbitrary set character string items 1 to Q, and the item value information of arbitrary set character string items 1 to Q.

When the button on the side of the diagnosis name input item 224 is clicked with the mouse #23, the item value setting section 183 uses the hierarchical information to store and display the item values of the corresponding items in a menu 230, as shown in FIG. 66, and selects the contents of the diagnosis name input item 224 from the menu 230.

Referring to FIG. 66, when, for example, the button on the side of the diagnosis name input item 224 is clicked, stomach as the information stored in the examination region input item is acquired, and a diagnosis name whose upper item is stomach is acquired from the hierarchical information and stored in the menu 230.

Similar processing is performed with respect to the examination region input item 222, occupying region input item 223, fine classification 1 input item 225, fine classification 2 input item 226, fine classification 3 input item 224, and fine classification 4 input item 226.

In addition, the item value setting section 183 displays item names in the fine classification 1 item name field 231, fine classification 2 item name field 232, fine classification 3 item name field 233, and fine classification 4 item name field 234 by using the hierarchical information in accordance with the input item name of the diagnosis name.

Referring to FIG. 68, when, for example, cancer is selected as a diagnosis name, Borrman classification, early stomach cancer macroscopic classification, and progression degree, which are item names located at lower hierarchical levels with respect to stomach as an examination lesion and cancer as a diagnosis name, are selected from the hierarchical information and displayed in the fine classification 1 item name field 231, fine classification 2 item name field 232, and fine classification 3 item name field 233, respectively. Assume that the display contents of the fine classification 1 item name field 231, fine classification 2 item name field 232, and fine classification 3 item name field 233 are names contained in the hierarchical information. In this case, when the button on the right side of each input item is clicked, the item value of the corresponding item is stored and displayed in the menu 230, as in the case of the diagnosis name input item 224, and the item content of each input item is selected from the menu 230.

(Effects)

A menu of a list of choices to be input is created and displayed in accordance with hierarchical information and the contents set in higher hierarchical levels. This prevents the operator from erroneously inputting information to a choice that cannot be selected. In addition, since no choice that cannot be selected is displayed, the display becomes readable, and the operability is improved.

Furthermore, in accordance with the contents set at higher hierarchical levels, the names of items to be input are updated/displayed, and items to be input are set. This prevents the operator to erroneously input information to items that cannot be selected. In addition, since unnecessary display in the window is omitted, the operability is improved.

[Sixth Embodiment]

The sixth embodiment will be described with reference to the several views of the accompanying rendering. The sixth embodiment is the same as the fourth embodiment except that the arrangement of a diagnostic support content creating section #127 is different from that in the fourth embodiment.

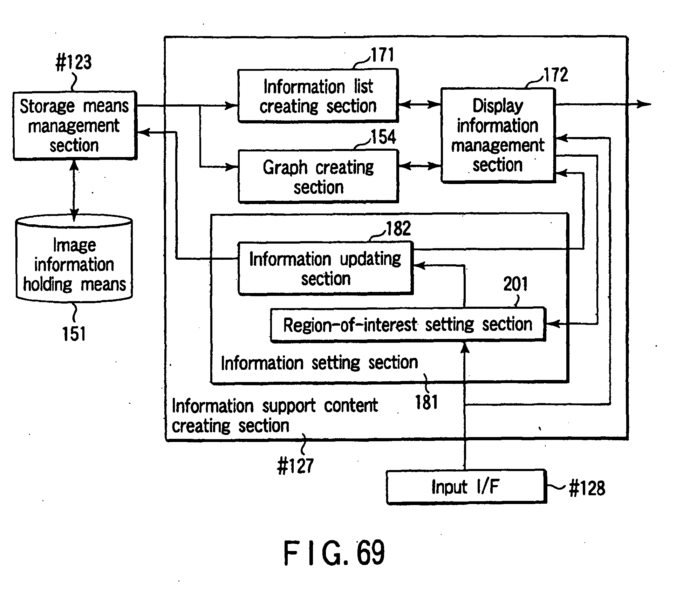

FIG. 69 is a block diagram of a main program #121, which shows the arrangement of the diagnostic support content creating section #127 according to the sixth embodiment. The difference between the sixth and fifth embodiments will be described below.

Unlike the diagnostic support content creating section #127 in the fifth embodiment, the diagnostic support content creating section #127 in the sixth embodiment has a region-of-interest setting section 201 in place of the item value setting section 183.

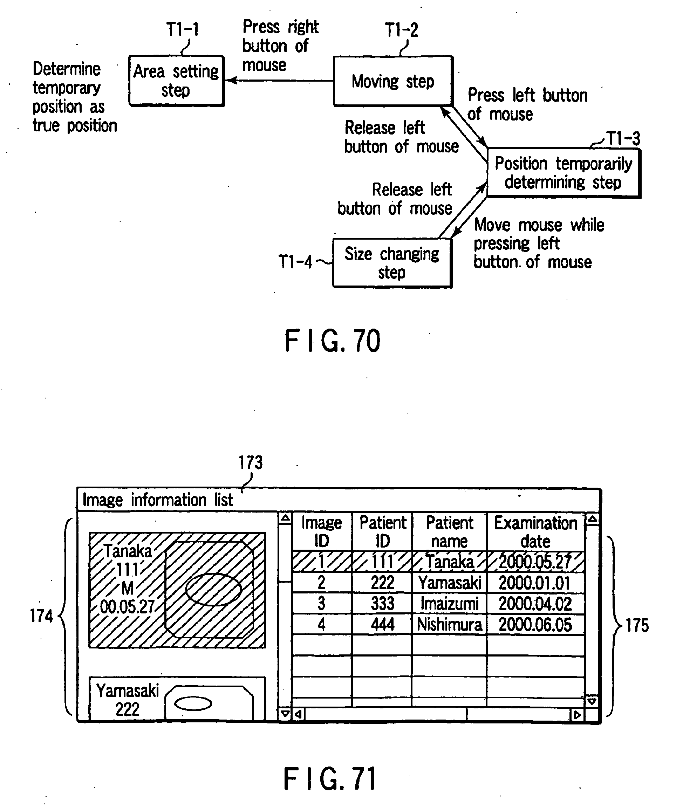

In this embodiment, an information list creating section 171 renders a region of interest on an image contained in an image data display area 174 of an image information list 173 on the basis of the image data of image information and region-of-interest data. FIG. 71 shows display of the image information list 173 in this embodiment.

The region-of-interest setting section 201 sets region-of-interest data corresponding to the image data with respect to the image information acquired from a display information management section 172 by operating a mouse #23 in the image data display area 174 of the image information list 173.

The image information containing the set region-of-interest data is held in an image information holding means 151 through an information updating section 182. In addition, display of the image data and region-of-interest data stored in the image data display area 174 is updated through the information updating section 182.

The display information management section 172 displays a menu 190 shown in FIG. 63 at the display position of a mouse cursor 176 upon selection of an image in the image data display area 174, a line in the accompanying data display area 174, and a graph element on graph information 160 by the operation of the mouse cursor 176. The menu 190 includes selected element information updating 191 and selected element region-of-interest setting 192.

When the selected element region-of-interest setting 192 is selected, the display information management section 172 acquires image information corresponding to the selection of an image in the image data display area 174, a line in the accompanying data display area 175, or a graph element on the graph information 160 with the mouse cursor 176, and outputs the information to an information setting section 181.

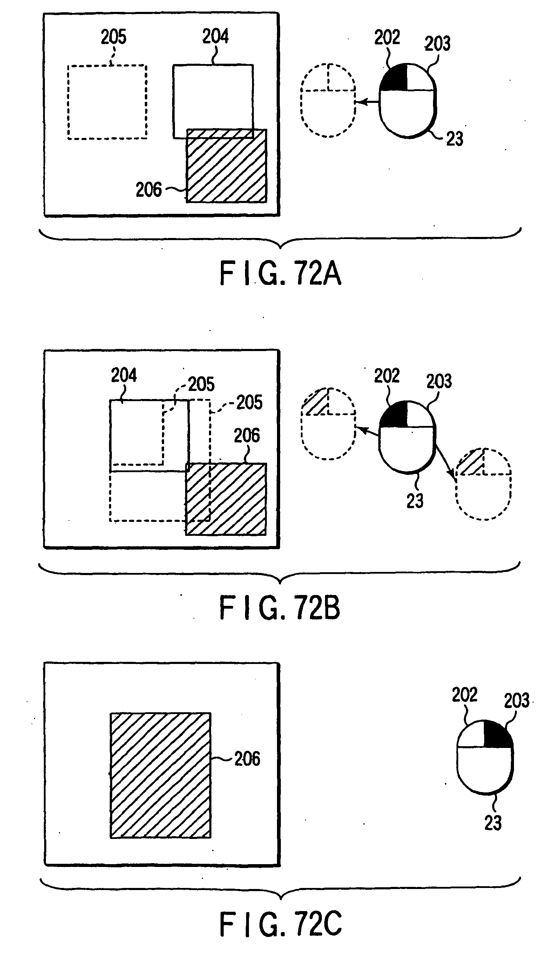

A region of interest is set by operating the mouse #23. As shown in FIG. 72A, the mouse #23 has a left button 202 and right button 203.

Assume that in this embodiment, a region of interest is rectangular.

FIG. 70 is a flowchart for the region-of-interest setting section 201, which explains how a region of interest is set in this embodiment. The following description is based on the assumption that a region of interest is set on an image in the image data display area 174 of the image information list 173, and the selected element information updating 191 has been selected from the menu 190 by the operation of the mouse #23.

This embodiment will be described with reference to FIGS. 72A, 72B, and 72C.

FIG. 72A shows the operation of the mouse 23 in a moving step (TI-2) and display on an image.

FIG. 72B shows the operation of the mouse 23 in a size changing step (TI-4) and display on an image.

FIG. 72C shows the operation of the mouse 23 in a position temporarily determining step (TI-3) and display on an image.

In addition to a region 206 of interest set on image information, a temporary region 204 of interest which is a temporary region of interest set on the image information, and an in-process region 205 of interest which is a region of interest which is being set by the operation of the mouse #23 are rendered/displayed on the image on which a region of interest is to be set.

On each of the images shown in FIGS. 72A, 72B, and 72C, the hatched closed area indicates the region 206 of interest set on the image information, the solid outlined closed area indicates the temporary region 204 of interest, and the dotted outlined closed area indicates the region 205 of interest that is being set by the operation of the mouse #23.

When the region-of-interest setting section 201 starts processing, the flow advances to the moving step (TI-2).

In the moving step (TI-2), the in-process region 205 of interest is moved/displayed in accordance with the movement of the mouse 23. When the operator moves the mouse 23 without pressing the left button 202 and right button 203 as shown in FIG. 72A, the in-process region 205 of interest moves in accordance with the movement of the mouse 23.

If the left button 202 is pressed in the moving step (TI-2), the flow advances to the position temporarily determining step (TI-3).

In the position temporarily determining step (TI-3), when the temporary region 204 of interest is erased and the right button 203 is released, the position and size of the in-process region 205 of interest are set to the position and size of the temporary region 204 of interest.

In the position temporarily determining step (TI-3), when the mouse #23 is moved while the right button 203 is pressed, the flow advances to the size changing step (TI-4). In the size changing step (TI-4), the upper left coordinates of the in-process region 205 of interest are fixed as an origin, and the size of the rectangle is changed in accordance with the movement of the mouse #23 with the left button 202 is being pressed.

When the left button 202 is pressed, the flow advances from the moving step (TI-2) to the position temporarily determining step (TI-3) to set/change the temporary region 204 of interest. The mouse #23 is then moved to cause the flow to advance to the size changing step (TI-4), in which the size of the in-process region 205 of interest is changed in accordance with the movement of the mouse #23 while the upper left coordinates of the rectangle of the in-process region 205 of interest are fixed.

In the size changing step (TI-4), when the left button 202 is released, the flow advances to the moving step (TI-2) through the position temporarily determining step (TI-3). The position and size of the in-process region 205 of interest are set to the position and size of the in-process region 205 of interest through the position temporarily determining step (TI-3).

In the moving step (TI-2), when the right button 203 is pressed, the flow advances to the area setting step (TI-1) to set the position and size of the temporary region 204 of interest as the region-of-interest data of the image information. The processing in the region-of-interest setting section 201 is then terminated.

The information updating section 182 holds the image information containing the region-of-interest data set by the region-of-interest setting section 201 in the image information holding means 151 through the storage means management section #123. In addition, the rendered display of the region of interest is updated with respect to the image data in an image data display area 187 through the display information management section 172.

In this embodiment, a region of interest is rectangular. However, it may be elliptic or an arbitrary set closed area. The rectangular area in this embodiment is replaced with a rectangle or arbitrary set closed area enclosing an ellipse, and the same operation as described above is performed. In this embodiment, in the size changing step (TI-4), the size is changed while the upper left coordinates of the rectangle are fixed as an origin. However, the size may be changed while the center of the rectangle is fixed as an origin.

(Effect)

A region of interest can be easily set because the position and size of the region of interest are set by performing region-of-interest setting operation once with the mouse #23.

[Seventh Embodiment]

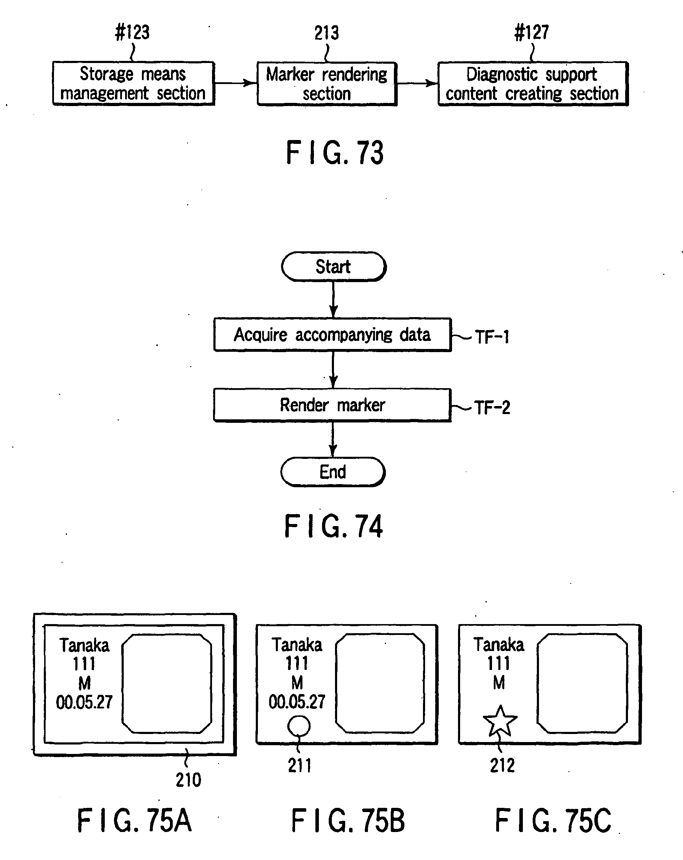

The seventh embodiment will be described with reference to the several views of the accompanying rendering. FIG. 73 is a block diagram of a main program #121 according to the seventh embodiment. No unnecessary portion is shown in this embodiment. The seventh embodiment is the same as the fourth embodiment except that a marker rendering section 213 is inserted between a storage means management section #123 and a diagnostic support content creating section #127.

The difference between the seventh and fourth embodiments will be described below.

The marker rendering section renders a frame 210 shown in FIG. 75A with respect to image data in accordance with the information of an item contained in the accompanying data of input image information. The image information containing the image data on which the frame is rendered is output to the diagnostic support content creating section #127.

In this embodiment, the frame 210 is rendered in accordance with information indicating a patient sex in the accompanying data. If the patient sex is male, the frame 210 is rendered with respect to the image data. If the patient sex is female, the frame 210 is not rendered.

FIG. 74 is a flowchart for the marker rendering section 213, which explains how a marker is rendered on image data in accordance with an item contained in accompanying data in this embodiment.

In step TF-1, the marker rendering section 213 acquires patient sex information in the accompanying data of input image information.

In step TF-2, if the patient sex information acquired in step TF-1 indicates male, the frame 210 is rendered as a maker with respect to the image data. If the patient sex information acquired in step TF-1 indicates female, no marker is rendered with respect to the image data.

In this embodiment, patient set information in accompanying data is used as an determination item for marker rendering. However, information such as a category classification, diagnosis name, examination region, or graph display attribute, which is classified as a classification key item, may be used as a determination item. Alternatively, each item stored in a numerical value item may be used as a determination item to perform determination in accordance with the range of values.

In this embodiment, the presence/absence of a rendered frame is used as a marker. However, the color tone of a frame may be changed in accordance with determination on a determination item. Alternatively, as shown in FIGS. 75B and 75C, a marker whose shape is changed, e.g., a circular marker 211 or star marker 212, may be rendered in image data in accordance with determination on a determination item. Furthermore, the display position of the circular marker 211 may be changed in accordance with determination on a determination item.

(Effect)

Since the contents of accompanying data can be known at a glance of a list of image data, operability is improved.

[Eighth Embodiment]

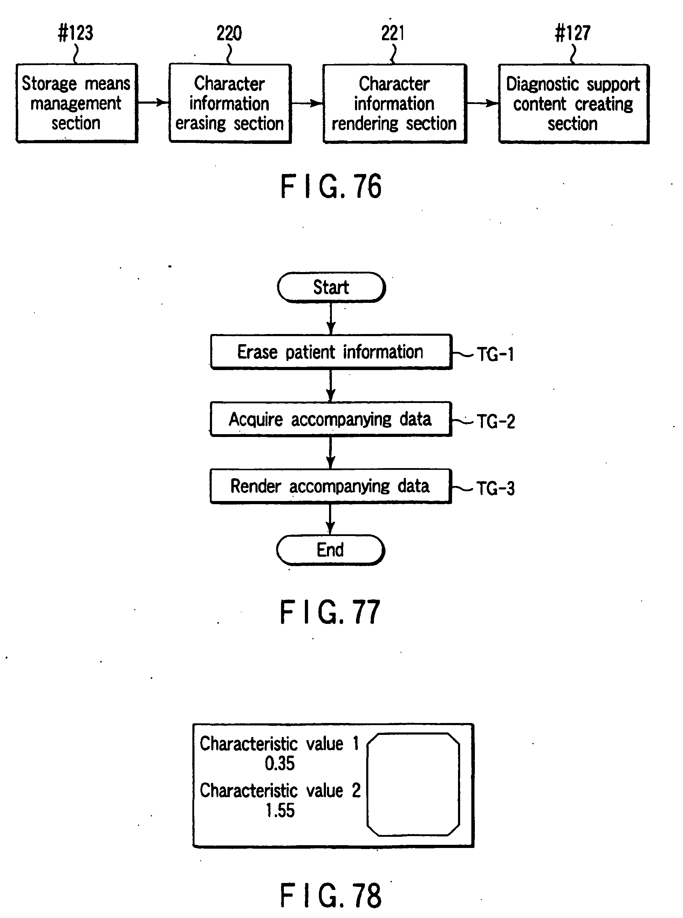

The eighth embodiment will be described with reference to the several views of the accompanying rendering. FIG. 76 is a block diagram of a main program #121 according to the eighth embodiment. No unnecessary portion is shown in this embodiment. The eighth embodiment is the same as the seventh embodiment except that a character information erasing section 240 and character information rendering section 241 are inserted in place of the marker rendering section 213.

The difference between the eighth and seventh embodiments will be described below.

The character information erasing section 240 performs erase processing with respect to an area in which patient examination information is rendered, of the image data of input image information, and erases the patient examination information rendered on the image data. The shape, position, and size of an area subject to erase processing are determined in advance.

The image information containing image data from which the patient examination information is erased is output to the character information rendering section 241.

The character information rendering section 241 renders, on the image data, the contents of items contained in the accompanying data of the input image information.

Assume that in this embodiment, the values of characteristic value 1 and characteristic value 2 in the accompanying data are rendered.

FIG. 77 is a flowchart for processing performed by the character information erasing section 240 and character information rendering section 241, which explains how patient examination information is erased from image data and item information contained in the accompanying data is rendered on the image data.

In step TG-1, the character information erasing section 240 erases patient examination information from the image data of input image information.

In step TG-2, the character information rendering section 241 renders the values of characteristic value 1 and characteristic value 2 contained in the accompanying data of the input image information on the image data having undergone the erase processing in step TG-1. The character information rendering section 241 also renders characteristic value 1 and characteristic value 2 as character strings representing the contents of rendering information above the respective values.

FIG. 78 shows a display example of the image data from which the patient examination information rendered thereon is erased and on which the values of characteristic value 1 and characteristic value 2 are rendered.

In this embodiment, characteristic value 1 and characteristic value 2 are rendered. However, other accompanying data items may be displayed together with their item names.

(Effects)

Since the patient examination information displayed on an image is erased, any leakage of patient privacy information can be prevented. In addition, since the patient examination information is erased, the blank area on the image data expands. The character size of character information to be newly superimposed/rendered can be increased. This makes the superimposed information easy to read and improves operability. In addition, since the information amount of character information to be superimposed/rendered can be increased, the operator can obtain more information by only looking at the image data display, resulting in improved operability.

[Ninth Embodiment]

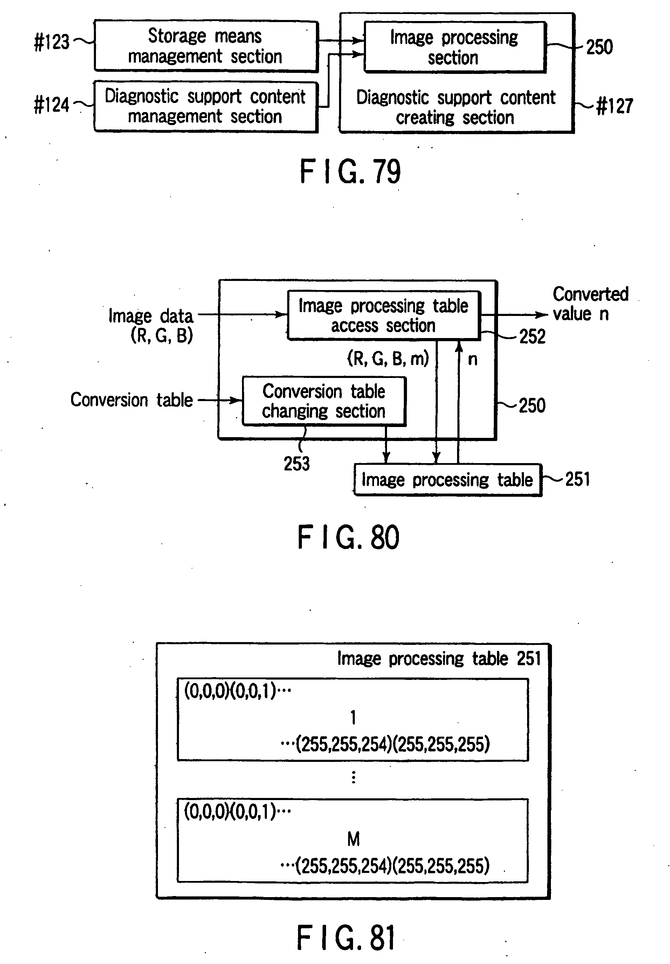

The ninth embodiment will be described with reference to the several views of the accompanying rendering. FIG. 79 is a block diagram of a main program #51 according to the ninth embodiment. No unnecessary portion is shown in this embodiment. The ninth embodiment is the same as the second embodiment except that an image processing section 250 is inserted in a diagnostic support content creating section #127.

The image processing section 250 acquires image information through a storage means management section #123, and calculates an image processing value on the basis of the image data contained in the image information. The image processing section 250 also acquires an image processing table 251 shown in FIG. 81 from a diagnostic support content management section #124, and holds it.

In this embodiment, image data is constituted by R, G, and B digital signal per pixel. R, G, and B each take a value from 0 to 255. The image processing value calculated by the image processing section 220 is set to an index of hemoglobin for each pixel.

The image processing section 250 receives the pixel data (R, G, B) of the image data, and outputs an image processing value $\underline{n}$ which is a converted value of the pixel data.

In this embodiment, $\underline{n}$ represents an index of hemoglobin. An index of hemoglobin is a value calculated from an expression of $32\log_2(R/G)$ using pixel data (R, G, B). This value reflects the magnitude of a mucosal blood flow, and is used as one of techniques of evaluating a mucosal color tone in the endoscopic medical field.

As shown in FIG. 81, the image processing table 251 has an array of M storage areas each having pixel values sequentially arranged on a byte basis from (R, G, B) = (0, 0, 0) to (R, G, B) = (255, 255, 255).

FIG. 80 is a block diagram showing the details of the image processing section 250. The image processing section 250 includes an image processing table access section 252 and conversion table changing section 253.

The image processing table access section 252 receives a pixel value (R, G, B) and outputs the converted value $\underline{n}$. The image processing table access section 252 holds $\underline{m}$ ($1 \leqq m \leqq M$), as a set value, corresponding to an index of hemoglobin, which is an identifier of an image processing type.

The image processing table access section 252 loads the access position of an image processing table on the basis of the input pixel value (R, G, B) and the image processing type identifier m, and outputs it as the image processing value n. In this embodiment, the image processing table access section 252 loads, as n, the table value at a position of m × 256 × 256 × 256 + R × 256 × 256 + G × 256 + B from the head of the image processing table.

The conversion table changing section 253 loads the image processing table from the diagnostic support content management section #124, changes the contents of the image processing table 251, and changes the image processing type identifier of the image processing table access section 252.

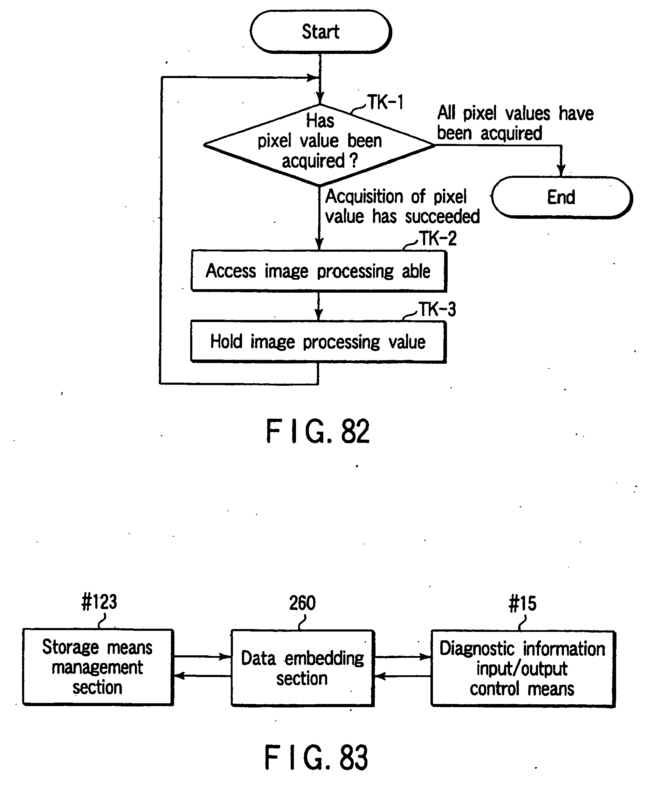

FIG. 82 is a flowchart for the image processing section 250, which explains how an image processing value corresponding to the pixel value of image data is calculated.

In step TK-1, the image processing section 250 acquires pixels one by one from the upper left pixel as a start position to the lower right pixel from the image data of image information by scanning it to the right line by line.

When all the pixels are acquired, the processing in the image processing section 250 is terminated.

In step TK-2, the image processing section 250 calculates m × 256 × 256 × 256 + R × 256 × 256 + G × 256 + B (to be referred to as an offset value hereinafter) from the acquired pixel (R, G, B) and the image processing identifier m held by itself, and acquires a table value at the position of the offset value from the head of the image processing table 251.

In step TK-3, the image processing section 250 holds the table value acquired in step TK-2.

With the above processing, the image processing section 250 holds the image processing value corresponding to a pixel of the image data of the image information. The image processing value is used by other component in the diagnostic support content creating section #127.

(Effect)

An image processing value can be obtained at high speed because it is computed by only addition and multiplication without any slow computations such as division and log computation.

[10th Embodiment]

In exchanging image information between terminals, when an image file converted into a general format (e.g., Windows or bitmap) is used, the contents of the image can be checked by using a user's favorite image viewer. Such a file is therefore often used.

However, region-of-interest data and accompanying data cannot be embedded in an image file in a general format. Conventionally, therefore, region-of-interest data and accompanying data are attached and output as other files. This complicates file management. Accompanying data contains private information of a patient. That is, if the accompanying data can be easily read, a problem arises in terms of privacy.

The 10th embodiment will be described with reference to the several views of the accompanying rendering. FIG. 83 is a block diagram of a main program #51 according to the 10th embodiment. No unnecessary portion is shown in this embodiment. The 10th embodiment is the same as the second embodiment except that a data embedding section 260 is added.

The data embedding section 260 loads image information from an image information holding means 151 through a storage means management section #123, embeds accompanying data and region-of-interest data in the image data, and outputs the resultant data to the outside through a diagnostic information input/output control means #15.

In addition, the data embedding section 260 acquires external image data in which accompanying data and region-of-interest data are embedded, creates image information by extracting the accompanying data and region-of-interest data from the image data, and holds the image information in the image information holding means 151 through the storage means management section #123.

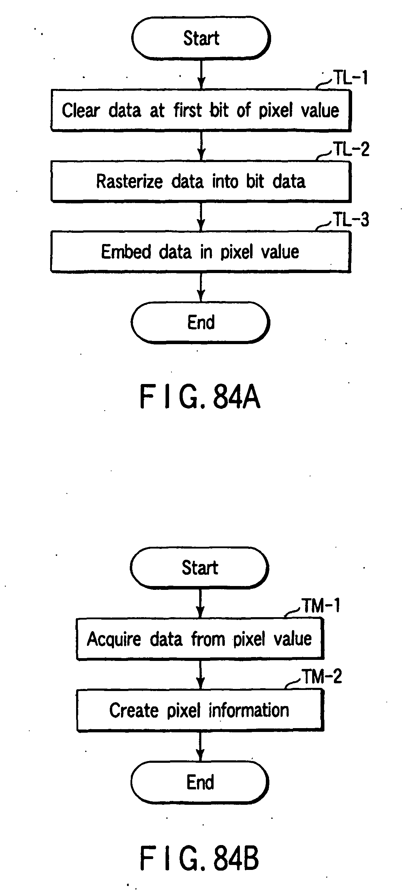

FIG. 84A is a flowchart for the data embedding section 260, which explains how accompanying data and region-of-interest data are embedded in image data.

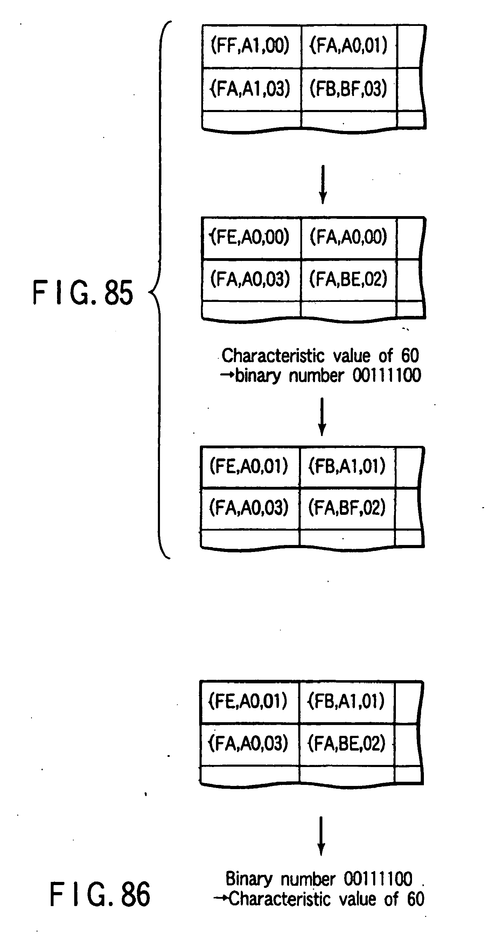

This operation will be described with reference to FIGS. 84A and 85.

In step TL-1, the data embedding section 260 sets the data at the first bit of the pixel values of loaded image data to 0. For example, at the upper position in FIG. 85, the pixel values of the loaded image data are arrayed as follows: (R, G, B) = (FF, A1, 00),.... When the processing of setting the data at the first bit to 0 in step TL-1 is applied to one upper left line, the pixel values are changed into (R, G, B) = (FE, A0, 00),..., as indicated at the middle position in FIG. 85.

In step TL-2, the accompanying data and region-of-interest data to be embedded are rasterized into bit data. If, for example, numerical data of 60 held as a characteristic value is rasterized into bit data, 00111100 is obtained.

In step TL-3, the data embedding section 260 embeds the accompanying data and image data, which have been rasterized into bit data in step TL-2, in the image data. Assume that the embedding position is set to the first bit of the pixel values of the image data. For example, at the lower position in FIG. 85, the characteristic value, 60, which has been rasterized into bit data in step TL-2, is embedded in one upper left line, and the resultant pixel values of the upper left line become (R, G, B) = (FE, A0, 01),....

With the above processing, the accompanying data and region-of-interest data are embedded in the image data.

FIG. 84B is a flowchart for the data embedding section 260, which explains how the accompanying data and region-of-interest data embedded in image data are acquired in this embodiment.

This operation will be described with reference to FIGS. 84B and 86.

In step TM-1, the data embedding section 260 acquires the data at the first bit of the pixel values of externally obtained image data. For example, referring to FIG. 86, the data embedding section 260 acquires the data at the first bit of the pixel data of one upper left line to obtain bit data 00111100.

In step TM-2, the data embedding section 260 acquires the accompanying data or region-of-interest data from the acquired bit data, creates image information, and holds the image information in the image information holding means 151 through the storage means management section #123. For example, referring to FIG. 86, the data embedding section 260 converts acquired bit data 00111100 into numerical data of 60, and creates image information by using this value as the value of the characteristic value of the accompanying data.

With the above processing, the accompanying data and region-of-interest data are extracted from the image data.

Assume that in this embodiment, the same pixel value range, the same correspondence between accompanying data and region-of-interest data, and the same correspondence between a pixel value arrangement and bit data arrangement are set in the two terminals.

(Effects)

The operability in image information exchange between terminals is improved by embedding accompanying data and region-of-interest data in image data. In addition, any leakage of the privacy information of a patient as a target image can be prevented.

[11th Embodiment]

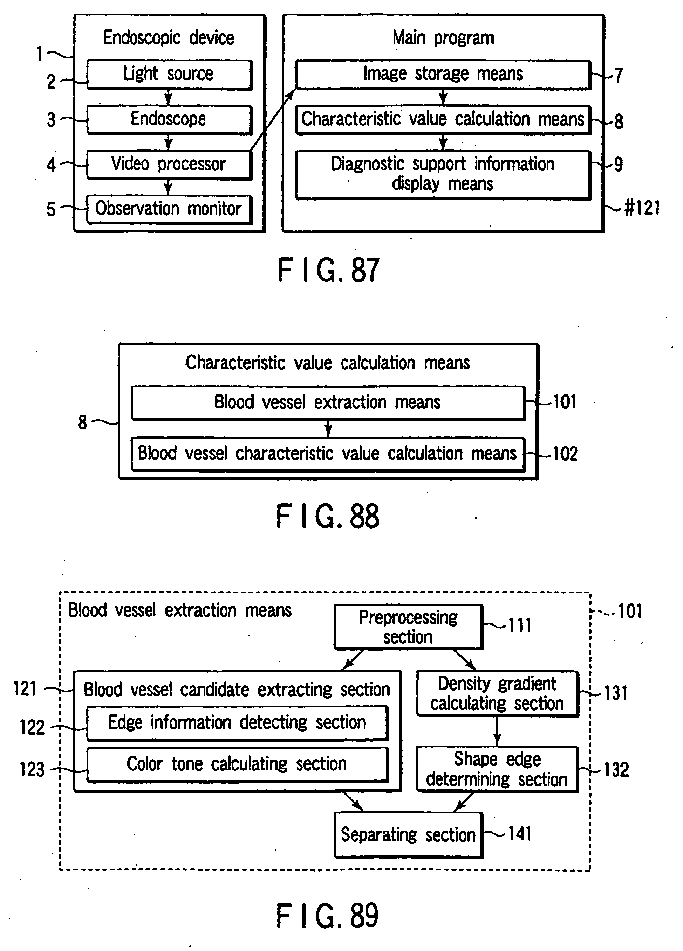

The 11th embodiment, which is an embodiment of the present invention, is characterized in a characteristic value calculation technique aimed at objectifying findings in endoscopic images. More specifically, this embodiment is configured to extract a see-through blood vessel image (to be referred to as a blood vessel image hereinafter) and calculate a characteristic value associated with the blood vessel running state of the image. The 11th embodiment will be described with reference to FIGS. 87 to 98.

(Arrangement)

A diagnostic support apparatus in the 11th embodiment has the same arrangement as that in the second embodiment, and hence a detailed description thereof will be omitted.

A method of calculating a characteristic value in this embodiment will be described below, assuming that the method is executed by a blood vessel characteristic value calculation means 102.

FIG. 87 is a view of a main program #121 having a characteristic value calculation means 008 according to the 11th embodiment. The main program #121 is comprised of an image storage means 007 for storing image data input from a video processor 004 in an endoscopic device 001 through a diagnostic information input/output control means #15, the characteristic value calculation means 008 for calculating a characteristic value from the image data stored in the storage means, and a diagnostic support information display means 009 for displaying diagnostic support information on the basis of the characteristic value calculated by the characteristic value calculation means.

FIG. 88 is a view showing the arrangement of the characteristic value calculation means 008 in the 11th embodiment. The characteristic value calculation means 008 is comprised of a blood vessel extraction means 101 for extracting a blood vessel image in the image data stored in the image storage means 007, and a blood vessel characteristic value calculation means 102 for evaluating a blood vessel running state on the basis of the output from the blood vessel extraction means 101, and calculating a characteristic value.

FIG. 89 is a view showing the arrangement of the blood vessel extraction means 101 in the characteristic value calculation means 008. The blood vessel extraction means 101 is comprised of a preprocessing section 111 which performs preprocessing for the image data, a blood vessel candidate extracting section 121 which extracts a blood vessel candidate on the basis of an output from the preprocessing section 111, a density gradient calculating section 131 which calculates density gradient information using the image data on the basis of an output from the preprocessing section 121, a shape edge determining section 132 which determines a shape edge on the basis of an output from the density gradient calculating section 131, and a separating section 141 which separates and removes a shape edge from a blood vessel candidate on the basis of outputs from the blood vessel candidate extracting section 121 and shape edge determining section 132. The blood vessel candidate extracting section 121 is comprised of an edge information detecting section 122 and color tone calculating section 123.

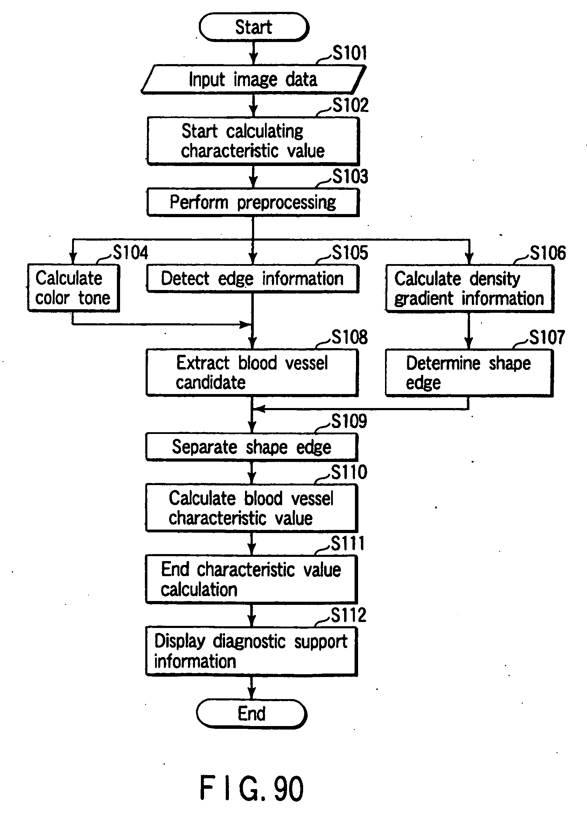

FIG. 90 is a flowchart for mainly explaining processing in the blood vessel extraction means 101.

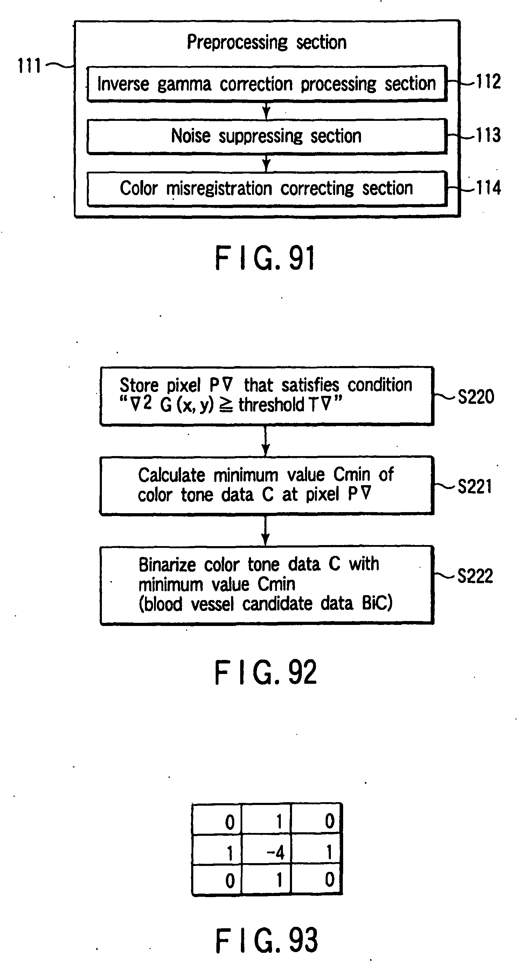

FIG. 91 is a block diagram of the preprocessing section 111. The preprocessing section 111 is comprised of the following blocks: an inverse gamma correction processing section 112 which cancels gamma correction applied to the image data, a noise suppressing section 113 which suppresses noise in the image data, and a color misregistration correcting section 114 which corrects displacements between color signals due to the difference in imaging timing between the respective types of color signals when the image data is constituted by a plurality of color signals.

FIG. 92 is a schematic flowchart showing processing in the blood vessel candidate extracting section 121 which extracts a blood vessel candidate on the basis of outputs from the edge information detecting section 122 and color tone calculating section 123.

FIG. 93 shows an example of a spatial filter for performing second-order differentiation processing in the edge information detecting section 122.

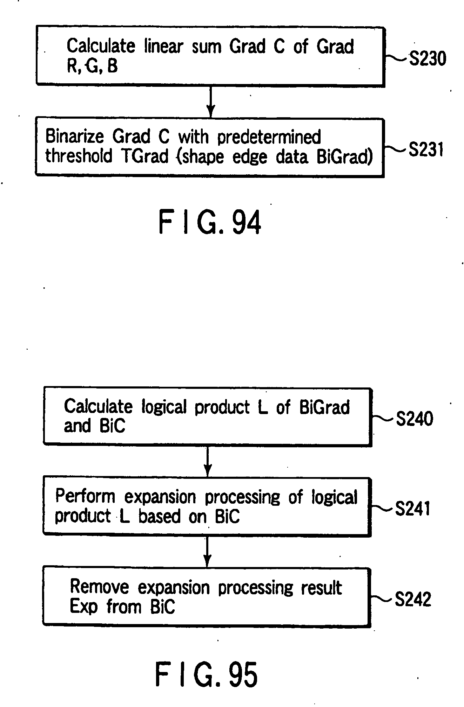

FIG. 94 is a schematic flowchart showing processing in the shape edge determining section 132 based on an output from the density gradient calculating section 131. FIG. 95 is a schematic flowchart showing the processing of separating and removing a shape edge from a blood vessel candidate on the basis of the results obtained by the blood vessel candidate extracting section 121 and shape edge determining section 132.

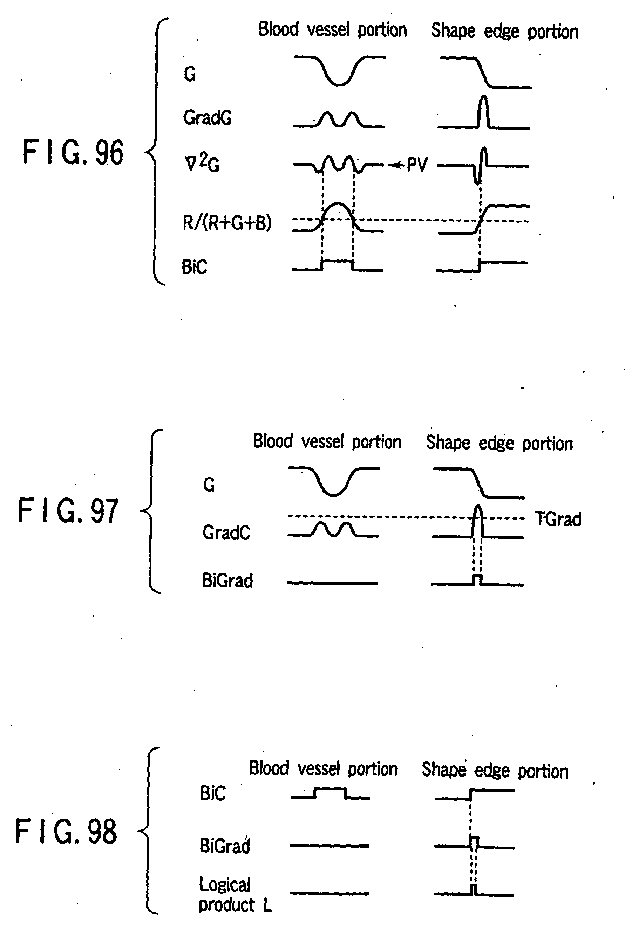

FIG. 96 is a conceptual view of a density distribution, density gradient, second-order differentiation, color tone data, and blood vessel candidate data (to be described later) on a horizontal line of an image on which a blood vessel and shape edge exist.

FIG. 97 is a conceptual view of the density distribution, density gradient, shape edge data based on shape edge determination (to be described later) at a blood vessel and shape edge. FIG. 98 is a conceptual view of the logical product of the blood vessel candidate data and shape edge data at a blood vessel and shape edge.

(Operation)

In this embodiment, the image data input from the video processor 004 and recorded on the image storage means 007 is constituted by three image data, i.e., R, G, and B image data, obtained by a field sequential type encoscope.

The characteristic value calculation means 008 reads out a predetermined area of the image data (image data in a set region of interest) from the image storage means 007 (step S101). The blood vessel extraction means 101 shown in FIG. 88 then performs blood vessel image extraction processing (step S102).

As shown in FIG. 89, the blood vessel extraction means 101 inputs R, G, and B image data to the preprocessing section 111.

As shown in FIG. 91, in the preprocessing section 111, the inverse gamma correction processing section 112 performs inverse gamma correction for each of the R, G, and B image data by looking up a predetermined correction table, and outputs the result to the noise suppressing section 113. The noise suppressing section 113 performs noise suppression using a median filter having a mask size of $3 \times 3$. The noise suppression result is input to the color misregistration correcting section 114. The color misregistration correcting section 114 calculates a correlation coefficient between the G and R image data when the R image data is shifted from the G image data by predetermined numbers of pixels in the horizontal and vertical directions, and the R image data is shifted by a shift amount that provides a maximum value, thus terminating the correction processing. The above correction processing is executed with respect to the B image data with reference to the G image data in the same manner as described above. This operation corrects the color misregistration between the R, G, and B image data which is caused in the field sequential type endoscope. The image data whose misregistration with respect to the G image data is corrected by the color misregistration correction processing are newly called R, G, and B image data, and the image data at the respective pixels are represented by R $(x, y)$, G$(x, y)$, and B$(x, y)$. Note that $\underline{x}$ and $\underline{y}$ represent coordinate positions in the image data in the horizontal and vertical directions (the above operation is done in step S103).

The R, G, and B image data obtained by the preprocessing section 111 are input to the blood vessel candidate extracting section 121 and density gradient calculating section 131.

As shown in FIG. 89, in the blood vessel candidate extracting section 121, the image data obtained by the preprocessing section 111 are input to the edge information detecting section 122 and color tone calculating section 123.

The edge information detecting section 122 performs second-order differentiation processing for the G image data (step S105). In the above processing, convolution computation of the G image data is performed using a $3 \times 3$ spatial filter like the one shown in FIG. 93. The result obtained by the above computation is represented by $\nabla^2 G(x, y)$.

The color tone calculating section 123 calculates color tone data $C(x, y)$ from the R, G, and B image data according to mathematical expression ※1 (step S104).

$$R(x, y)/(R(x, y) + G(x, y) + B(x, y))$$

expression ※1

As shown in FIG. 92, the blood vessel candidate extracting section 121 stores, in a memory (not shown), a pixel P∇, of ∇²G(x, y) output from the edge information detecting section 122, which has a value equal to or larger than a predetermined threshold T∇ (step S220). With respect to the pixel P∇, the blood vessel candidate extracting section 121 calculates a minimum value Cmin of the color tone data C(x, y) output from the color tone calculating section 123 (step S221). The blood vessel candidate extracting section 121 further executes binarization processing of assigning value 1 to each pixel whose color tone data C(x, y) is equal to or more than the minimum value Cmin and assigning 0 to each pixel whose color tone data is less than Cmin (steps S222 and S108). The obtained binarized data is output as blood vessel candidate data BiC(x, y) to the separating section 141. As shown in FIG. 96, the blood vessel candidate data BiC(x, y) contains a shape edge portion together with a blood vessel portion, and hence the shape edge portion is separated by the separating section 141 (to be described later).

The density gradient calculating section 131 calculates the gradient of the R image data from the preprocessing section 111 according to mathematical expression ※2 (step S106) .

$$|R(x+1, y) - R(x, y)| + |R(x, y+1) - R(x, y)|$$

expression ※2

where | | indicates an absolute value.

The density gradient calculating section 131 also calculates the gradients of the G and B image data in the same manner, and outputs the results to the shape edge determining section 132. Note that the obtained results are respectively represented by Grad R(x, y), Grad G(x, y), and Grad B(x, y).

As shown in FIG. 94, the shape edge determining section 132 calculates a linear sum Grad C(x, y) of Grad R (x, y), Grad G(x, y), and Grad B(x, y) output from the density gradient calculating section 131 according to equation ※3 (steps S230 and S107) .

$$\text{Grad C}(x, y)$$
$$= \alpha \cdot \text{Grad R}(x, y) + \beta \cdot \text{Grad G}(x, y) + \theta \cdot \text{Grad B}(x, y)$$

expression ※3

Predetermined values are used as weighting factors α, β, and θ, and all are set to 1 in this embodiment.

The difference in Grad C between a blood vessel portion and a shape edge portion will be described. Blood vessel images differ in contrast among R, G, and B image data depending on differences in depth at which blood vessels run in the mucous membrane. More specifically, a blood vessel at a deep depth is formed into R image data by imaging return light of irradiation light of R (red) having a long wavelength, a blood vessel at a shallow depth is formed into B image data, and a blood vessel at an intermediate depth is formed into G image data. The density gradient of a blood vessel portion is smaller than that of a shape edge portion. In contrast to this, at a shape edge, the difference in contrast between image data is relatively small, and the difference in density gradient is relatively large. For this reason, as shown in FIG. 97, the linear sum Grad C of the shape edge portion is large.

Threshold processing is performed for the linear sum Grad C(x, y) using a predetermined threshold TGrad, and binarization is performed such that value 1 is assigned to each pixel whose Grad C(x, y) is equal to or more than TGrad, and value 0 is assigned to each pixel whose Grad C(x, y) is less than TGrad (step S231), thereby creating shape edge data BiGrad(x, y). This data is output to the separating section 141.

As shown in FIG. 95, the separating section 141 calculates a logical product L(x, y) of the blood vessel candidate data BiC(x, y) output from the blood vessel candidate extracting section 121 and the shape edge data BiGrad(x, y) output from the shape edge determining section 132 (step S240). A portion based on the shape edge in the blood vessel candidate data is obtained from this result, as indicated by the conceptual view of FIG. 98. Since the logical product L does not include all blood vessel candidate data based on the shape edge, the separating section 141 executes expansion processing with respect to the logical product L (step S241), and removes expanded data Exp as the obtained result from the blood vessel candidate data BiC, thereby separating the shape edge portion (steps S242 and S109). That is, if the blood vessel candidate data BiC at a pixel (i, j) of the logical product L(x, y) or any one of eight pixels adjacent to (i, j) is value 1, the value of expanded data Exp(i, j) is set to 1; otherwise, value 0 is assigned to the expanded data, thereby creating expanded data Exp(x, y), which is subtracted from the blood vessel candidate data BiC(x, y).

With this operation, blood vessel extraction data Bv(x, y) can be created, which is the image data of an image vessel image obtained by separating the shape edge from the blood vessel candidate data.

The blood vessel extraction data Bv(x, y) created by the blood vessel extraction means 101 is output to the blood vessel characteristic value calculation means 102.

The blood vessel characteristic value calculation means 102 counts the number of pixels constituting a blood vessel image in the blood vessel extraction data Bv(x, y) created by the blood vessel extraction means 101, and calculates the ratio of the counted number of pixels to the number of pixels in the predetermined area, thereby calculating a blood vessel area ratio as a characteristic value which indicates the proportion of the blood vessel to the predetermined area (step S110). The blood vessel area ratio is output to the diagnostic support information display means 009.

After the characteristic value is calculated (step S111), the diagnostic support information display means 009 displays the numerical value of the blood vessel area ratio calculated by the blood vessel characteristic value calculation means 102 as a quantitative evaluation value associated with the running state of the blood vessel image (step S112).

(Effect)

According to the above arrangement, since a shape edge in the living body can be separated and extracted from a blood vessel image, erroneous extraction can be suppressed, and quantitation can be realized in accordance with a blood vessel.

(Effects of Embodiments)

According to the first embodiment, the diagnostic support apparatus can selectively use diagnostic support in accordance with diagnosis purposes and contents, and can use latest diagnostic support contents.

According to the diagnostic support apparatus of the (1-B)th embodiment, when a diagnostic support content is updated/added in the diagnostic support content server, the content is distributed to the diagnostic support execution terminal, thereby always allowing the use of the latest diagnostic support content in a diagnosis.

According to the diagnostic support apparatus of the (1-C)th embodiment, a latest diagnostic support content can always be used by inquiring the diagnostic support content server whether a diagnostic support content is updated or added.

According to the diagnostic support apparatus of the second embodiment, many medical facilities/organizations can arbitrarily create diagnostic support contents, various medical information, image data, and expert medical knowledge accumulated in the respective facilities/organizations can be widely used on diagnostic support apparatuses. In addition, since, for example, data can be easily added to a created diagnostic support content, case data dispersed in many medical facilities/organizations can be effectively used to improve the performance of the diagnostic support apparatus.

According to the third embodiment, displaying statistically processed information on a graph facilitates comparison with statistically processed information and provides an objective understanding of graph display based on the statistically processed information.

In addition, in creating a graph, classification items and data values are separately displayed and selected. This prevents the operator from mistakenly selecting a classification item as a data value or mistakenly selecting a data value as a classification item, and improves operability. Since a combination of a plurality of selected classification items is used as a classification item, the labor spent to create a graph is reduced. Since only items selected from a combination of a plurality of selected classification items are used as classification items, the labor spent to create a graph is reduced.

According to the fourth embodiment, images, accompanying data, and graph elements can be referred to in association with each other, resulting in improved operability.

According to the fifth embodiment, an improvement in operability in graph creation and prevention of error setting of image information can be realized. In providing information, a menu of a list of choices to be input is created and displayed in accordance with hierarchical information and the contents set in higher hierarchical levels. This prevents the operator from erroneously inputting information to a choice that cannot be selected. In addition, since no choice that cannot be selected is displayed, the display becomes easy to read, and the operability is improved. Furthermore, in accordance with the contents set at higher hierarchical levels, the names of items to be input are updated/displayed, and items to be input are set. This prevents the operator to erroneously input information to items that cannot be selected. In addition, since unnecessary display in the window is omitted, the operability is improved.

According to the sixth embodiment, a region of interest can be easily set on an image.

According to the seventh embodiment, accompanying data can be easily recognized with respect to image data.

According to the eighth embodiment, since the patient examination information displayed on an image is erased, a leakage of patient privacy information can be prevented.

According to the ninth embodiment, an image processing value can be obtained at high speed because it is computed by only addition and multiplication without any slow computations such as division and log computation.

According to the 10th embodiment, the operability in image information exchange between terminals is improved by embedding accompanying data and region-of-interest data in image data. In addition, a leakage of the privacy information of a patient as a target image can be prevented.

Industrial Applicability

According to the present invention, various information, image data, and expert medical knowledge accumulated in many medical facilities can be widely used on diagnostic support apparatuses. In addition, the performance of the diagnostic support apparatus can be improved. Diagnostic support can be selectively used in accordance with purposes and contents. Furthermore, various processes and operations required to create diagnostic support contents can be easily and effectively assisted.

**Claims**

1. A diagnostic support apparatus for supporting a diagnosis by an examiner, comprising
   image storage means (7) for storing image data input from an endoscopic device (1),
   characteristic value calculation means (8) for calculating at least one characteristic value to quantify a finding associated with a diagnosis from image data stored in the image storage means (7), and
   diagnostic support information display means (9) for displaying diagnostic support information on the basis of a calculation result obtained by the characteristic value calculation means (8),
   the characteristic value calculation means (8) comprising
   blood vessel extraction means (101) for extracting a blood vessel image from the image data, which is stored in the image storage means (7) as R, G and B image data, and
   blood vessel characteristic value calculation means (102) for calculating a blood vessel area ratio on the basis of the output from the blood vessel extraction means (101),
   the blood vessel extraction means (101) comprising
   blood vessel candidate extraction means (121),
   gradient information determining means (131) which determine density gradient data based on the R, G and B image data,
   shape edge determining means (132) which determine sum gradient data based on the density gradient data and binarize the sum gradient data in order to obtain edge data of a blood vessel, and
   separation means (141) which determine blood vessel image data based on the blood vessel candidate data and the edge data,
   the blood vessel candidate extraction means (121) comprising
   an edge information detecting section (122) which performs second-order differentiation processing based on the G image data, and
   a color tone calculating section (123) which calculates color tone data based on the R, G and B image data, wherein
   the blood vessel candidate extraction means (121) extract blood vessel candidate data based on an output from the edge information detecting section (122) and the color tone calculating section (123).

2. The diagnostic support apparatus according to claim 1, wherein
   the edge information detection section (122) detects edge information with respect to at least one color signal from image data constituted by a plurality of color signals and stored in the image storage means (7), and
   the color tone calculating section (123) calculates a value associated with a color tone with respect to at least one color signal from image data constituted by a plurality of color signals and stored in the image storage means (7).

3. The diagnostic support apparatus according to claim 1 or 2, wherein
   the gradient information determining means (131) determine density gradient information of image data constituted by a plurality of color signals and stored in the image storage means (7), and
the shape edge determining means (132) determine the shape edge data on the basis of threshold processing for a linear sum of density gradient information of a plurality of color signals output from the gradient information determining means (131).

4. The diagnostic support apparatus according to one of claims 1 to 3, wherein the separation means (141) determine blood vessel image data by means of expansion processing.

5. The diagnostic support apparatus according to one of claims 1 to 4, wherein the color tone calculating section (123) sets a value calculated by $R/(R + G + B)$ as a value associated with a color tone when the plurality of color signals comprise R, G, and B.

6. A diagnostic support method of supporting a diagnosis by an examiner, comprising the steps of:

storing image data input from an endoscopic device (1) in image storage means (7),
calculating at least one characteristic value to quantify a finding associated with a diagnosis from image data stored in the image storage means (7) by characteristic value calculation means (8), and
displaying diagnostic support information on the basis of a calculation result obtained by the characteristic value calculation means (8) in diagnostic support information display means (9), wherein
the characteristic value calculation means (8) provide the steps of extracting a blood vessel image from the image data, which is stored in the image storage means (7) as R, G and B image data by blood vessel extraction means (101), and
calculating a blood vessel area ratio on the basis of the output from the blood vessel extraction means (101) in blood vessel characteristic value calculation means (102), wherein
the blood vessel extraction means (101) provide the steps of
performing second-order differentiation processing based on the G image data by an edge information detecting section (122) of a blood vessel candidate extraction means (121),
calculating color tone data based on the R, G and B image data by a color tone calculating section (123) of the blood vessel candidate extraction means (121),
extracting blood vessel candidate data based on an output from the edge information detecting section (122) and the color tone calculating section (123),
determining density gradient data based on the R, G and B image data by gradient information determining means (131),
determining sum gradient data based on the density gradient data and binarizing the sum gradient data in order to obtain edge data of a blood vessel by shape edge determining means (132), and
determining blood vessel image data based on the blood vessel candidate data and the edge data by separation means (141).

7. The diagnostic support method according to claim 6, wherein
the edge information detection section (122) detects edge information with respect to at least one color signal from image data constituted by a plurality of color signals and stored in the image storage means (7), and
the color tone calculating section (123) calculates a value associated with a color tone with respect to at least one color signal from image data constituted by a plurality of color signals and stored in the image storage means (7).

8. The diagnostic support method according to claim 6 or 7, wherein
the gradient information determining means (131) determine density gradient information of image data constituted by a plurality of color signals and stored in the image storage means (7), and
the shape edge determining means (132) determine the shape edge data on the basis of threshold processing for a linear sum of density gradient information of a plurality of color signals output from the gradient information determining means (131).

9. The diagnostic support method according to one of claims 6 to 8, wherein the separation means (141) determine blood vessel image data by means of expansion processing.

10. The diagnostic support method according to one of claims 6 to 9, wherein the color tone calculating section (123) sets a value calculated by $R/(R + G + B)$ as a value associated with a color tone when the plurality of color signals comprise R, G, and B.

**Patentansprüche**

1. Diagnose-Hilfsvorrichtung zum Unterstützen einer Diagnose durch einen Untersucher, aufweisend Bildspeichermittel (7) zum Speichern von über ein endoskopisches Gerät (1) eingegebenen Bilddaten, Berechnungsmittel (8) eines charakteristischen Werts zum Berechnen mindestens eines charakteristischen Werts zum Quantifizieren eines Befunds in Verbindung mit einer Diagnose aus in dem Bildspeichermittel (7) gespeicherten Bilddaten, und

   Diagnose-Hilfsinformations-Anzeigemittel (9) zum Anzeigen diagnostischer Hilfsinformation auf der Basis eines von dem Berechnungsmittel (8) eines charakteristischen Werts erhaltenen Rechenergebnisses,

   wobei die Berechnungsmittel (8) eines charakteristischen Werts umfassen

   Blutgefäß-Extraktionsmittel (101) zum Extrahieren eines Blutgefäßbildes aus den Bilddaten, die in dem Bildspeichermittel (7) als R-, G- und B-Bilddaten gespeichert sind, und

   Berechnungsmittel (102) eines charakteristischen Blutgefäßwerts zum Berechnen eines Blutgefäß-Flächenverhältnisses auf der Basis der Ausgabe von den Blutgefäß-Extraktionsmitteln (101),

   wobei die Blutgefäß-Extraktionsmittel (101) aufweisen

   Blutgefäßproben-Extraktionsmittel (121), Gradienteninformation-Bestimmungsmittel (131), die Dichte-Gradientendaten basierend auf den R-, G- und B-Bilddaten bestimmen,

   Formrand-Bestimmungsmittel (132), die basierend auf den Dichte-Gradientendaten Summen-Gradientendaten bestimmen und die Summen-Gradientendaten binarisieren, um Randdaten eines Blutgefäßes zu erhalten, und

   Trennmittel (141) zum Bestimmen von Blutgefäß-Bilddaten auf der Basis der Blutgefäßproben-Daten und der Randdaten,

   wobei die Blutgefäßproben-Extraktionsmittel (121) aufweisen

   einen Randinformation-Erfassungsabschnitt (122), der basierend auf den G-Bilddaten eine Differentialverarbeitung zweiter Ordnung durchführt, und

   einen Farbton-Berechungsabschnitt (123), der basierend auf den R-, G- und B-Bilddaten Farbtondaten berechnet, wobei

   die Blutgefäßproben-Extraktionsmittel (121) Blutgefäßproben-Daten basierend auf einer Ausgabe aus dem Randinformation-Erfassungsabschnitt (122) und dem Farbton-Berechungsabschnitt (123) extrahieren.

2. Diagnose-Hilfsvorrichtung nach Anspruch 1, wobei

   der Randinformation-Erfassungsabschnitt (122) Randinformation bezüglich mindestens eines Farbsignals aus Bilddaten, die aus mehreren Farbsignalen gebildet sind und in dem Bildspeichermittel (7) gespeichert sind, erfasst, und

   der Farbton-Berechungsabschnitt (123) einen Wert in Verbindung mit einem Farbton bezüglich mindestens eines Farbsignals aus Bilddaten, die aus mehreren Farbsignalen gebildet sind und in dem Bildspeichermittel (7) gespeichert sind, berechnet.

3. Diagnose-Hilfsvorrichtung nach Anspruch 1 oder 2, wobei

   die Gradienteninformation-Bestimmungsmittel (131) Dichte-Gradienteninformation aus Bilddaten bestimmen, die aus mehreren Farbsignalen gebildet sind und in dem Bildspeichermittel (7) gespeichert sind, und

   die Formrand-Bestimmungsmittel (132) die Formranddaten auf der Basis einer Schwellenwertverarbeitung für eine lineare Summe von Dichte-Gradienteninformation aus mehreren von dem Gradienteninformation-Bestimmungsmittel (131) ausgegebenen Farbsignalen bestimmen.

4. Diagnose-Hilfsvorrichtung nach einem der Ansprüche 1 bis 3, wobei die Trennmittel (141) Blutgefäß-Bilddaten mittels Expansionsverarbeitung bestimmen.

5. Diagnose-Hilfsvorrichtung nach einem der Ansprüche 1 bis 4, wobei der Farbton-Berechnungsabschnitt (123) einen durch $R/(R + G + B)$ berechneten Wert als einen einem Farbton zugeordneten Wert festsetzt, wenn die mehreren Farbsignale R, G und B umfassen.

6. Diagnose-Hilfsverfahren zum Unterstützen einer Diagnose durch einen Untersucher, mit folgenden Schritten:

   Speichern einer Bilddateneingabe von einem endoskopischen Gerät (1) in Bildspeichermitteln (7),
   Berechnen mindestens eines charakteristischen Werts zum Quantifizieren eines Befunds in Verbindung mit einer Diagnose aus in den Bildspeichermitteln (7) gespeicherten Bilddaten durch Berechnungsmittel (8) eines charakteristischen Werts, und
   Anzeigen diagnostischer Hilfsinformation auf der Basis eines von dem Berechnungsmittel (8) eines charakteristischen Werts erhaltenen Rechenergebnisses durch Diagnose-Hilfsinformations-Anzeigemittel (9), wobei

die Berechnungsmittel (8) eines charakteristischen Werts folgende Schritte vorsehen:

Extrahieren eines Blutgefäßbildes aus den Bilddaten, die in dem Bildspeichermittel (7) als R-, G- und B-Bilddaten gespeichert sind, durch Blutgefäß-Extraktionsmittel (101), und

Berechnen eines Blutgefäß-Flächenverhältnisses auf der Basis der Ausgabe aus den Blutgefäß-Extraktionsmitteln (101) in Berechnungsmitteln (102) eines charakteristischen Blutgefäßwerts,

wobei die Blutgefäß-Extraktionsmittel (101) die folgenden Schritte vorsehen:

Durchführen einer Differentialverarbeitung zweiter Ordnung basierend auf den G-Bilddaten durch einen Randinformation-Erfassungsabschnitt (122) eines Blutgefäßproben-Extraktionsmittels (121),

Berechnen von Farbtondaten basierend auf den R-, G- und B-Bilddaten durch einen Farbton-Berechungsabschnitt (123) des Blutgefäßproben-Extraktionsmittels (121),

Extrahieren von Blutgefäßprobendaten basierend auf einer Ausgabe aus dem Randinformation-Erfassungsabschnitt (122) und dem Farbton-Berechnungsabschnitt (123)

Bestimmen von Dichte-Gradientendaten basierend auf den R-, G- und B-Bilddaten durch Gradienteninformation-Bestimmungsmittel (131),

Bestimmen von Summen-Gradientendaten basierend auf den Dichte-Gradientendaten und Binarisieren der Summen-Gradientendaten, um Randdaten eines Blutgefäßes durch Formrand-Bestimmungsmittel (132) zu erhalten, und

Bestimmen von Blutgefäß-Bilddaten basierend auf den Blutgefäß-Probedaten und den Randdaten durch Trennmittel (141).

**7.** Diagnose-Hilfsverfahren nach Anspruch 6, wobei

der Randinformation-Erfassungsabschnitt (122) Randinformation bezüglich mindestens eines Farbsignals aus Bilddaten, die aus mehreren Farbsignalen gebildet sind und in dem Bildspeichermittel (7) gespeichert sind, erfasst, und

der Farbton-Berechungsabschnitt (123) einen Wert in Verbindung mit einem Farbton bezüglich mindestens eines Farbsignals aus Bilddaten berechnet, die aus mehreren Farbsignalen gebildet sind und in dem Bildspeichermittel (7) gespeichert sind.

**8.** Diagnose-Hilfsverfahren nach Anspruch 6 oder 7, wobei

die Gradienteninformation-Bestimmungsmittel (131) Dichte-Gradienteninformation aus Bilddaten bestimmen, die aus mehreren Farbsignalen gebildet sind und in dem Bildspeichermittel (7) gespeichert sind, und

die Formrand-Bestimmungsmittel (132) die Formranddaten auf der Basis einer Schwellenwertverarbeitung für eine lineare Summe von Dichtegradienteninformation aus mehreren von dem Gradienteninformation-Bestimmungsmittel (131) ausgegebenen Farbsignalen bestimmen.

**9.** Diagnose-Hilfsverfahren nach einem der Ansprüche 6 bis 8, wobei die Trennmittel (141) Blutgefäß-Bilddaten mittels Expansionsverarbeitung bestimmen.

**10.** Diagnose-Hilfsverfahren nach einem der Ansprüche 6 bis 9, wobei der Farbton-Berechnungsabschnitt (123) einen durch R/(R + G + B) berechneten Wert als einen einem Farbton zugeordneten Wert festsetzt, wenn die mehreren Farbsignale R, G und B umfassen.

## Revendications

**1.** Appareil de support de diagnostic pour supporter un diagnostic par un examinateur, comprenant

un moyen de stockage d'images (7) pour stocker des données d'images entrées d'un dispositif endoscopique (1),

un moyen de calcul de valeurs caractéristiques (8) pour calculer au moins une valeur caractéristique pour quantifier une découverte associée à un diagnostic à partir de données d'images stockées dans le moyen de stockage d'images (7), et

un moyen d'affichage d'informations de support de diagnostic (9) pour afficher des informations de support de diagnostic sur la base d'un résultat de calcul obtenu par le moyen de calcul de valeurs caractéristiques (8),

le moyen de calcul de valeurs caractéristiques (8) comprenant

un moyen d'extraction de vaisseau sanguin (101) pour extraire une image de vaisseau sanguin des données d'images, qui sont stockées dans le moyen de stockage d'images (7) comme des données d'images R, G et B, et

un moyen de calcul de valeur caractéristique de vaisseau sanguin (102) pour calculer un rapport de superficie de vaisseau sanguin sur la base de la sortie du moyen d'extraction de vaisseau sanguin (101),

le moyen d'extraction de vaisseau sanguin (101) comprenant

un moyen d'extraction de vaisseau sanguin candidat (121),

un moyen de détermination d'information de gradient (131) qui détermine des données de gradient de densité sur la base des données d'images R, G et B,

un moyen de détermination de bords de formes (132) qui détermine des données de gradient de somme sur la base des données de gradient de densité et binarise les données de gradient de somme afin d'obtenir des données de bords d'un vaisseau sanguin, et

un moyen de séparation (141) qui détermine des données d'images de vaisseau sanguin sur la base des données de vaisseau sanguin candidat et des données de bords,

le moyen d'extraction de vaisseau sanguin candidat (121) comprenant

une section de détection d'informations de bords (122) qui exécute un traitement par différentiation de deuxième ordre sur la base des données d'images -B, et

une section de calcul de tonalités de couleurs (123) qui calcule des données de tonalités de couleurs sur la base des données d'images R, G et B, dans lequel

le moyen d'extraction de vaisseau sanguin candidat (121) extrait des données de vaisseau sanguin candidat sur la base d'une sortie de la section de détection d'informations de bords (122) et de la section de calcul de tonalités de couleurs (123).

2.  Appareil de support de diagnostic selon la revendication 1, dans lequel

la section de détection d'informations de bords (122) détecte des informations de bords par rapport à au moins un signal de couleur parmi des données d'images constituées par une pluralité de signaux de couleurs et stockées dans le moyen de stockage d'images (7), et

la section de calcul de tonalités de couleurs (123) calcule une valeur associée avec une tonalité de couleur par rapport à au moins un signal de couleur parmi des données d'images constituées par une pluralité de signaux de couleurs et stockées dans le moyen de stockage d'images (7).

3.  Appareil de support de diagnostic selon la revendication 1 ou 2, dans lequel

le moyen de détermination d'information de gradient (131) détermine des informations de gradient de densité de données d'images constituées par une pluralité de signaux de couleurs et stockées dans le moyen de stockage d'images (7), et

le moyen de détermination de bords de formes (132) détermine les données de bords de formes sur la base d'un traitement par seuil pour une somme linéaire d'informations de gradient de densité d'une pluralité de signaux de couleurs délivrés en sortie du moyen de détermination d'information de gradient (131).

4.  Appareil de support de diagnostic selon l'une des revendications 1 à 3, dans lequel le moyen de séparation (141) détermine des données d'images de vaisseau sanguin au moyen d'un traitement par extension.

5.  Appareil de support de diagnostic selon l'une des revendications 1 à 4, dans lequel la section de calcul de tonalités de couleurs (123) définit une valeur calculée par R/(R + G + B) comme une valeur associée avec une tonalité de couleur lorsque la pluralité de signaux de couleurs comprennent R, G et B.

6.  Procédé de support de diagnostic pour supporter un diagnostic par un examinateur, comprenant les étapes de :

    stockage de données d'images d'un dispositif endoscopique (1) dans un moyen de stockage d'images (7),

    calcul d'au moins une valeur caractéristique pour quantifier une découverte associée à un diagnostic à partir de données d'images stockées dans le moyen de stockage d'images (7) par un moyen de calcul de valeurs caractéristiques (8), et

    affichage d'informations de support de diagnostic sur la base d'un résultat de calcul obtenu par le moyen de calcul de valeurs caractéristiques (8) sur un moyen d'affichage d'informations de support de diagnostic (9), dans lequel

    le moyen de calcul de valeurs caractéristiques (8) exécute les étapes de

    extraction d'une image de vaisseau sanguin des données d'images, qui sont stockées dans le moyen de stockage d'images (7) comme des données d'images R, G et B par le moyen d'extraction de vaisseau sanguin (101), et

    calcul d'un rapport de superficie de vaisseau sanguin sur la base de la sortie du moyen d'extraction de vaisseau sanguin (101) dans un moyen de calcul de valeur caractéristique de vaisseau sanguin (102), dans lequel

    le moyen d'extraction de vaisseau sanguin (101) exécute les étapes de

exécution d'un traitement par différentiation de deuxième ordre sur la base des données d'images G par une section de détection d'informations de bords (122) d'un moyen d'extraction de vaisseau sanguin candidat (121), calcul de données de tonalités de couleurs sur la base des données d'images R, G et B par une section de calcul de tonalités de couleurs (123) du moyen d'extraction de vaisseau sanguin candidat (121)

extraction de données de vaisseau sanguin candidat sur la base d'une sortie de la section de détection d'informations de bords (122) et de la section de calcul de tonalités de couleurs (123),

détermination de données de gradient de densité sur la base des données d'images R, G et B par un moyen de détermination d'information de gradient (131),

détermination de données de gradient de somme sur la base des données de gradient de densité et binarisation des données de gradient de somme afin d'obtenir des données de bords d'un vaisseau sanguin par un moyen de détermination de bords de formes (132), et

détermination de données d'images de vaisseau sanguin sur la base des données de vaisseau sanguin candidat et des données de bords par un moyen de séparation (141).

7.  Procédé de support de diagnostic selon la revendication 6, dans lequel

la section de détection d'informations de bords (122) détecte des informations de bords par rapport à au moins un signal de couleur parmi des données d'images constituées par une pluralité de signaux de couleurs et stockées dans le moyen de stockage d'images (7), et

la section de calcul de tonalités de couleurs (123) calcule une valeur associée avec une tonalité de couleur par rapport à au moins un signal de couleur parmi des données d'images constituées par une pluralité de signaux de couleurs et stockées dans le moyen de stockage d'images (7).

8.  Procédé de support de diagnostic selon la revendication 6 ou 7, dans lequel

le moyen de détermination d'information de gradient (131) détermine des informations de gradient de densité de données d'images constituées par une pluralité de signaux de couleurs et stockées dans le moyen de stockage d'images (7), et

le moyen de détermination de bords de formes (132) détermine les données de bords de formes sur la base d'un traitement par seuil pour une somme linéaire d'informations de gradient de densité d'une pluralité de signaux de couleurs délivrés en sortie du moyen de détermination d'information de gradient (131).

9.  Procédé de support de diagnostic selon l'une des revendications 6 à 8, dans lequel le moyen de séparation (141) détermine des données d'images de vaisseau sanguin au moyen d'un traitement par extension.

10. Procédé de support de diagnostic selon l'une des revendications 6 à 9, dans lequel la section de calcul de tonalités de couleurs (123) définit une valeur calculée par R/(R + G + B) comme une valeur associée avec une tonalité de couleur lorsque la pluralité de signaux de couleurs comprennent R, G et B.

EP 1 704 816 B1

FIG. 1

FIG. 2

FIG. 3

#10

Input/
output
control
means

#32

Diagnostic support content
distribution executing section

#33

Storage means
management section

Main program

31

Distribution
destination
management file
storage means

#8

Diagnostic
support content
storage means

#6

F I G. 4

#32

#41 — Input/output control means I/F

#42 — Diagnostic support content
designating section

#43 — Diagnostic support content updating/
addition detecting section

#44 — Diagnostic support content
management means

#45 — Diagnostic support execution
terminal authenticating section

#46 — Diagnostic support content
selecting section

#47 — Diagnostic support content
list creating section

F I G. 5

Start

Detect updating/addition of
diagnostic support content — S1

Acquire distributed diagnostic
support content — S2

Specify distribution destination
terminal — S3

Is distribution
destination terminal
authenticated ? — S4

NO

YES

Distribute diagnostic support
content — S5

Is content
distributed to all distribution
destination terminals
? — S6

NO

YES

End

F I G. 6

#11 Input/output control means

#52 Terminal authentication information transmitting section

#53 Storage means management section

#16 Terminal authentication information storage means

#54 Diagnostic support content management section

#55 Diagnostic support content communication section

#13 Diagnostic support content storage means

#57 Diagnostic support information creating section

#17 Display control means

#56 Diagnostic information input/output I/F

#58 Input I/F

#51 Main program

#15 Diagnostic information input/output control means

#23 Keyboard or the like

F I G. 7

```
                    ┌──────────────┐
                    │    Start     │
                    └──────┬───────┘
                           │              ╭S11
          ┌────────────────▼──────────────┐
          │ Acquire and transmit terminal │
          │    specifying information     │
          └────────────────┬──────────────┘
                           │              ╭S12
          ┌────────────────▼──────────────┐
          │ Receive diagnostic support content │
          └────────────────┬──────────────┘
                           │              ╭S13
          ┌────────────────▼──────────────┐
          │ Update diagnostic support content │
          │     management information    │
          └────────────────┬──────────────┘
                           │              ╭S14
          ┌────────────────▼──────────────┐
          │ Store diagnostic support content │
          └────────────────┬──────────────┘
                           │
                    ┌──────▼───────┐
                    │     End      │
                    └──────────────┘
```

# F I G. 8

**FIG. 9**

Diagnostic support for Helicobacter pylori infection/gastritis based on IHb value

A2

A1

A3

A4

IHb value : 67.5
Occurrence probability information :
　Normal : 0.05 (5%)
　Gastritis : 0.15 (15%)
There is a suspicion of gastritis due to Helicobacter pylori infection

A5

Diagnosis of gastritis based on IHb value
Normal groups : 48.5±3.5
Gastritis group : 74.5±4.0
Boundary value : 60.0
Sensitivity : 90%
Specificity : 85%

Occurrence probability

A6

Normal group

Gastritis group

0.2

0.1

0

50

100

IHb value

Boundary value : 60.0

A11

| A12 | Diagnostic support for protruded lesion of stomach based on identification/classification technique |

| A13 | Characteristic value | IHb value 63.5 | G variation coefficient 0.66 | Blood vessel area ratio 0.55 |
|---|---|---|---|---|
| Normal group average value | 54.5 | 0.43 | 0.30 |
| Adenocarcinoma group average value | 44.5 | 0.31 | 0.20 |
| Early cancer group average value | 64.3 | 0.77 | 0.46 |

Identification technique :
    linear discrimination
    function
Class count : 3

Normal, adenocarcinoma, early cancer

Identification/
classification result :
early cancer

Biopsy is required

A14

F I G. 10

EP 1 704 816 B1

A21  A22  A25

Refer to typical and similar case images

Switch between typical and similar cases

Similar case

IIa type early cancer No.12  ~A28

A23

Characteristic value    Target  Target
IHb value              : 67.5   68.9
G variation coefficient : 0.64   0.71
Blood vessel area ratio : 0.44   0.67

~A29

Before    Next    Details

A26      A27      A30

F I G. 11

IIa type early cance
Typical case  No.12
Sex : M
Age : 66
Region : body of stomach, curvature ventriculi
Peripheral mucous membrane : atrophy+
Size : 13mm×16mm
Boundary region : asymmetry

Detailed display window

Close

F I G. 12

**A42** **A43**

Display of diagnosis point :
protruded lesion of stomach

Ⅱa type early cancer (well differentiated type)

**A41**

**A45**

Image Findings : note the following
①Color tone : erythematous, 60 IHb value or more
②Irregular protuberance
③Border indistinct
④Irregular pit pattern
⑤Abnormal blood vessel structure
⑥Major axis length of 20 nun or more : suspicion of
   sm invasion

**A44**

Differentiation target : adenocarcinoma
Discolored 60 IHb value or less
Smooth protuberance
Border distinct
Dense, fine pit pattern

Other diseases

**A46** **A47**

FIG. 13

EP 1 704 816 B1

A52 — Test item/treatment content table : protruded lesion of stomach

A53 — Ⅱa type early cancer (well differentiated type)

A56

A51 / A54:

Test item : Execute following
①Observation of surface condition and boundary by dye spreading
②Biopsy, including boundary region
③Test of Helicobacter pylori infection

◎Multiple diseases caution : other lesions ?
◎This patient has developed recurrence

A55:

Treatment content information :
◎EMR can be used for well differentiated type protruded lesion with major axis length of 25mm or less

FIG.14

A60

A61

Diagnostic support content
specifying information
·Content ID
·Content name
·Modality
·Examination region
·Target disease count N
·Working feature amount
 calculation technique count P
·Working identification/
 classification technique count K

A62

Disease information : disease 1
·Statistical information
·Diagnostic information
·Calculated feature amount data list
·Examination/treatment information
·Other text information

A63

Reference image information
·Typical/similar image
·Diagnostic information

A64

Characteristic value
calculation technique library
 1 to P

Disease information : disease N
·Statistical information
·Diagnostic information
·Calculated feature amount data list
·Examination/treatment information
·Other text information

Reference image information
·Typical/similar image
·Diagnostic information

Identification/classification
technique library
 1 to K

A65

Graph creation data

A66

FIG.15

```
        ┌─────────────┐
        │    Start    │
        └─────────────┘
               │
               ▼                      S21
   ┌──────────────────────────────┐
   │ Set diagnostic support content│
   └──────────────────────────────┘
               │
               ▼                      S22
   ┌──────────────────────────────┐
   │ Acquire patient/examination  │
   │ information                  │
   └──────────────────────────────┘
               │
               ▼                      S23
   ┌──────────────────────────────┐
   │ Acquire image information     │
   └──────────────────────────────┘
               │
               ▼                      S24
   ┌──────────────────────────────┐
   │ Create diagnostic support    │
   │ information                  │
   └──────────────────────────────┘
               │
               ▼                      S25
   ┌──────────────────────────────┐
   │ Display diagnostic support   │
   │ information                  │
   └──────────────────────────────┘
               │
               ▼         S26
          ◇ End ? ◇──── NO
               │
              YES
               ▼
        ┌─────────────┐
        │     End     │
        └─────────────┘
```

F I G. 16

Diagnostic support content update/add menu

Message : there are three types of updates/additions

| ID | Type | Modality | Test region | Name | Status | Date |
|---|---|---|---|---|---|---|
| 11 | ② | Endoscope | Stomach | Depressed lesion identification/classification | Update Ver.2.0 | 2001/3/14 |
| 14 | ② | Endoscope | Large intestine | Pit pattern diagnosis | Addition Ver.1.0 | 2001/3/15 |
| 22 | ④ | Ultrasound | Stomach | Lymphoma test support information | Update Ver.3.5 | 2001/3/15 |

Select all    A72    Cancel    OK

A75    A74    A73    A76

A71

FIG. 17

F I G. 18

#2 Diagnostic support content server

#103 Diagnostic support content creating tool server

#4 Network (WAN/LAN)

#102 Blood vessel characteristic value calculation means

#101

#3 Diagnostic support execution terminal

Diagnostic information

Medical system #5

Medical system #5

FIG.19

Diagnostic support content creating terminal #102

Diagnostic support content

Network #4

Diagnostic support content storage means #13

Diagnostic support content creating tool storage means #111

Main program storage means #14

Input/output control means #11

Control means #12

Display control means #17

Diagnostic support information

Keyboard or the like #23

Diagnostic information input/output control means #15

Terminal authentication information storage means #16

Display means #18

Electronic clinical chart #21

Image file system #22

#5

In-hospital network #20

Patient/examination image information

Endoscopic system #19

57

FIG. 20

Start

Create data set ⟋S41

Is image
analysis characteristic value
used ? ⟋S42

NO

YES ⟋S43

Calculate characteristic value

Create diagnostic support content ⟋S44

End

FIG. 21

FIG. 22

EP 1 704 816 B1

Examination condition setting

Modality    Endoscope ▽   ~A120

Examination
region    Stomach ▽

Cancel    OK

F I G. 23

A125

Text information input

Ⅱa type early stomach cancer ▽

Important
findings
   Erythematous and surface roughness are recognized. Differentiation from adenocarcinoma is important. Check pit pattern with magnifying endoscope.

Treatment
instruction
   Use EMR for size of 20mm or less. Adaptive dilation up to 30mm is reported. Check it by biopsy.

Input other information    Cancel    OK

F I G. 24

Reference image determination

A130

A131

IIa type early stomach cancer ▽

A132 — Confirm/check information    Cancel    OK

# FIG. 25

Call diagnostic support content selection ⊠

A140

Esophageal tumor lesion diagnosis

A141

Depressed stomach lesion identification/classification

A142

Large intestine information remission diagnosis

Large intestine pit pattern diagnosis identification/classification

Cancel    Confirm

A143

# FIG. 26

Diagnostic support main menu

A201 — Execute diagnostic support

A200

A202 — Update/add diagnostic support content

End — A203

FIG. 27

Test condition setting

A210

Modality    Endoscope    ▽ — A211

Test region    Upper gastrointestinal tract (stomach/gullet)    ▽ — A212

Cancel    OK — A213

FIG. 28

Diagnostic support content setting

Select diagnostic support content ~A220

Modality : endoscope
Examination region : Upper gastrointestinal tract (stomach/ gullet)

A222
| √ | Helicobacter pylori infection/gastritis diagnosis | △ |
| √ | Depressed lesion identification/classification | |
| √ | Protruded lesion identification/classification | |
| | Similar/typical case reference | ▽ |

~A221

Cancel | Start test ~A223

F I G. 29

Terminal authentication information setting

Facility name | Prefectural university hospital | ~A230
| | | A231

Terminal name | First internal medicine endoscope | A232

ID | 111806 | A233

Password | * * * * * * * * * | A234

Cancel | OK | A235

F I G. 30

Step S21

Execution of diagnostic support       Updating of diagnostic support content

**S51**
Set diagnostic support content condition

**S52**
Set diagnostic support content

**S53**
Load diagnostic support content

To step S22

**S54**
Select diagnostic support content server

**S55**
Transmit terminal specifying information

**S56**
Has communication with server been established ?

NO → **S62** Error

YES

**S57**
Refer to diagnostic support content management information

**S58**
Select updated/added diagnostic support content

**S59**
Receive diagnostic support content

**S60**
Update diagnostic support content management information

**S61**
Store diagnostic support content

Return to step S21

# F I G. 31

FIG. 32

Patient name : ◯◯
ID : 12321
Date of birth : S32/1/22
Sex : M

A253

A252

A251

End examination

A254    A250

Diagnostic support content server selection

Select diagnostic support content server — A260

Modality | All modalities ▽ — A261

Examination region | Upper gastrointestinal tract (stomach/gullet) ▽ — A262

A264

| ✓ | Server 1 | △ |
| ✓ | Server 2 | |
| ✓ | Server 3 | |
| | Server 4 | ▽ |

— A263

Cancel | OK — A265

# F I G. 33

Diagnostic support content creation main menu

A271 — Create new content — A270

A272 — Use existing content

End — A273

# FIG. 34

Diagnostic support content update/add menu

Message : there are three types of updates/additions

| ID | Type | Modality | Examination region | Name | Status | Date | Creator |
|---|---|---|---|---|---|---|---|
| 11 | ② | Endoscope | Stomach | Depressed lesion identification/classification | Update Ver.2.0 | 2001/3/14 | First hospital |
| 14 | ② | Endoscope | Large intestine | Pit pattern diagnosis | Addition Ver.1.0 | 2001/3/15 | Medical college internal medicine |
| 22 | ④ | Ultrasound | Stomach | Lymphoma test support information | Update Ver.3.5 | 2001/3/15 | ABC medical center |

Select all

Cancel

OK

A281

A280

F I G. 35

EP 1 704 816 B1

FIG. 36

Input/output control means ~#11

#15
Diagnostic information input/output control means

#56
Diagnostic information input/output I/F

#55
Diagnostic support content communication section

#54
Diagnostic support content management section

#52
Terminal authentication information transmitting section

#23
Keyboard or the like

#58
Input I/F

Diagnostic support information creating section

Storage means management section ~#53

Main program

#57

#51

Display control means

#17

Diagnostic support content storage means

#13

Terminal authentication information storage means ~#16

Update/add inquiry information storage means ~#60

EP 1 704 816 B1

70

Start

**S71** Load update/add inquiry information

**S72** Is inquiry started ? — NO

YES

**S73** Select diagnostic support content server

**S74** Transmit terminal specifying information

**S75** Has communication with server been established ? — NO

YES

**S76** Refer to diagnostic support content management information

**S77** Select updated/added diagnostic support content

**S78** Receive diagnostic support content

**S79** Update diagnostic support content management information

**S80** Store diagnostic support content

**S81** Display error information

**S82** Is inquiry ended ? — NO

YES

**S83** Wait for inquiry

**S84** End ? — NO

YES

End

F I G. 37

EP 1 704 816 B1

Inquiry timing : startup, end of examination, two-hour intervals, 15:00  ~A291

| Diagnostic support content server ID | Diagnostic support content ID |
|---|---|
| 001A | 12345 |
| 001A | 23456 |
| 002B | ALL |
| 003C | 22422 |
| 003C | 33333 |

~A290

~A292

F I G. 38

#127

Diagnostic support content creating section

Item selecting section ~153

155

#123

Storage means management section

156

Classified data set creating section

Statistical processing section

154

Graph creating section

Graph information creating section

152

Image information holding means

151

F I G. 39

72

Image information
·Image data
·Region-of-interest data
·Accompanying data
  ·Image ID
  ·Patient ID
  ·Patient name
  ·Examination date
  ·Classification key item
    ○Category classification
    ○Graph display attribute
    ○Diagnosis name
    ○Examination region
    ○Patient sex
    ○Arbitrary set character string items 1 to Q
·Numerical value item
    ○Characteristic value information 1 to P
      (characteristic values, calculation parameters)
    ○Patient age
    ○Arbitrary set numerical value items 1 to R

F I G. 40A

Auxiliary information
·Graph type information
·Statistical amount type information
·Statistical examination type information

F I G. 40C

Item management information
  ·Item name information of item classified to
    classification key item
  ·Item name information of item classified to
    numerical value item
  ·Item value information of category classification
    (Category A, category B,···)
  ·Item value information of diagnosis name
    (Normal, cancer,···)
  ·Item value information of examination region
    (Stomach, gullet. large intestine,···)
  ·Item value information of patient sex
    (Male, female)
  ·Item name information of arbitrary set character
    string items 1 to Q
    (Color tone, mucous membrane pattern,···)
  ·Item value information of arbitrary set character
    string items 1 to Q
    Color tone (rubefaction, discoloration,···)
    Mucous membrane pattern (present. absent)
    ···
  ·Item name information of arbitrary set numerical
    value items 1 to R
    (Red blood cell count, test date intervals,···)

F I G. 40B

EP 1 704 816 B1

## Graph creation

**Graph type** 157
| Histogram |
| One-dimensional scatter diagram |
| Two-dimensional scatter diagram |
| Average value bar graph |
| Case count bar graph |

**Classificatio item** 158
| Category classification |
| Diagnosis name |
| Test region |
| Patient sex |
| Findings |

**Information to be superimposed** 159
| Average value |
| Standard deviation |
| Standard error |
| Intermediate value |
| Mode value |
| t test |
| $x^2$ test |

**Data value 1** 160
| Patient age |
| Characteristic value 1 |
| Characteristic value 2 |
| Characteristic value 3 |
| Characteristic value 4 |

**Data value 2** 161
| Patient age |
| Characteristic value 1 |
| Characteristic value 2 |
| Characteristic value 3 |
| Characteristic value 4 |

**Classified data set** 162
| Adenocarcinoma/male |
| Adenocarcinoma/female |
| Stomach cancer/male |
| Stomach cancer/female |
| Polyp/male |
| Polyp/female |
| Ulcer/male |
| Ulcer/female |
| Lymphoma/male |
| Lymphoma/female |
| Erosion/male |
| Erosion/female |

Execute 163   Cancel 164

## FIG. 41

EP 1 704 816 B1

F I G. 42

B

TB-1

Execute classification
information setting A

TB-2

Is
image information
acquired ?

All pieces of image
information have been
acquired

To C

Acquisition of image
information has
succeeded

TB-3

NO

Coincidence
of classified date
set ?

YES

TB-4

Register image information
in corresponding classified
data set

# F I G. 43

F I G. 44

F I G. 45

F I G. 46

F I G. 47

F I G. 48

F I G. 49

F I G. 50

| Statistical information | | |
|---|---|---|
| | Adenocarcinoma | Stomach cancer |
| Total | 32 | 22 |
| Patient age | 50±15 | 70±5 |
| Characteristic value 1 | 0.4±0.05 | 0.7±0.13 |
| Characteristic value 3 | 1.5±0.08 | 1.7±0.01 |

F I G. 51

Graph creation

| Graph type | Histogram | Data value 1 | Patient age | Classified data set | Adenocarcinoma/male |
|---|---|---|---|---|---|

Graph type

Histogram
One-dimensional scatter diagram
Two-dimensional scatter diagram
Average value bar graph
Case count bar graph

Classification item

Category classification
Diagnosis name
Examination region
Patient sex
Findings

Information to be superimposed

Average value
Standard deviation
Standard error
Intermediate value
Mode value
t test
x^2 test

Data value 1

Patient age
Characteristic value 1
Characteristic value 2
Characteristic value 3
Characteristic value 4

Data value 2

Patient age
Characteristic value 1
Characteristic value 2
Characteristic value 3
Characteristic value 4

Classified data set

Adenocarcinoma/male
Adenocarcinoma/female
Stomach cancer/male
Stomach cancer/female
Polyp/male
Polyp/female
Ulcer/male
Ulcer/female
Lymphoma/male
Lymphoma/female
Erosion/male
Erosion/female

Execute    Cancel

165

⊠ Creates a graph according to classfication combination

F I G. 52

EP 1 704 816 B1

FIG. 53

F I G. 54A

F I G. 54B

F I G. 55A

F I G. 55B

F I G. 55C

#123

Storage means
management
section

Image
information
holding means

151

171

Information list
creating section

154

Graph creating
section

Diagnostic support content creating section

#127

172

Display
information
management
section

#128 Input I/F

# F I G. 56

Start

TH-1

Detect selection of graph element,
image, or list item

TH-2

Cancel selected state of graph,
image, and list

TH-3

Acquire image information
corresponding to selected item

TH-4

Acquire graph element, image, and
list item corresponding to image
information

TH-5

Set graph element, image, or list
item in selected state

End

# F I G. 57

173

Image information list

| Image ID | Patient ID | Patient name | Examination date |
|---|---|---|---|
| 1 | 111 | Tanaka | 2000.05.27 |
| 2 | 222 | Yamasaki | 2000.01.01 |
| 3 | 333 | Imaizumi | 2000.04.02 |
| 4 | 444 | Nishimura | 2000.06.05 |
| | | | |
| | | | |
| | | | |

Tanaka
111
M
00.05.27

Yamasaki
222

174

175

## F I G. 58

F I G. 59

F I G. 60

Start

Input item to be changed and item value    TJ-1

Detect selection of graph element, image, or list item    TJ-2

Acquire image information of selected item    TJ-3

Update data of image information of selected item    TJ-4

Update display information    TJ-5

End

F I G. 61

FIG. 62

FIG. 63

Updating of information of selected element — 184

Item to be changed

| Category classification | △ |
| Diagnosis name | |
| Examination region | |
| Patient sex | |
| Graph creation attribute | ▽ |

185

| Used for graph creation |
| Not used for graph creation |

186

Tanaka
111
M
00.05.27 — 187

Update — 188

FIG. 64

| Updating of information of selected element | | 184 |
|---|---|---|
| Patient name | Tanaka | 220 |
| Examination date | 2001.02.10 | 221 |
| Examination region | Stomach ▽ | 222 |
| Occupying region | Cardia ▽ | 223 |
| Diagnosis name | ▽ | 224 |
| 231 [Fine classification 1] | ▽ | 225 |
| 232 [Fine classification 2] | ▽ | 226 |
| 233 [Fine classification 3] | ▽ | 227 |
| 234 [Fine classification 4] | ▽ | 228 |
| | Update | 229 |

F I G. 65

| Updating of information of selected element | | 184 |
|---|---|---|
| Patient name | Tanaka | 220 |
| Examination date | 2001.02.10 | 221 |
| Examination region | Stomach ▽ | 222 |
| Occupying region | Cardia ▽ | 223 |
| Diagnosis name | ▽ | 224 |
| 231 [Fine classification 1] | Ulcer ▽ | 225 |
| 232 [Fine classification 2] | Gastritis ▽ | 226 |
| 233 [Fine classification 3] | Polyp ▽ | 227 |
| 234 [Fine classification 4] | Erosion ▽ | 228 |
| | Update | 229 |

230

F I G. 66

| Examination region | Occupying lesion | Diagnosis name | Fine classi-fication 1 | Fine classi-fication 2 | Fine classi-fication 3 | Fine classi-fication 4 |
|---|---|---|---|---|---|---|

Stomach
— Cardia
— Curvature ventriculi minor

Cancer
Borrman classification
— Type 1
— Type 2
— Type 3
— Type 4

Early stomach cancer macroscopic classification
— Type I
— Type IIa
— Type IIb
— Type IIc
— Type III

Progression degree
— m
— sm
— mp

Colon

Ulcer
— Temporal classification
— Forrest classification

Gullet

Gastritis
— Sydney classification
— Schindler classification
— Macroscopic classification
— EUS classification

Polyp
— Yamada/ fukutomi classification
— WHO classification

Erosion

## F I G. 67

---

| Updating of information of selected element | | ～184 |
|---|---|---|
| Patient name | Tanaka | ～220 |
| Examination date | 2001.02.10 | ～221 |
| Examination region | Stomach ▽ | ～222 |
| Occupying region | Cardia ▽ | ～223 |
| Diagnosis name | Cancer ▽ | ～224 |
| [Borrman classification] | ▽ | ～225 |
| [Macroscopic classification] | ▽ | ～226 |
| [Progression degree] | ▽ | ～227 |
| [Fine classification 4] | ▽ | ～228 |
| | Update | ～229 |

231 — [Borrman classification]
232 — [Macroscopic classification]
233 — [Progression degree]
234 — [Fine classification 4]

## F I G. 68

#123

Storage means
management
section

Image
information
holding means

151

171

Information list
creating section

154

Graph creating
section

172

Display
information
management
section

182

Information updating
section

201

Region-of-interest setting section

Information setting section

181

Information support content
creating section

#127

Input I/F    #128

F I G. 69

T1-1

Press right
button of
mouse

T1-2

Determine
temporary
position as
true position

Area setting
step

Moving step

Press left button
of mouse

Release left
button of mouse

T1-3

Position temporarily
determining step

Release left
button of mouse

Move mouse while
pressing left
button. of mouse

T1-4 — Size changing
step

FIG. 70

173

Image information list

| | Image ID | Patient ID | Patient name | Examination date |
|---|---|---|---|---|
| Tanaka 111 M 00.05.27 | 1 | 111 | Tanaka | 2000.05.27 |
| | 2 | 222 | Yamasaki | 2000.01.01 |
| | 3 | 333 | Imaizumi | 2000.04.02 |
| | 4 | 444 | Nishimura | 2000.06.05 |
| Yamasaki 222 | | | | |

174

175

FIG. 71

F I G. 72A

F I G. 72B

F I G. 72C

#123          213          #127

| Storage means management section | → | Marker rendering section | → | Diagnostic support content creating section |

## F I G. 73

Start

Acquire accompanying data   — TF-1

Render marker   — TF-2

End

## F I G. 74

Tanaka
111
M
00.05.27

210

## F I G. 75A

Tanaka
111
M
00.05.27
○
211

## F I G. 75B

Tanaka
111
M
☆
212

## F I G. 75C

#123     220     221     #127

| Storage means management section | → | Character information erasing section | → | Character information rendering section | → | Diagnostic support content creating section |

F I G. 76

Start

Erase patient information    ~TG-1

Acquire accompanying data    ~TG-2

Render accompanying data    ~TG-3

End

F I G. 77

Characteristic value 1
0.35
Characteristic value 2
1.55

F I G. 78

```
#123 ──┐  Storage means                    Image processing   ──── 250
        │  management section               section
        └─────────────┐          ┌───────►
#124 ──┐  Diagnostic support content        Diagnostic support content  ──── #127
        │  management section               creating section
```

**F I G. 79**

```
                    ┌─────────────────────────────┐
 Image data ────────┼──►  Image processing table  ─┼──►  Converted
 (R, G, B)          │     access section           │     value n
                    │                        ──── 252
                    │     (R, G, B, m)      │  n
                    │                       ▲
 Conversion table ──┼──►  Conversion table  │     ──── 250
                    │     changing section  │
                    └──────────┬──────┬─────┘
                          253   │      │
                                ▼      ▼
                      ┌─────────────────────┐
                      │ Image processing table │ ──── 251
                      └─────────────────────┘
```

**F I G. 80**

```
┌──────────────────────────────────────────────┐
│                    Image processing table 251  │
│  ┌──────────────────────────────────────────┐ │
│  │ (0,0,0)(0,0,1)···                          │ │
│  │                  1                         │ │
│  │      ···(255,255,254)(255,255,255)         │ │
│  └──────────────────────────────────────────┘ │
│                      ⋮                          │
│  ┌──────────────────────────────────────────┐ │
│  │ (0,0,0)(0,0,1)···                          │ │
│  │                  M                         │ │
│  │      ···(255,255,254)(255,255,255)         │ │
│  └──────────────────────────────────────────┘ │
└──────────────────────────────────────────────┘
```

**F I G. 81**

```
                    ┌─────────────┐
                    │    Start    │
                    └──────┬──────┘
                           │
        ┌──────────────────▼─────── TK-1
        │               ╱Has╲
        │          ╱ pixel value been ╲  All pixel values have
        │          ╲   acquired ?    ╱   been acquired
        │           ╲              ╱     ┌──────────┐
        │            ╲          ╱        │   End    │
        │          Acquisition of pixel  └──────────┘
        │          value has succeeded
        │                  │  ── TK-2
        │         ┌────────▼──────────────┐
        │         │ Access image processing able │
        │         └────────┬──────────────┘
        │                  │  ── TK-3
        │         ┌────────▼──────────────┐
        │         │ Hold image processing value │
        └─────────└───────────────────────┘
```

# F I G. 82

```
   #123                    260                    #15
┌──────────────┐    ┌──────────────┐    ┌──────────────────────┐
│ Storage means│───▶│Data embedding│───▶│Diagnostic information│
│ management   │    │   section    │    │    input/output      │
│ section      │◀───│              │◀───│    control means     │
└──────────────┘    └──────────────┘    └──────────────────────┘
```

# F I G. 83

F I G. 84A

F I G. 84B

FIG. 85

| (FF,A1,00) | (FA,A0,01) | |
|---|---|---|
| (FA,A1,03) | (FB,BF,03) | |
| | | |

↓

| (FE,A0,00) | (FA,A0,00) | |
|---|---|---|
| (FA,A0,03) | (FA,BE,02) | |
| | | |

Characteristic value of 60
→binary number 00111100

↓

| (FE,A0,01) | (FB,A1,01) | |
|---|---|---|
| (FA,A0,03) | (FA,BF,02) | |
| | | |

FIG. 86

| (FE,A0,01) | (FB,A1,01) | |
|---|---|---|
| (FA,A0,03) | (FA,BE,02) | |
| | | |

↓

Binary number 00111100
→Characteristic value of 60

Endoscopic device

| | |
|---|---|
| 2 | Light source |
| 3 | Endoscope |
| 4 | Video processor |
| 5 | Observation monitor |

1

Main program

| | |
|---|---|
| Image storage means | 7 |
| Characteristic value calculation means | 8 |
| Diagnostic support information display means | 9 |

#121

## F I G. 87

Characteristic value calculation means

| | |
|---|---|
| Blood vessel extraction means | 101 |
| Blood vessel characteristic value calculation means | 102 |

8

## F I G. 88

Blood vessel extraction means

Preprocessing section — 111

101

121 — Blood vessel candidate extracting section

122 — Edge information detecting section

123 — Color tone calculating section

Density gradient calculating section — 131

Shape edge determining section — 132

Separating section — 141

## F I G. 89

```
                    ┌──────────────┐
                    │    Start     │
                    └──────┬───────┘
                           │          S101
              ┌────────────▼─────────────┐
             /      Input image data      /
            └─────────────┬──────────────┘
                          │            S102
              ┌───────────▼──────────────┐
              │    Start calculating     │
              │    characteristic value  │
              └───────────┬──────────────┘
                          │            S103
              ┌───────────▼──────────────┐
              │   Perform preprocessing  │
              └───────────┬──────────────┘
          ┌───────────────┼────────────────────┐
   S104   │        S105   │             S106    │
┌─────────▼───┐  ┌────────▼──────────┐  ┌───────▼──────────┐
│ Calculate   │  │ Detect edge       │  │ Calculate density│
│ color tone  │  │ information       │  │ gradient         │
└─────┬───────┘  └────────┬──────────┘  │ information      │
      │                   │             └───────┬──────────┘
      └───────────────────┤          S108       │      S107
                ┌─────────▼─────────┐  ┌─────────▼────────┐
                │ Extract blood     │  │ Determine shape  │
                │ vessel candidate  │  │ edge             │
                └─────────┬─────────┘  └─────────┬────────┘
                          │◄─────────────────────┘
                          │            S109
              ┌───────────▼──────────────┐
              │   Separate shape edge    │
              └───────────┬──────────────┘
                          │            S110
              ┌───────────▼──────────────┐
              │  Calculate blood vessel  │
              │  characteristic value    │
              └───────────┬──────────────┘
                          │            S111
              ┌───────────▼──────────────┐
              │ End characteristic value │
              │ calculation              │
              └───────────┬──────────────┘
                          │            S112
              ┌───────────▼──────────────┐
              │ Display diagnostic support│
              │ information               │
              └───────────┬──────────────┘
                    ┌──────▼───────┐
                    │     End      │
                    └──────────────┘
```

# F I G. 90

```
┌─────────────────────────────────────────────────────┐
│              Preprocessing section                  │
│  ┌───────────────────────────────────────────────┐  │
│  │ Inverse gamma correction processing section   │──┼─112
│  └───────────────────────────────────────────────┘  │
│                        │                            │
│                        ▼                            │
│  ┌───────────────────────────────────────────────┐  │
│  │            Noise suppressing section          │──┼─113
│  └───────────────────────────────────────────────┘  │
│                        │                            │
│                        ▼                            │
│  ┌───────────────────────────────────────────────┐  │
│  │      Color misregistration correcting section │──┼─114
│  └───────────────────────────────────────────────┘  │
└─────────────────────────────────────────────────────┘
```

111

F I G. 91

```
┌──────────────────────────────────────────┐
│ Store pixel P∇ that satisfies condition   │
│ "∇2 G (x, y) ≧ threshold T∇"              │──S220
└──────────────────────────────────────────┘
                   │
                   ▼
┌──────────────────────────────────────────┐
│ Calculate minimum value Cmin of           │
│ color tone data C at pixel P∇             │──S221
└──────────────────────────────────────────┘
                   │
                   ▼
┌──────────────────────────────────────────┐
│ Binarize color tone data C with           │
│ minimum value Cmin                        │──S222
│ (blood vessel candidate data BiC)         │
└──────────────────────────────────────────┘
```

F I G. 92

| 0 | 1 | 0 |
|---|---|---|
| 1 | -4 | 1 |
| 0 | 1 | 0 |

F I G. 93

Calculate linear sum Grad C of Grad R, G, B ~S230

Binarize Grad C with predetermined threshold TGrad (shape edge data BiGrad) ~S231

F I G. 94

Calculate logical product L of BiGrad and BiC ~S240

Perform expansion processing of logical product L based on BiC ~S241

Remove expansion processing result Exp from BiC ~S242

F I G. 95

FIG.96

Blood vessel portion    Shape edge portion

G

GradG

$\nabla^2 G$    ←PV

R/(R+G+B)

BiC

FIG.97

Blood vessel portion    Shape edge portion

G

GradC    TGrad

BiGrad

FIG.98

Blood vessel portion    Shape edge portion

BiC

BiGrad

Logical
product L

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 10014864 A

- US 5872861 A

**Non-patent literature cited in the description**

- **Krevsky et al.** Enhanced imaging of angiodysplasias using remote endoscopic digital spectroscopy. *GAS-TROINTESTINAL ENDOSCOPY,* November 1996, vol. 44 (5), 598-602